# EUROPEAN PATENT APPLICATION

(11) **EP 2 703 005 A1**
(43) Date of publication of application: **05.03.2014**
(21) Application number: 12182994.9
(22) Date of filing: 04.09.2012
(51) Int. Cl.: A61K 38/17, C12N 15/00, G01N 33/00, A61P 35/04

(54) **Inhibitors of alpha6 Integrin/E-Cadherin Complex**

(71) Applicant: Università Degli Studi Di Torino, 10124 Torino (IT); Fondazione Piemontese per la Ricerca sul Cancro-ONLUS, 10060 Candiolo (TO) (IT)
(72) Inventor: Bussolino, Federico., 10129 Torino (IT); Marchio, Serena., 10134 Torino (IT)
(74) Representative: Weiss, Wolfgang

(57) **Abstract**

In one aspect, the invention relates to an inhibitor of the α₆ integrin/E-cadherin molecular complex for use as a medicament, in particular for the prevention or/and treatment of metastases of a primary cancer disease and a pharmaceutical composition or kit comprising as an active agent at least one of the inhibitors. In a further embodiment, the present invention relates to a method for determining the prognosis of metastatic homing of a primary cancer disease, in particular the aggressiveness of the metastatic potential of a primary cancer disease.

## Description

The present invention relates to inhibiting agents of a α₆ integrin/E-cadherin molecular complex for use as a medicament, particularly for the prevention or/and treatment of metastases of a primary cancer disease, and a method of determining the prognosis of metastatic homing of a primary cancer disease.

Cancer known medically as a malignant neoplasm, is a term for a large group of different diseases, all involving unregulated cell growth. In cancer, cells divide and grow uncontrollably, forming malignant tumors, and invade nearby parts of the body. The cancer may also spread to more distant parts of the body through the lymphatic system or bloodstream. When the area of cancer cells at the originating site becomes clinically detectable, it is called a primary tumor. Some cancer cells acquire the ability to penetrate and infiltrate surrounding normal tissues in the local area, forming a new tumor. The newly formed tumor within the tissue is called a local metastasis. Some cancer cells acquire the ability to penetrate the walls of lymphatic and/or blood vessels, after which they are able to circulate through the blood stream (circulating tumor cells) to other sites and tissues in the body. This process is known as lymphatic and hematogenous spread, respectively.

After the tumor cells come to rest at another site, they repenetrate through the vessel or walls, continue to multiply and eventually another clinically detectable tumor is formed. This new tumor is known as a metastatic (or secondary) tumor. The impact of secondary tumors is often more fatal than that of the primary tumor.

Advanced colorectal cancer (CRC) is a leading cause of cancer-related mortality, particularly due to the spreading of malignant tumor cells to the liver, among other organs. Despite increased public awareness and screening colonoscopy, up to 25% of patients diagnosed with CRC already have hepatic metastasis. In a further 30-40% of patients suffering from CRC, metastases will develop later in the course of the disease, usually within two years from the resection of the primary tumor (Parkin et al. 2005, CA Cancer J Clin 55:74-108.). Patients with operable liver-confined metastases may be cured by resection, but surgical cures are relatively rare in this setting (Tomlinson et al. 2007, J Clin Oncol 25:4575-4580). Most patients with metastatic disease are candidates for systemic chemotherapy to palliate symptoms and, potentially, downstage unresectable tumors to a resectable status (Meric et al. 2000, Ann Surg Oncol, 7:490-495; Adam et al. 2004, Ann Surg, 240:644-657). Without treatment, the median survival of patients with hepatic metastases is 6-8 months, and 5-year survival rates are lower than 5% (Wagner et al. 1984, Ann Surg, 199:502-508). The introduction of novel therapeutic agents (targeted bio-drugs such as Bevacizumab, Cetuximab and Panitumumab), in combination with cytotoxic drugs (i.e. oxaliplatin and irinotecan), has prolonged the median survival expectancy up to 24 months, albeit cure remains anecdotal (Hurwitz et al. 2004. N Engl J Med 350:2335-2342; Giantonio et al. 2007, J Clin Oncol 25:1539-1544; Hochster et al. 2008, J Clin Oncol 26:3523-3529; Saltz, 2008, Gastrointest Cancer Res, 2:S20-22; Kopetz et al. J Clin Oncol, 28:453-459; Jonker et al. 2007, N Engl J Med, 357:2040-2048; Hecht et al. 2009, J Clin Oncol, 27:672-680; Saltz et al. 2006, Nat Rev Drug Discov, 5:987-988; van Cutsem et al. 2007,J Clin Oncol, 25:1658-1664; Barugelet al. 2009, Expert Rev Anticancer Ther, 9:1829-1847). Therefore, hepatic metastasis clearly remains the central clinical challenge in the management of CRC.

A pivotal contribution to metastatic colonization comes from components of the host tissue and stroma. Therefore, targeting cancer microenvironments provides a promising strategy for the prevention or/and treatment of metastases.

It has long been recognized that several proteins integrate their stepwise action during the natural history of the progression and metastasis of human CRC (Fearon et al. 1990, Cell, 61:759-767; Vogelstein et al. 1988, N Engl J Med, 319:525-532). Insights into the molecular mechanisms underlying this disease have also begun to emerge through genomics and proteomics (Lin et al. 2007, Oncol Rep, 17:1541-1549; Zeitoun et al. 2008. Anticancer Res, 28:3609-3612; Nibbe et al. 2009, Mol Cell Proteomics, 8:827-845; Koh et al. 2008. Oncology, 75:92-101; Nannini et al. 2009, Cancer Treat Rev, 35:201-209). However, the fact that mRNA levels are not necessarily correlated with protein levels confers limitations on the significance of gene expression analyses (Nie et al. 2007. Crit Rev Biotechnol, 27:63-75). For example, comparative studies on metastatic prostate cancers revealed a concordance between protein and mRNA levels as low as 48-64% (Varambally et al. 2005. Cancer Cell, 8:393-406; Taylor et al. 2006. Cancer Res, 66:5537-5539). Alternatively, classical proteomic approaches are extremely time-consuming and expensive, which render their routine use difficult.

Angiopoietin-like 6 is a secreted factor whose mRNA has been detected particularly in the liver of humans. Although this protein shares a common structure with other members of the angiopoietin family, and particularly a coiled-coil domain in the N-terminal portion and a fibrinogen-like domain in the C-terminal portion, it does not bind to the Tie1 or Tie2 receptor and is currently considered an orphan ligand (Kim et al. 2000, Biochem J, 346 Pt 3:603-610; Oike et al. 2003, Proc Natl Acad Sci USA, 100:9494-9499; Oike et al. 2004, Blood, 103:3760-3765). Angiopoietin-like 6 regulates angiogenesis by preventing endothelial cell apoptosis, inducing endothelial cell migration and vascular leakiness and enhancing blood flow (Kim et al; Oike et al; Urano et al. 2008, Arterioscler Thromb Vasc Biol, 28:827-834). Some evidence suggests that RGD-binding integrins might be involved in angiopoietin-like 6-mediated cell adhesion, spreading and migration, although a direct interaction with integrins has not been described thus far (Zhang et al. 2006, Biochem Biophys Res Commun, 347:100-108).

Integrin α₆, complexed with either β₁ or β₄ subunit, is a receptor for laminin with an emerging role in regulating angiogenesis and cancer progression through both direct and indirect mechanisms (Humphries et al. 2006, J Cell Sci, 119:3901-3903; Primo et al. 2010, Cancer Res, 70:5759-5769, Lee et al. 2006, J Biol Chem, 281:40450-40460; Gonzalez et al. 2002, Proc Natl Acad Sci U S A, 99:16075-16080, Rabinovitz et al. 2001, Mol Biol Cell, 12:4030-4043; Robertson et al. 2008, Curr Pharm Des, 14:296-305). First, cellular delocalization of α₆β₄ integrin from hemidesmosomes to the edge of lamellipodia and filopodia has been related to a functional switch from adhesion to the extracellular matrix to migration at the invading front (Lipscomb et al. 2005. Cancer Metastasis Rev, 24:413-423). Second, physical interaction of α₆β₄ integrin with different tyrosine-kinase receptors has been shown to amplify pro-invasive signals (Bertotti et al. 2005. Cancer Res 65:10674-10679, Yoon et al. 2006, Cancer Res, 66:2732-2739). Third, both α₆β₁ and α₆β₄ integrins seem to be involved in CRC cell binding to hepatocytes as well as extravasation during the onset of metastasis, although a molecular mechanism for these functions remains to be elucidated (Enns et al. 2004. J Gastrointest Surg, 8:1049-1060; Robertson et al. 2009, Clin Exp Metastasis, 26:769-780).

E-cadherin is a well-described oncosuppressor protein, whose expression in the primary tumor counteracts cell detachment and is therefore associated with a better outcome (Christofori, 2003, Embo J, 22:2318-2323). Decreased production of E-cadherin, on the contrary, is one of the central events underlying epithelial-mesenchymal transition and carcinoma progression, in response to different cellular events such as the acquisition of loss-of-function mutations and loss-of-heterozygosis for the mutant allele, transcriptional or epigenetic repression and aberrant cellular localization (Ilyas et al. 1997, Gut, 40:654-659; Natalwala et al. 2008, World J Gastroenterol, 14:3792-3797; Kwak et al. 2007, Dis Colon Rectum, 50:1873-1880; Elzagheid et al. 2006, World J Gastroenterol, 12:4304-4309). Conversely, the role of E-cadherin in late stages of cancer progression needs further characterization. Remarkably, different reports show that mRNA and protein expression is regained in metastases, particularly in a subset of liver metastases from CRC and prostate carcinomas and increased levels of E-cadherin were found in metastatic tissue, particularly in liver metastases (Elzagheid et al.; Wells et al. 2008, Clin Exp Metastasis, 25:621-628, Truant et al., 2008, J Surg Res, 150:212-218).

It was found by the inventors that angiopoietin-like 6 acts as a ligand for cells that express a receptor complex of α₆ integrin and E-cadherin. The interaction between the angiopoietin-like 6 and the α₆ integrin/E-cadherin complex is found to have significant influence in metastasis homing and colonization. Experimental results show that inhibition of the α₆ integrin/E-cadherin molecular complex may inhibit/reduce metastases on different levels.

Thus, a first aspect of the present invention refers to an inhibiting agent of the α₆ integrin/E-cadherin molecular complex for use as a medicament, particularly for the prevention or/and treatment of metastasis of a primary cancer disease such as colorectal, bone, brain, breast, cervix, colon, gastric, liver, lung, pancreas, exocrine pancreas, duodenum, ovarian, renal, prostate, stomach, soft tissue, bone marrow, esophagus, skin cancer or lymphoma, particularly colorectal, stomach and lung cancer, more particularly colorectal cancer. The inhibiting agent may be used e.g. in human or veterinary medicine.

The α₆ integrin/E-cadherin molecular complex is formed by direct and/or indirect molecular interaction between the full length α₆ integrin protein (140 kD, SEQ ID No 16) or a proteolytic fragment thereof and the full length E-cadherin protein (120 kD, SEQ ID No 17) or a proteolytic fragment thereof.

Proteolytic fragments of α₆ integrin protein preferably have a molecular weight of 10 to 130 kDa, preferably 20 to 120 kDa. More preferably, the proteolytic fragments include the fragments of aa24-1073, aa24-899, aa903-1073, aa24-594, aa595-899 and/or aa595-1073 of the full-length α₆ integrin protein (SEQ ID No 16). Full length or proteolytic fragments of the α₆ integrin are described by Pawar et al., Exp Cell Res, 2007, 313, 6, 1080-9; Demetriou et al., Open Cancer J., 2008, 2:1-4; Pawar et al.; Int J Radiat Biol., 2007, 83(11-12):761-7); Sroka et al., Carcinogenesis, 2006, 27(9):1748-57; Demetriou et al., Exp Cell Res, 2004, 294(2):550-8 and Davis et al., Cell Growth Differ., 2002, 13(3):107-13, which are herein incorporated by reference.

Proteolytic fragments of E-cadherin protein preferably have a molecular weight of 20 to 100 kDa, preferably 30 to 97 kDa, particularly 30 kDa, 40 kDa, 80 kDa or 97 kDa. More preferably, the proteolytic fragments of E-Cadherin include amino acids aa36-882 of the full-length sequence (SEQ ID No 17). Full length or proteolytic fragments of E-cadherin are described by Solanas et al. Nat Cell Biol. 2011; Céspedes et al. Am J Pathol, 2010, 177, 4, 2067-79; Lynch et al., J Oncol, 2010, 2010:53074-5; Elston et al., J Clin Endocrinol Metab, 2009, 94, 4, 1436-42; Huguenin et al. PLoS One, 2008, 3, 5, e2153; Najy et al. J Biol Chem, 2008, 283, 26, 18393-401; Ferber et al. J Biol Chem, 2008, 283, 19, 12691-700 and Lee et al. Eur Surg Res, 2007, 39, 4, 208-15 which are herewith incorporated by reference.

The term *"direct molecular interaction"* means a covalent bond or noncovalent interactions, such as electrostatic or van-der-Waals interactions or hydrogen bonds, particularly van-der-Waals interactions. The term *"indirect molecular interactions"* refers to domains and/or regions where the complex partners α₆ integrin as well as E-cadherin are accumulated, i.e. where the concentration of both complex partners (α₆ integrin + E-cadherin) is increased as compared to the average concentration of (α₆ integrin + E-cadherin).

The α₆ integrin/E-cadherin molecular complex is preferably expressed by a plurality of tumor cells, preferably by metastatic tumor cells, preferably metastatic tumor cells of primary colorectal, bone, brain, breast, cervix, colon, gastric, liver, lung, pancreas, exocrine pancreas, duodenum, ovarian, renal, prostate, stomach, soft tissue, bone marrow, esophagus, skin cancer or metastatic tumor cells of lymphoma. Particularly the α₆ integrin/E-cadherin molecular complex is expressed by metastatic tumor cells of primary colorectal, stomach and lung cancer, more particularly primary colorectal cancer.

In a preferred embodiment, the molecular complex is expressed on the surface of metastatic tumor cells.

The inhibiting agent of the α₆ integrin/E-cadherin molecular complex may be selected from inhibitors acting on the protein level or on the nucleic acid level.

In a preferred embodiment of the invention, the complex inhibitor acts on the protein level. In a preferred embodiment, the inhibitor binds to the α₆ integrin/E-cadherin complex. By binding of the inhibitor to the complex, the activity of the complex, particularly its binding activity, may be altered, particularly reduced.

In one embodiment the inhibitor may be selected from an antibody, an antibody fragment or an antigen binding fragment specific for α₆ integrin or/and E-cadherin or/and E-cadherin/α₆ integrin complex, preferably for E-cadherin/α₆ integrin complex, or an aptamer directed against E-cadherin or/and α₆ integrin or/and E-cadherin/α₆ integrin complex, preferably an aptamer directed against E-cadherin/α₆ integrin complex.

Preferably, the inhibitor is an antibody. The antibody may be selected from a polyclonal antibody, a monoclonal antibody, a chimeric antibody, a humanized antibody, a human antibody, a recombinant antibody or a fragment thereof, preferably Fab' fragments, F(ab')₂ fragments or single-chain Fv fragments.

For the production of antibodies, a host animal, e.g. a mouse or rabbit, may be immunized with E-cadherin or/and α₆ integrin or/and E-cadherin/α₆ integrin antigen, optionally together with an adjuvant to increase the immunological response. A monoclonal antibody may be prepared by using known techniques, including but not limited to the hybridoma technique developed by Köhler and Millstein. Chimeric antibodies may be obtained from monoclonal antibodies by replacing non-human constant regions by appropriate human constant regions. Humanized antibodies may be obtained by replacing non-human framework regions in the variable antibody domains by appropriate human sequences. Human antibodies may be obtained from host animals, e.g. mice, comprising a xenogenic human immune system. Recombinant antibodies may be obtained by phage display and affinity maturation of given antibody sequences. Recombinant antibodies may be single-chain antibodies, bispecific antibodies etc.

Antibody fragments, which contain at least one binding site for E-cadherin or/and α₆ integrin or/and α₆ integrin/E-cadherin complex may be selected from Fab fragments, Fab' fragments, F(ab')₂ fragments or single-chain Fv fragments. Aptamers directed against E-cadherin or/and α₆ integrin or/and α₆ integrin/E-cadherin complex may be obtained by affinity selection of nucleic acid and/or peptidic sequences according to known protocols.

WO 2008/064910 discloses peptides capable of selectively binding to metastatic cells having a sequence motif LRS and a length of 6 to 100 amino acids. The peptides, if labeled, can be used for the detection of hepatic metastases already in pre-clinical stages. The authors further suggest conjugating the peptides with chemotherapeutic drugs for target therapy. WO 2008/064910, however, does not give any hint to use these peptides alone, i.e. in non-conjugated form, as a medicament. It has now surprisingly been found that such peptides - without conjugated drugs or diagnostic agents - effectively inhibit the E-cadherin/α₆ integrin complex.

Thus, in a preferred embodiment of the invention, the inhibiting agent of the E-cadherin/α₆ integrin complex is a peptide having the sequence motif LRS and a length of 6 to 100, preferably to 70, more preferably to 40, most preferably to 35, amino acids. In a preferred embodiment, such peptides are not conjugated, e.g. chemically or physically, to other active agents, such as drugs or diagnostic agents, which are preferably different from the inhibitors according to the invention.

The term *"peptide"* includes amino acid sequences constituted by at least one of the 20 common amino acids that can be found in natural proteins, modified, e.g. non genetically encoded, amino acids, amino acid mimetics known in the art or unusual amino acids such as Aad, 2- Aminoadipic acid; EtAsn, N-Ethylasparagine; Baad, 3-Aminoadipic acid, Hyl, Hydroxylysine; Bala, beta-alanine, beta-Amino-propionic acid; AHyl, allo- Hydroxylysine; Abu, 2-Aminobutyric acid; 3Hyp, 3-Hydroxyproline; 4Abu, 4- Aminobutyric acid, piperidinic acid; 4Hyp, 4-Hydroxyproline; Acp, 6-Aminocaproic acid, Ide, Isodesmosine; Ahe, 2-Aminoheptanoic acid; Alle, allo-Isoleucine; Aib, 2-Aminoisobutyric acid; MeGly, N-Methylglycine, sarcosine; Baib, 3-Aminoisobutyric acid; Melle, N-Methylisoleucine; Apm, 2- Aminopimelic acid; MeLys, 6-N-Methyllysine; Dbu, 2,4-Diaminobutyric acid; 5 MeVal, N-Methylvaline; Des, Desmosine; Nva, Norvaline; Dpm, 2,2'-Diaminopimelic acid; Nle, Norleucine; Dpr, 2,3-Diaminopropionic acid; Orn, Ornithine; and EtGly, N-Ethylglycine. Also included are amino acids in D-configuration.

In particular embodiments, the amino acid sequence may include one or more non-amino acids. In particular embodiments, the sequence of a peptide of the present invention may be interrupted by one or more non-amino acids.

The peptides of the present invention may be linear or cyclic peptides, preferably linear.

In a preferred embodiment of the present invention, peptides inhibiting the E-cadherin/α₆ integrin complex comprise an amino acid sequence selected from the group consisting of ARPGLRS (SEQ ID NO. 1), MRYALRS (SEQ ID NO. 2), LRPGLRS (SEQ ID NO. 3), LRSGSGS (SEQ ID NO. 4), GIYRLRS (SEQ ID NO. 5), GVYSLRS (SEQ ID NO. 6), LRSGRGS (SEQ ID NO. 7), RREGLRS (SEQ ID NO. 8), SWYTLRS (SEQ ID NO. 9), LAYRLRS (SEQ ID NO. 10), LTYRLRS (SEQ ID NO. 11), VRPGLRS (SEQ ID NO. 12), LRSGRGS (SEQ ID NO. 13), preferably GIYRLRS (SEQ ID NO. 5) and GVYSLRS (SEQ ID NO. 6).

In a preferred embodiment, the inhibiting agent is a peptide comprising the amino acid sequence CGIYRLRSC (SEQ ID NO. 14) and CGVYSLRSC (SEQ ID NO. 15).

Due to their relatively small size, the peptides according to the invention can be synthesized in solution or on solid supports, according to well known techniques. Short peptides, generally from about 6 to 35-40 amino acids, can be easily produced with these techniques. Alternatively, recombinant cDNA technology can be used, in which a nucleotidic sequence coding for a peptide of the invention is inserted in an expression vector, transformed or transfected in proper host cells, and cultured in conditions suitable for protein expression.

In another embodiment the inhibitor of E-cadherin/α₆ integrin complex acts on the nucleic acid level, e.g. by inhibiting E-cadherin or/and α₆ integrin or/and E-cadherin/α₆ integrin complex transcription and/or translation.

The inhibitor of E-cadherin/α₆ integrin complex nucleic acid may be an α₆ integrin or/and an E-cadherin gene expression inhibitor, preferably selected from nucleic acid effector molecules directed against E-cadherin or/and α₆ integrin mRNA, such as RNAi molecules or precursors or templates thereof, antisense molecules or ribozymes.

RNAi molecules are RNA molecules or RNA analogues capable of mediating an interference of a target mRNA molecule. RNAi molecules may be siRNA molecules (short-interfering RNA molecules), which are short, doublestranded RNA molecules with a length of preferably 18-30 nucleotides and optionally at least one 3' overhang. Further RNAi molecules may be shRNA molecules (short hairpin RNA molecules) having a length of e.g. 14-50 nucleotides. Optionally, the RNAi molecules may comprise ribonucleotide analogues in order to enhance the stability against degradation. The invention also encompasses precursors of RNAi molecules, i.e. RNA molecules which are processed by cellular mechanisms into active RNAi molecules. Further, the invention encompasses DNA templates of RNAi molecules or precursors thereof, wherein the templates are operatively linked to an expression control sequence. The RNAi molecules have sufficient complementarity to the α₆ integrin or/and E-cadherin mRNA to allow specific degradation thereof, thereby inhibiting α₆ integrin or/and E-cadherin expression.

In a preferred embodiment, the siRNA molecule has a sense strand selected from
(i) SEQ ID NO 18, SEQ ID NO 19, SEQ ID NO 20, SEQ ID NO 21, SEQ ID NO 22, SEQ ID NO 23, SEQ ID NO 24, or SEQ ID NO 25 or a
(ii) nucleotide sequence which has an identity degree of at least 85%, at least 90% or at least 95% to any of the sequences according to (i).

In a further embodiment, the nucleic acid inhibitor molecule may be an antisense molecule, i.e. an antisense RNA, DNA or nucleic acid analogue molecule, which blocks translation of α₆ integrin or/and E-cadherin mRNA by binding thereto and preventing translation. Antisense molecules may be single-stranded and preferably have a length of 14-30 nucleotides. Antisense molecules directed against the translation initiation site of E-cadherin or/and α₆ integrin mRNA are preferred.

In a further embodiment, the E-cadherin or/and α₆ integrin nucleic acid inhibitor may be a ribozyme. Ribozymes are enzymatic RNA molecules which catalyze specific cleavage of RNA, e.g. hammerhead ribozymes.

The inhibiting agent of the present invention is used as a medicament, particularly as a medicament for the prevention or/and treatment of metastases of a primary cancer disease. The primary cancer disease may preferably selected from the group consisting of colorectal, bone, brain, breast, cervix, colon, gastric, liver, lung, pancreas, exocrine pancreas, duodenum, ovarian, renal, prostate, stomach, soft tissue, bone marrow, esophagus or skin cancer or lymphoma, particularly colorectal, stomach or lung cancer, preferably colorectal cancer.

In a preferred embodiment, the inhibiting agent is used to prevent or/and reduce metastases in liver tissue, breast tissue, lung tissue, lymph nodes, bone tissue or brain tissue, preferably in liver tissue.

In a particularly preferred embodiment of the invention, the inhibiting agent is used for the prevention or/and treatment of metastases deriving from primary colorectal cancer in liver tissue.

In other words, the inhibiting agent may be used to prevent or/and reduce secondary cancer, particularly in liver tissue, breast tissue, lung tissue, lymph nodes, brain tissue or bone tissue, preferably in liver tissue.

In another aspect, the inhibiting agent of the invention may be used in combination with another (other than the inhibiting agent) anti-cancer or/and anti-viral therapy, preferably anti-cancer therapy. The anti-cancer therapy may be selected from chemotherapy, radiation therapy, surgical intervention, immunotherapy, gene therapy, target therapy or combinations thereof.

The inhibiting agent is preferably used in combination with at least another additional chemotherapeutic or/and antiviral agent. The chemotherapeutic agent may be selected from antimetabolites, DNA-fragmenting agents, DNA-crosslinking agents, intercalating agents, protein synthesis inhibitors, Topoisomerase 1 and 2 inhibitors, microtubule-directed agents, kinase inhibitors, hormones and hormone antagonists, anti-tumor antibodies, or any combination thereof. Preferably, the anti-cancer agent is selected from platinum compounds (oxaliplatinum), fluoropyrimidines (inhibitors of the thymidylate synthetase, such as capecitabine and its derivative 5-fluorouracil), alkaloids (inhibitors of the topoisomerase I, such as campthotecin and its derivative irinotecan).

The anti-viral agent may be selected from a protease inhibitor, a polymerase inhibitor, an integrase inhibitor, an entry inhibitor, an assembly secretion inhibitor, a translation inhibitor, an immunostimulant or any combination thereof.

Preferably, the inhibiting agent may be co-administered with at least another chemotherapeutic or/and anti-viral agent. In another embodiment, the inhibiting agent and the chemotherapeutic or/and anti-viral agent may be administered separately.

A further aspect of the invention is a pharmaceutical composition or kit which comprises as an active agent at least one inhibiting agent of α₆ integrin or/and E-cadherin or/and α6 integrin/E-cadherin complex as described above, together with a pharmaceutically acceptable carrier, diluent and/or adjuvant. The pharmaceutical composition is preferably for use in medicine, e.g. in human or veterinary medicine.

The term *"pharmaceutically acceptable carrier"* preferably includes sterile water, buffers or isotonic saline.

The term *"diluent and adjuvant"* preferably includes solvents such as ethanol, antioxidants and/or preservatives.

The pharmaceutical composition may be formulated e.g. as tablets, pills, capsules, liquids, sirups, slurries, suspensions, injectable solutions etc. Depending on the specific disorder to be treated, the composition may be administered systemically or locally. Suitable routes may e.g. include oral, rectal, transmucosal, intestinal, intranasal, intraocular or pulmonal administration or parenteral delivery, including intramuscular, subcutaneous, intrathecal, intravenous or intraperitoneal injection or infusion.

The pharmaceutical composition comprises the active agent in an effective dose, sufficient to achieve its intended purpose. Determination of an effective dose can be carried out by the skilled person. For example, the effective dose may be estimated from cell culture assays and animal models. Usual dosages for administration in human medicine may range from e.g. 0.01 to 2000 mg/day, commonly from 0.1 to 1000 mg/day and typically from 1 to 500 mg/day.

The pharmaceutical composition according to the present invention may further comprise at least one other active agent, such as an anti-cancer, e.g. a chemotherapeutic agent or/and an anti-viral agent. The anti-cancer agent may or/and the anti-viral agent may be as defined above.
Another aspect of the invention is directed to a method of screening for an inhibiting agent for the α₆ integrin/E-cadherin molecular complex, comprising the steps of:
(i) incubating a α₆ integrin/E-cadherin molecular complex with an agent under conditions suitable to induce binding of the agent to the complex,
(ii) detecting the binding of the agent to the complex,
(iii) comparing the result obtained in step (ii) with a predetermined binding score, and
(iv) evaluating the agent to be an inhibiting agent for α₆ integrin/E-cadherin molecular complex.

In the method according to the invention, α₆ integrin/E-cadherin molecular complex and cells expressing the α₆ integrin/E-cadherin molecular complex, respectively, are incubated with a candidate agent. Incubation preferably takes place at 2-10° C, preferably 4-6° C, in phosphate buffer saline or in cell culture Hepes-buffered medium (such as Iscove's Modified Dulbecco's Minimal Essential Medium) for 0.5-4 hours, preferably 1.5-2.5 hours. The binding of the candidate agent to the α₆ integrin/E-cadherin molecular complex is detected via phage displayed peptide binding assay, radio- or dye-labelled ligand binding or/and surface plasmon resonance assay, preferably by phage displayed peptide binding assay. These methods are known in the art and are described, e.g. by Maynard et al., Biotechnol J., 2009, 4(11):1542-58; Hulme et al., Br J Pharmacol., 2010, 161(6):1219-37 and Marchiò et al., Cancer Cell., 2004, 5(2):151-62, which are herein incorporated by reference.

The results obtained are compared to the extent of binding of a known agent to the molecular complex. The predetermined binding score is a quantitative parameter of the binding of a known substance (standard) to the complex. Preferably, the predetermined binding score is selected such that any agent which has the same or a higher binding score than that of the standard can be regarded as an inhibitor.

Preferred standard substances are e.g. laminin 332 or E-cadherin.

In another aspect, the present invention provides a method for determining the prognosis of metastatic homing of a primary cancer disease, in particular the aggressiveness of the metastatic potential of a primary cancer disease, comprising the steps of:
(i) providing a sample of a patient suffering or suspicious to suffer from metastasis of a primary cancer disease,
(ii) determining the expression or/and amount of the α₆ integrin/E-cadherin molecular complex or/and the amount of angiopoietin like 6 in the sample, and
(iii) optionally classifying the results obtained in step (ii) in predetermined disease states.

In a preferred embodiment, a sample, e.g. a blood sample, tissue sample or lymph liquid from a patient suffering from metastases of, e.g. colorectal, bone, brain, breast, cervix, colon, gastric, liver, lung, pancreas, ovarian, renal, pancreas, prostate, stomach, soft tissue, bone marrow or skin primary cancer or lymphoma primary cancer, particularly colorectal primary cancer, is provided. The sample may be a blood sample, tissue sample or lymph liquid. Particularly, the sample may be a blood sample or a tissue sample of the organs affected by the primary cancer, e.g. a colorectum sample, or/and a tissue sample of the organ suspicious to suffer from a secondary cancer organ, e.g. liver tissue, breast tissue, lung tissue or lymph liquid, preferably liver tissue.

Determination of the expression or/and amount of the α₆ integrin/E-cadherin molecular complex or/and angiopoietin-like 6 protein, is carried out by conventional assays as known in the art, e.g. immunofluorescence staining.

High amounts or upregulated expression of α₆ integrin/E-cadherin molecular complex or/and angiopoietin-like 6 are usually associated with advanced metastasis homing and shorter disease-free survival.

The amounts or the expression of α₆ integrin/E-cadherin molecular complex or/and angiopoietin like 6 may be classified in predetermined disease states.

### Figure Legend

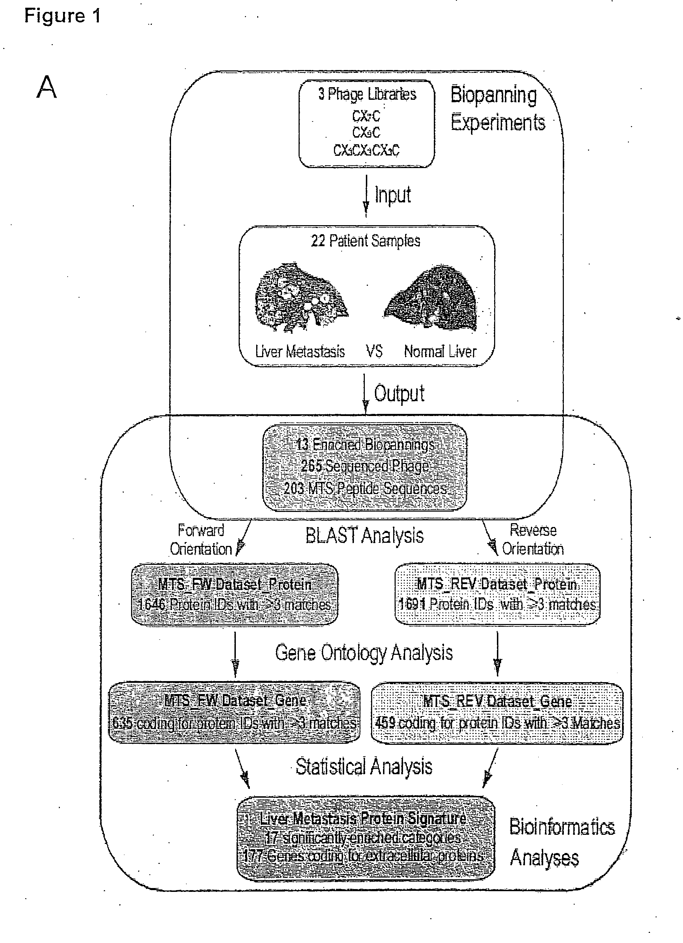

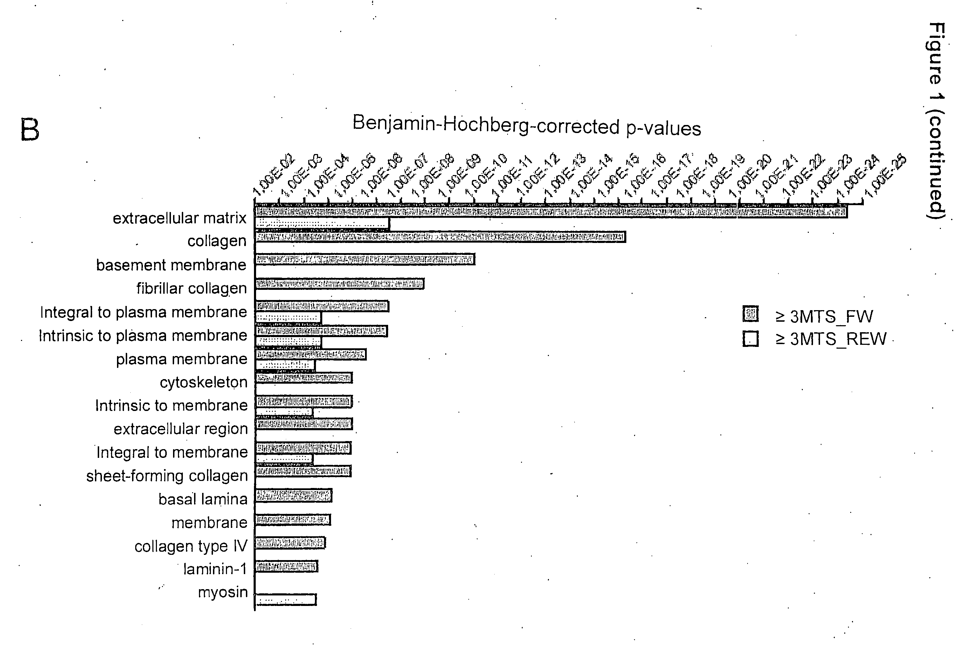

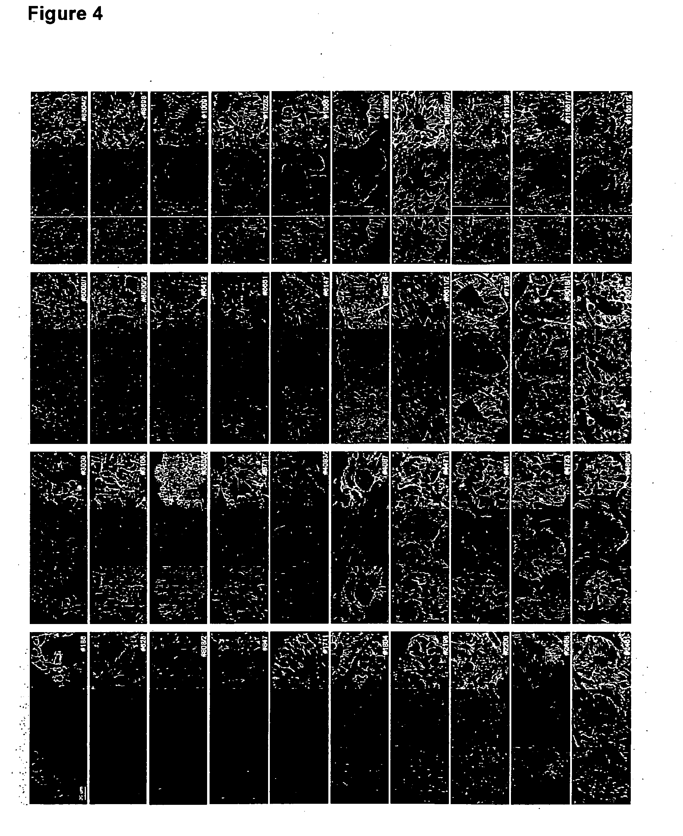

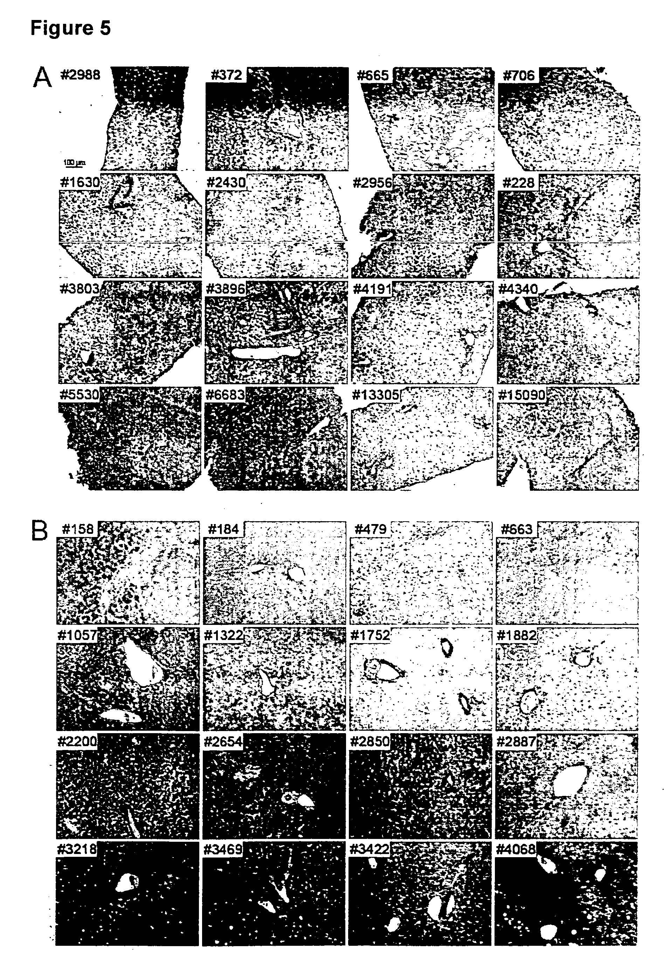

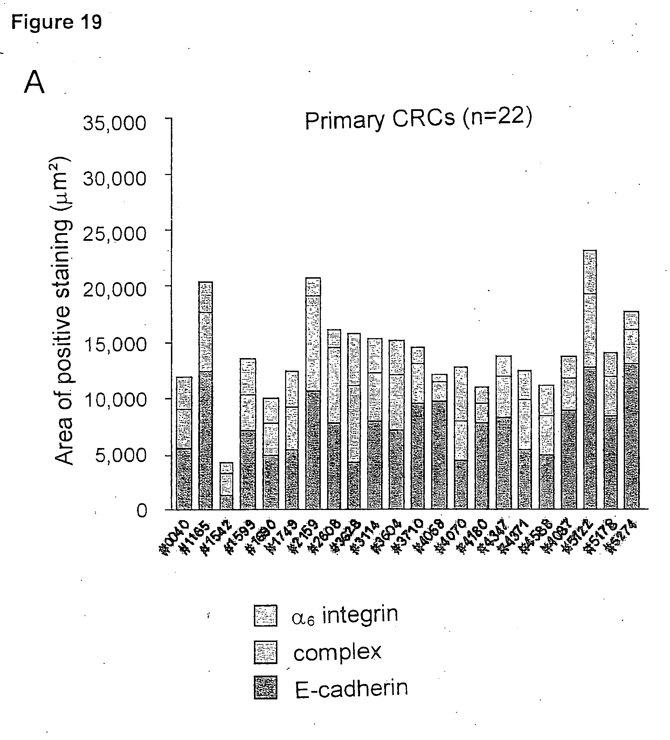

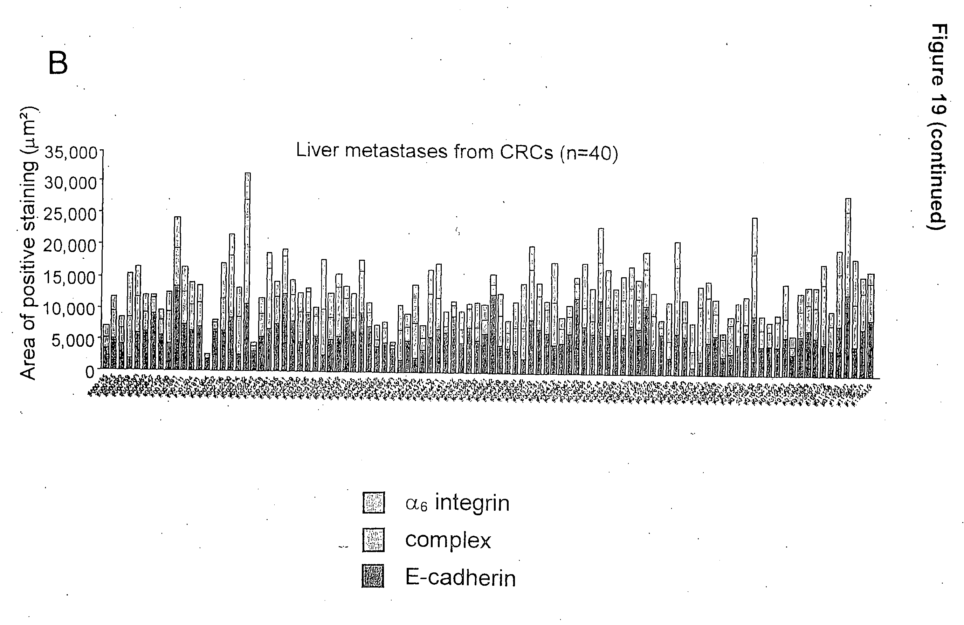

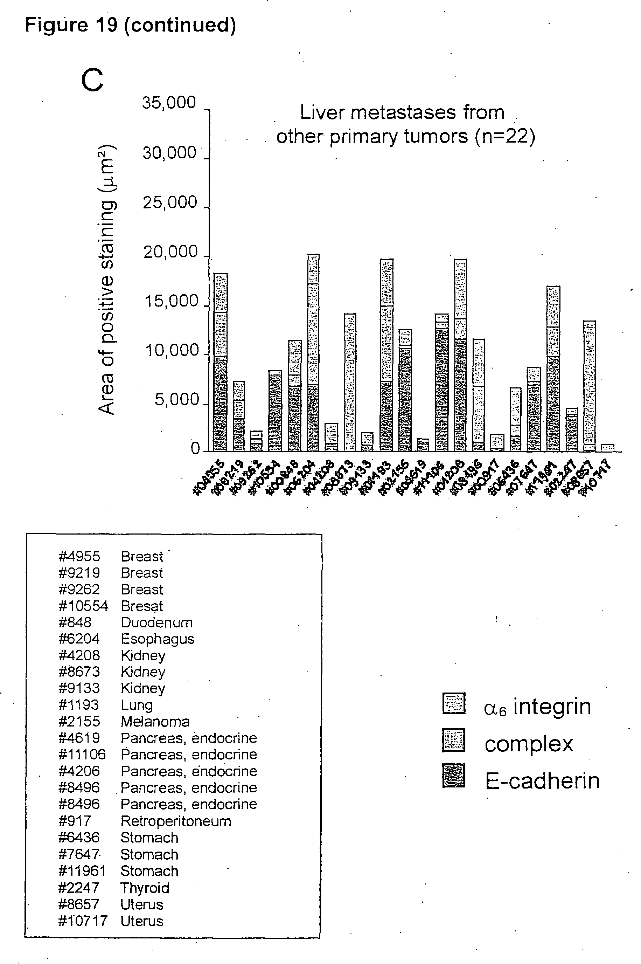

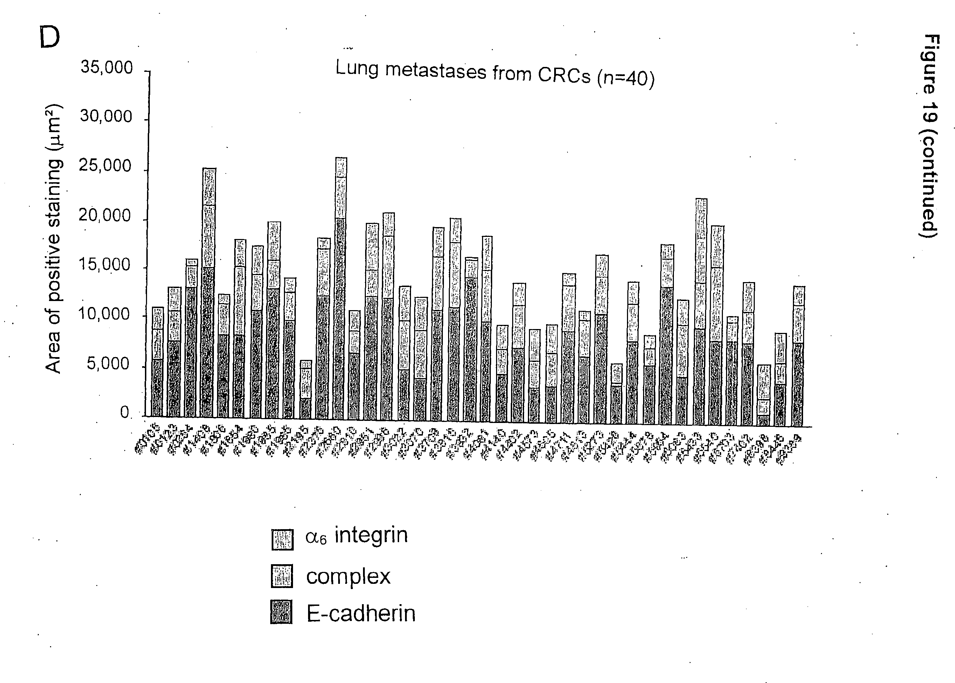

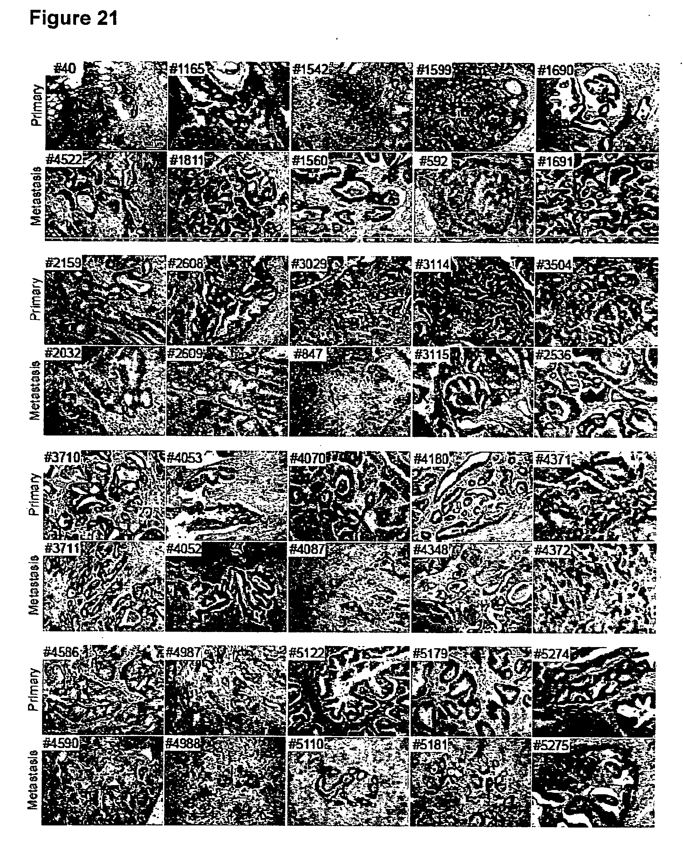

**Figure 1****: Phage display-selected peptides identify an extracellular signature for human liver metastases secondary to CRCs**
   **(A)** Flowchart of biopanning experiments and bioinformatics analyses. Three phage displayed-peptide libraries were biopanned on sample pairs from 22 patients, by pre-adsorption on control tissues (grossly normal liver) followed by enrichment on target tissues (liver metastasis) (Patient IDs: P2, P3a-b, P5, P6, P8a-b, P9, P10, P11, P12, P13, P14, P15, P16, P17, P18, P19, P20, P22, P23, P25). In 13 experiments a selective enrichment in phage binding was observed. Sequencing of the derived 265 phage inserts revealed 203 unique MTS peptide sequences. A search for human proteins similar to these peptides, either in the FW or in the REV orientation, produced the output MTS_FW and MTS_REV datasets, respectively. Comparable numbers of protein IDs in each dataset shared similarity with at least 3 different peptides or with a same peptide in at least 3 different regions. Genes coding for these protein IDs were assigned a GO-CC category with the DAVID Bioinformatics Resources Functional Annotation tool. Genes of the MTS-FW dataset (n=635) coding for extracellular proteins (n=177) were extracted to create the *"extracellular protein signature"* of liver metastasis from CRC. **(B)** Statistical analysis of GO-CC-enriched categories. The 17 significantly enriched GO-CC categories for both the MTS_FW and MTS_REV datasets are represented as Benjamini-Hochberg-corrected p-values.

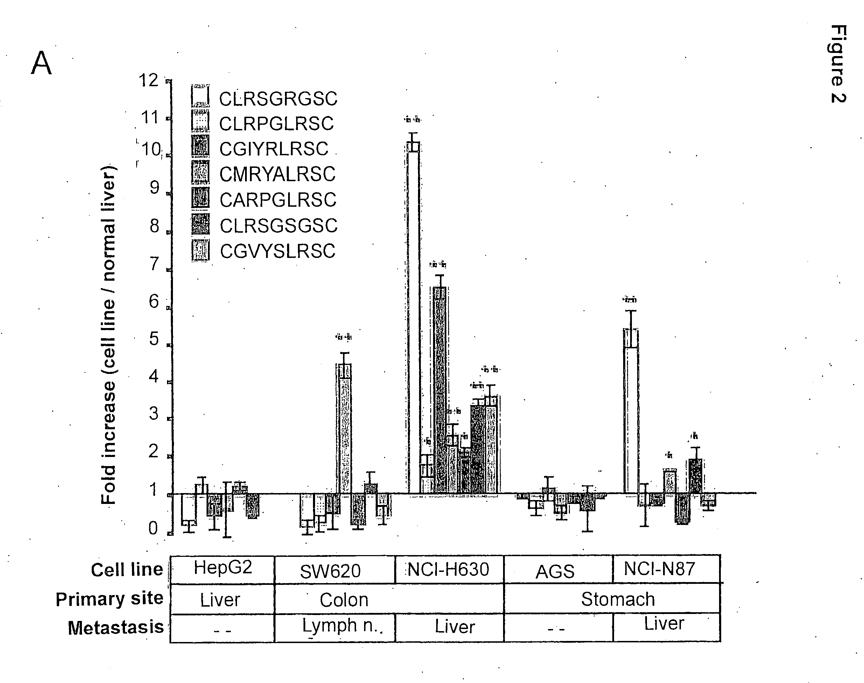

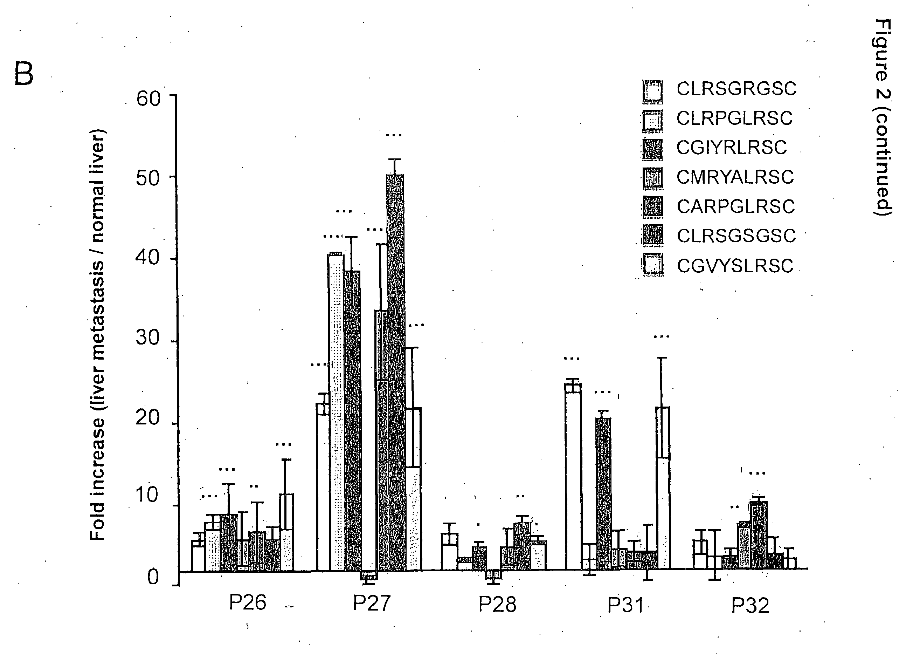

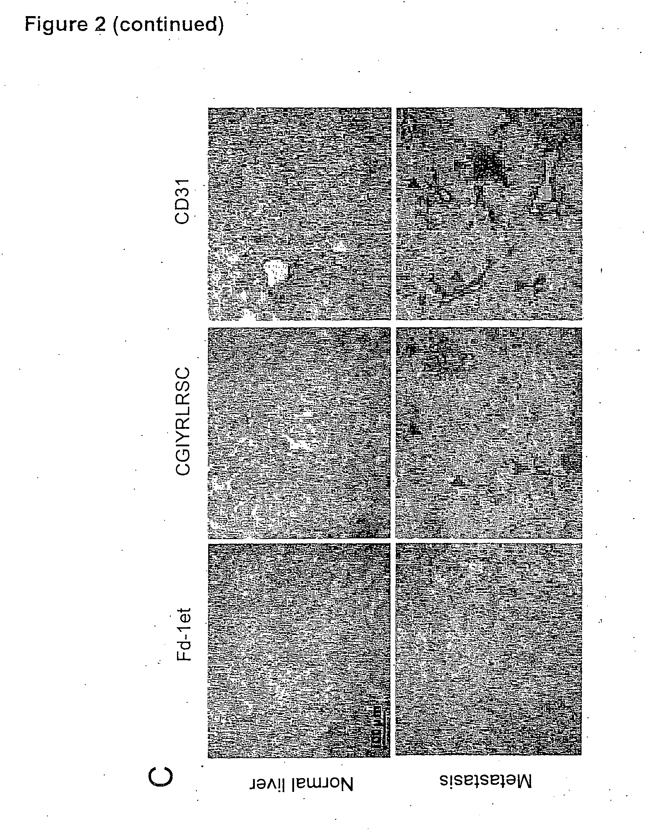

**Figure 2****: LRS-displaying MTS phage clones target human liver metastases from CRC**
   **(A, B)** Validation of MTS phage specificity. CLRSGRGSC- (SEQ ID NO. 38), CLRPGLRSC- (SEQ ID NO. 39), CGIYRLRSC-, CMRYALRSC- (SEQ ID NO. 40), CARPGLRSC- (SEQ ID NO. 41), CLRSGSGSC- (SEQ ID NO. 42) or CGVYSLRSC-phage was incubated with the indicated cell lines **(A)** or with suspended cells of human liver metastasis from 5 patients (Patient IDs: P26, P27, P28, P31, P32) **(B).** Numbers were normalized first to the degree of binding to the insertless fd-tet phage and subsequently to that of normal liver cells. Results are shown as mean ± standard deviation for each experimental point in 5 independent experiments. In A and B, statistical significance was evaluated by the use of ANOVA followed by Bonferroni's post-test, keeping as a reference a 1.5-fold threshold that is assumed as positive phage binding. (C) The tissue and cell specificity of CGIYRLRSC-phage was further evaluated in overlay binding assays on 10 µm cryostatic tissue sections of normal livers and of liver metastases from 14 patients (Table 1). A representative assay, performed on tissues from patient P30, is shown. The insertless fd-tet phage was used as a negative control and blood vessels were stained with anti-CD31 antibody. Arrowheads point to the same blood vessels in consecutive sections.

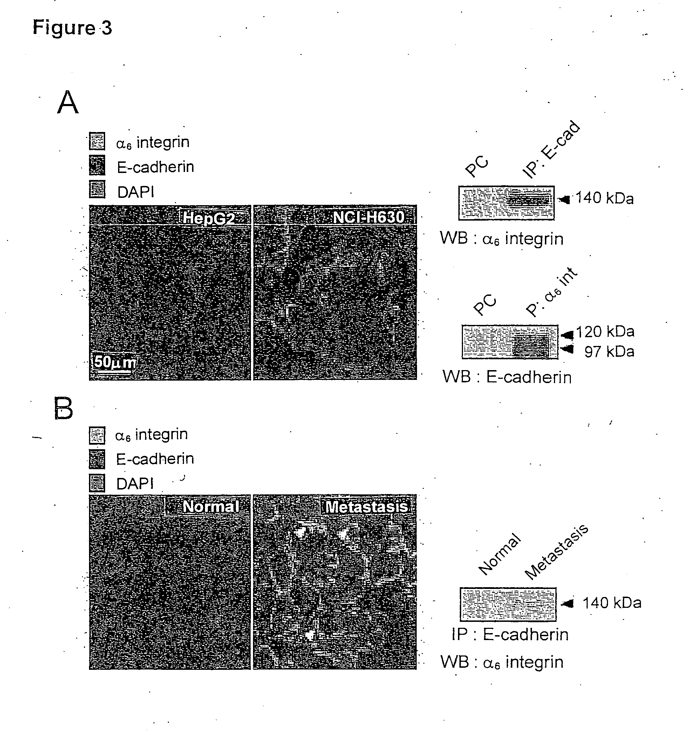

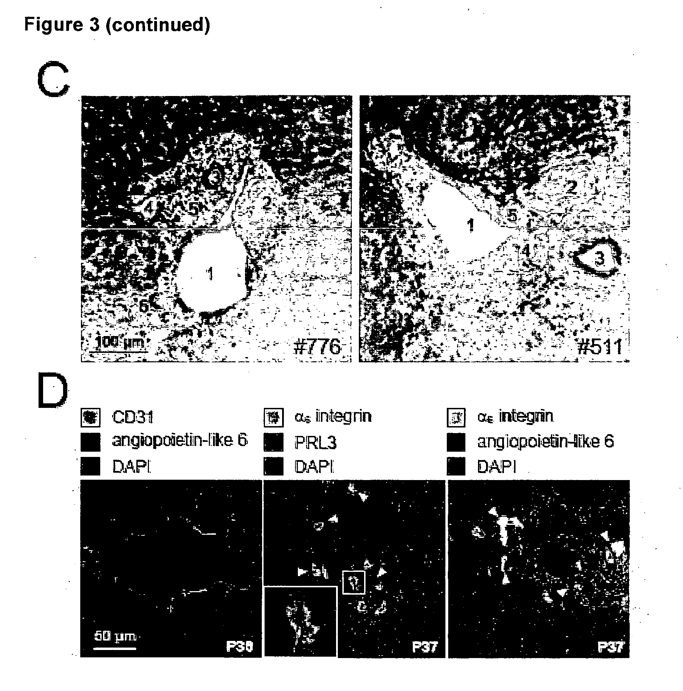

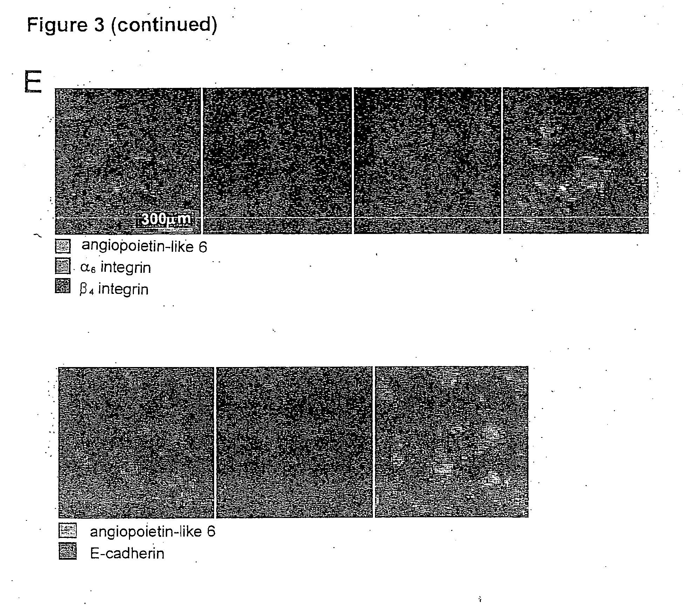

**Figure 3****: Coupling receptors and ligands: cell lines, human tissues and *in vitro* models of metastasis**
   **(A, B)** α₆ integrin and E-cadherin are part of a supramolecular complex in human liver metastases. NCI-H630 and HepG2 cells were cultured on positively-charged glass slides for 24 hours, followed by immunostaining of α₆ integrin and E-cadherin; in all the fluorescence images nuclei are stained blue (4',6-diamidino-2-phenylindole, DAPI) and co-localization is revealed by the yellow color. The interaction between α₆ integrin and E-cadherin was confirmed by co-immunoprecipitation: 10 mg of total NCI-H630 protein was subjected to immunoprecipitation with either anti-E-cadherin or anti-α₆ integrin antibody and proteins were separated on a 10% SDS-polyacrylamide gel. Blotted PVDF membranes were exposed to anti-α₆ integrin or anti-E-cadherin antibody, respectively. Proteins eluted from the pre-clearing step (PC) were loaded as specificity controls (A). The location of α₆ integrin and E-cadherin was also evaluated on 10-µm sections of OCT-frozen tissue pairs (grossly normal liver/liver metastasis) from CRC patients (see also Figure 4); arrowheads indicate regions of co-localization. A representative immunostaining of normal liver tissue from patient P36 is shown. The interaction between α₆ integrin and E-cadherin was confirmed by co-immunoprecipitation: 4 mg of total protein from 5 pooled samples (patient IDs: P48, P49, P53, P54, P55) of grossly normal liver and liver metastases was subjected to immunoprecipitation with anti-E-cadherin antibody and proteins were separated on a 10% SDS-polyacrylamide gel. The blotted PVDF membrane was incubated with anti-α₆ integrin antibody. **(B) (C, D)** Angiopoietin-like 6 is accumulated in hepatic blood vessels where it can interact with circulating CRC cells. The location of angiopoietin-like 6 was evaluated on 5-µm sections of paraffin-embedded normal livers from 79 patients (see also Figure 5); representative immunostaining of hepatic tissues from two patients is shown (1, branch of portal vein; 2, branch of hepatic artery; 3, bile duct; 4, lymphatic capillary; 5, interlobular connective tissue; 6, sinusoids). Numbers refer to the histological archive; tissues were counterstained with hematoxylin **(C).** The interaction of hepatic angiopoietin-like 6 with micrometastases was evaluated on 10-µm frozen sections of grossly normal liver tissues from 3 patients (Patient IDs: P36, P37, P44). Immunostaining for CD31 was performed to identify endothelial cells; immunostaining for PRL3 and α₆ integrin was performed to identify metastatic cells in grossly normal liver tissues; immunostaining for angiopoietin-like 6 and α₆ integrin was performed on tissue sections from the same patients to investigate the co-localization of hepatic angiopoietin-like 6 and CRC metastatic cells. Representative immunostaining of tissues from patient P36 (left panel) and P37 (middle and right panel) are shown; arrowheads indicate regions of co-localization **(D). (E)** α₆ integrin and E-cadherin confer a metastasis-like morphology to cells cultured in a host tissue-like microenvironment. U293 cells stably transduced with α₆Aβ₄ integrin and E-cadherin were mixed with cells expressing angiopoietin-like 6 and cultured for 48 hours, before staining with anti-angiopoietin-like 6, anti-α₆ integrin, anti-β₄ integrin and anti-E-cadherin antibodies. In **A, B,** and **D,** fluorescent images were acquired with a confocal microscope to allow identification of signal coincidence; in **E,** images were acquired with an optical microscope to evaluate overall morphology and angiopoietin-like 6 coverage of the metastasis-like structures.
**Figure 4****: Quantification of α₆ integrin, E-cadherin and their molecular complex in metastatic CRCs (liver metastasis)**
   The location of α₆ integrin and E-cadherin in paraffin-embedded liver metastases from CRCs was evaluated as described in Figure 16 and in Figure 19.
**Figure 5****: Angiopoietin-like 6 has a different expression pattern in livers from patients with metastatic CRC compared to livers from healthy donors**
   The amounts and localization of angiopoietin-like 6 in livers from healthy donors (n=17) **(A)** and from patients with metastatic CRC (n=79) **(B)** were evaluated by staining of 5 µm tissue sections. Pictures of 16 samples for each tissue panel are shown.
**Figure 6****: Protein quantification in all the described cell lines**
   For protein quantification, 50 µg of total lysate was loaded on an 8% SDS-polyacrylamide gel and proteins resolved by electrophoresis were blotted onto a PVDF membrane. Membranes were stained with the following primary antibodies: mouse monoclonal anti-β₄ integrin clone 7, goat polyclonal anti-α₆ integrin N-19, mouse monoclonal anti-β₁ integrin clone P4G11, mouse monoclonal anti-E-cadherin clone 36, mouse monoclonal anti-angiopoietin-like 6 clone Kairos-60, goat polyclonal anti-vinculin N-19. Vinculin was used as a loading control and as a normalizer for the densitometric quantification of band intensity (illustrated in the graphs). **(A)** CRC cell lines used for the *in vitro* and *in vivo* experiments. **(B)** U293 cells stably overexpressing E-cadherin, α₆Aβ₄ integrin, a combination of both or angiopoietin-like 6.

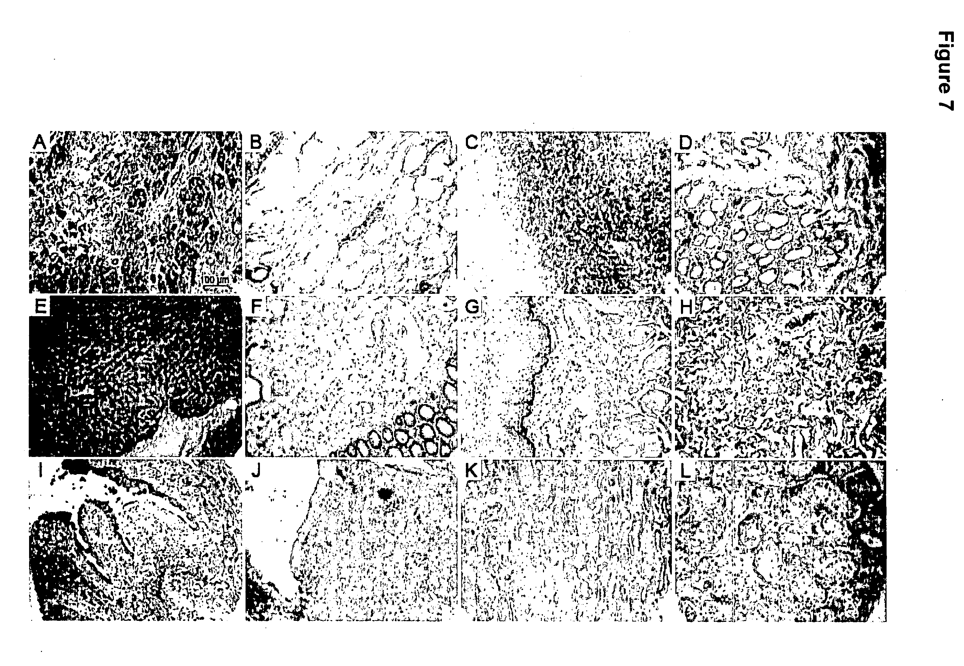

**Figure 7****: Angiopoietin-like 6 protein is highly expressed in hepatic tissues in humans**
   Paraffin-embedded normal tissue samples from healthy donors were cut in 5 µm sections and were stained with the rabbit polyclonal anti-angiopoietin-like 6 antibody. **(A)** pancreas, **(B)** breast, **(C)** cerebellum, **(D)** stomach, **(E)** liver, **(F)** intestine, **(G)** esophagus, **(H)** lung, **(I)** bladder, **(J)** spleen, **(K)** kidney, **(L)** testis.

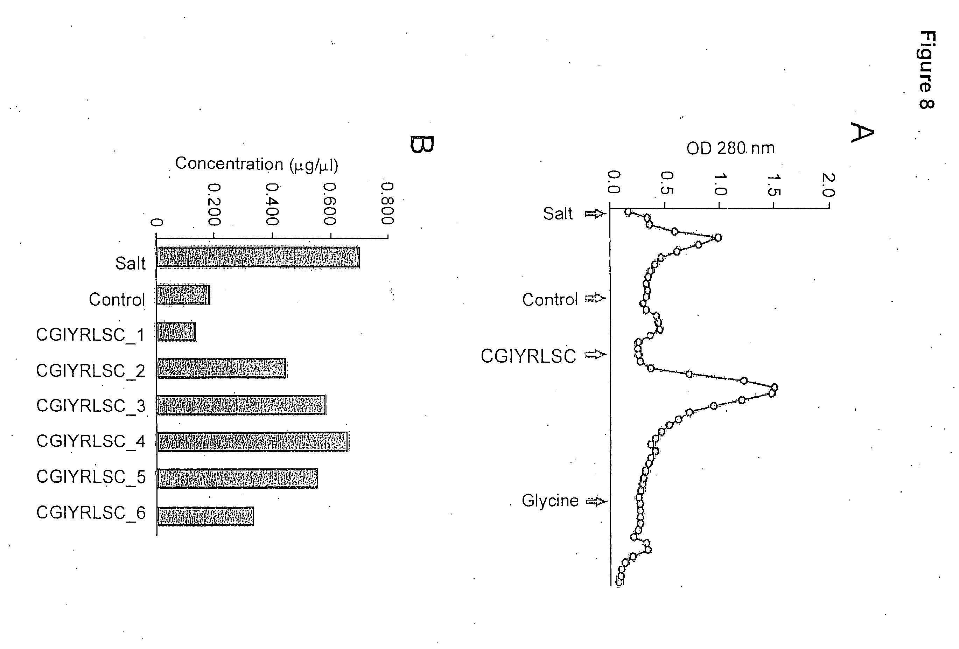

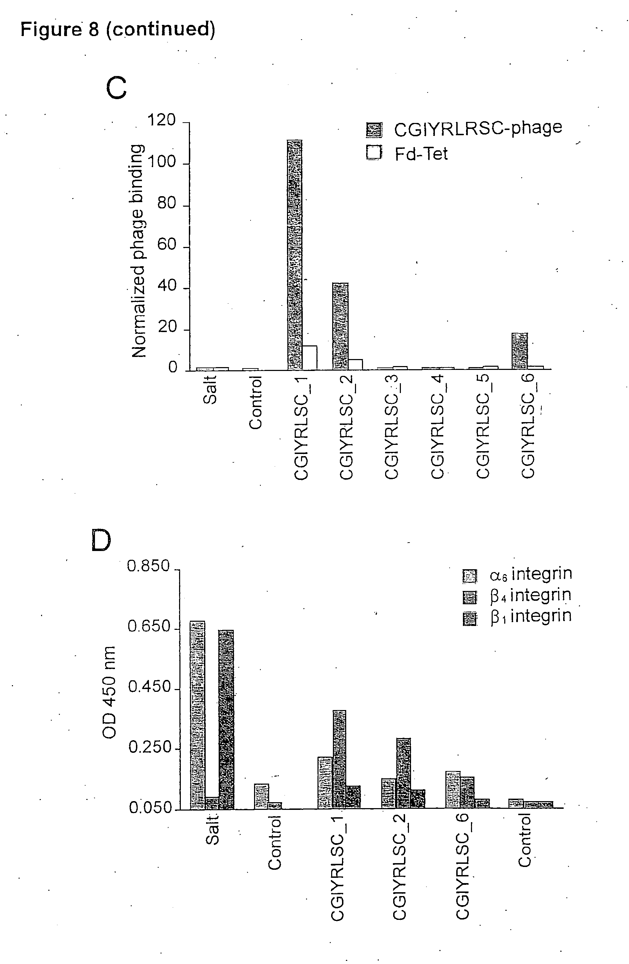

**Figure 8****: The β₄ subunit is the partner for α₆ integrin in the MTS peptide-target complex**
   **(A, B)** Isolation of CGIYRLRSC-targeted integrin partners. To identify the molecular partner of α₆ integrin in the peptide-targeted complex, synthetic CGIYRLRSC was immobilized on column-packed diaminodipropylamine-agarose and successively loaded with 30 mg of total protein from 7 pooled liver metastases secondary to CRCs. Protein fractions were collected after sequential incubation with high salt buffer (for the elution of unspecific proteins), CARAC-peptide (SEQ ID NO. 43) (as a negative control) and CGIYRLRSC-peptide (target elution). Protein amounts in collected fractions were followed by reading their OD at 280 nm **(A).** Selected fractions were concentrated by the use of centrifugal filter devices with cut-off 10,000 to remove residual synthetic peptides and their protein contents were confirmed by a Bradford assay **(B). (C, D)** CGIYRLRSC-targeted protein fractions are enriched in the α₆β₄ integrin. Selected fractions (2 µg each) were coated per microwell of a 96-well plate and subjected to a phage binding assay with an input of 10⁹ TU of either the insertless fd-tet or CGIYRLRSC-phage. Numbers were normalized to the degree of binding to BSA-coated microwells and are shown as fold increase **(C).** Control (salt, control peptide) and targeted (CGIYRLRSC_1, _2, and _6) protein fractions (500 ng each) were evaluated for the presence of specific integrin subunits by an ELISA assay **(D).**

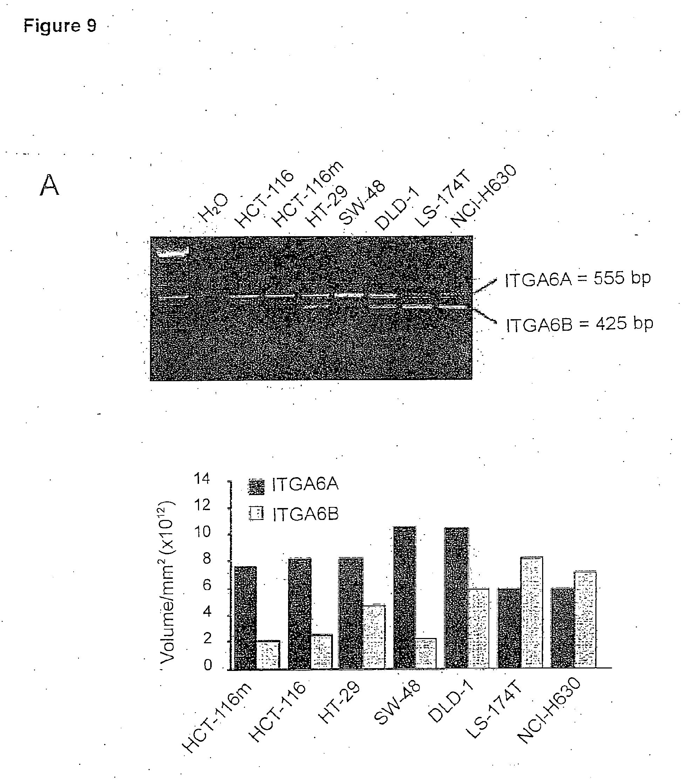

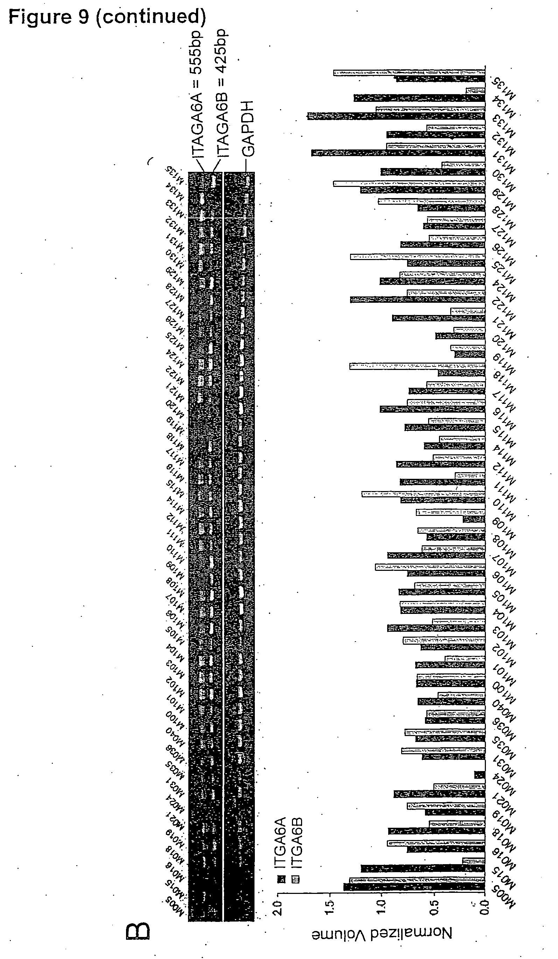

**Figure 9****: The ITGA6A isoform of α₆ integrin mRNA is predominant in CRC cell lines and in liver metastases from CRC patients**
   Total mRNA from CRC cell lines **(A)** and from samples (n=45) of human liver metastases secondary to CRC **(B)** was retrotranscribed and PCR-amplified for the evaluation of splicing isoforms of the α₆ integrin mRNA. Resulting PCR products were separated on a 2% agarose gel and the intensity of the amplified bands was quantified by densitometric analysis. Due to variability in the amounts of cDNAs retrotranscribed in samples from CRC patients, absolute values were further normalized on the levels of a housekeeping gene (Glyceraldehyde-3-phosphate dehydrogenase, GAPDH).

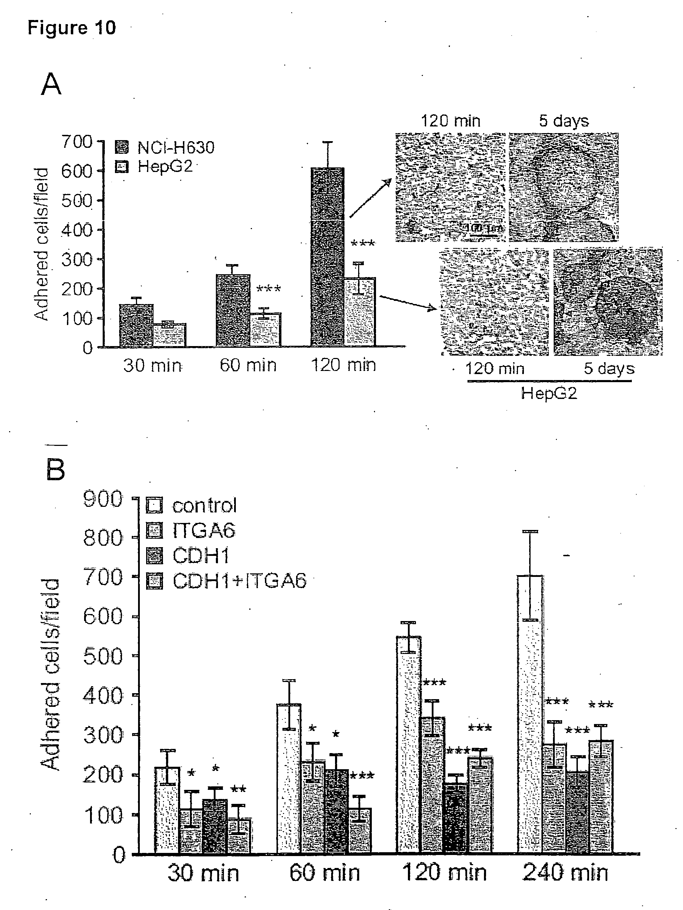

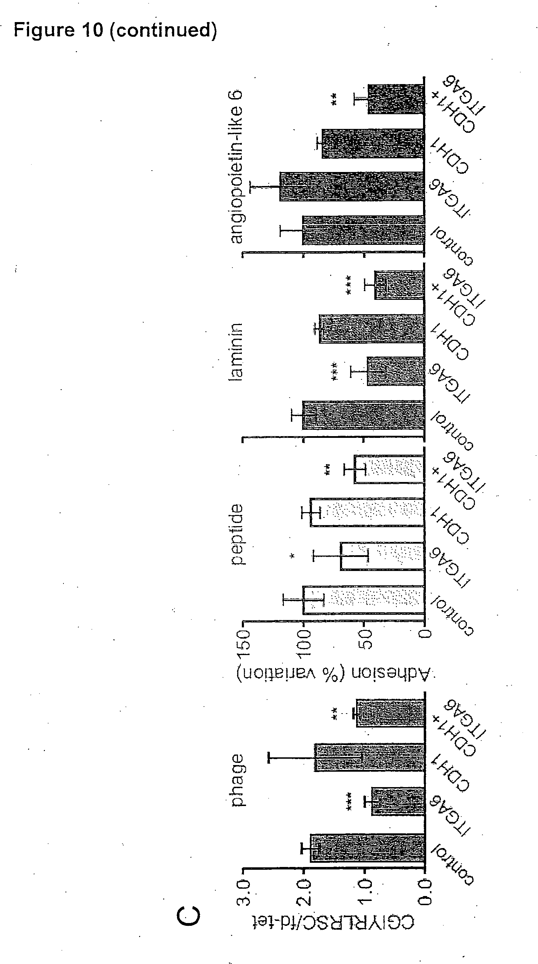

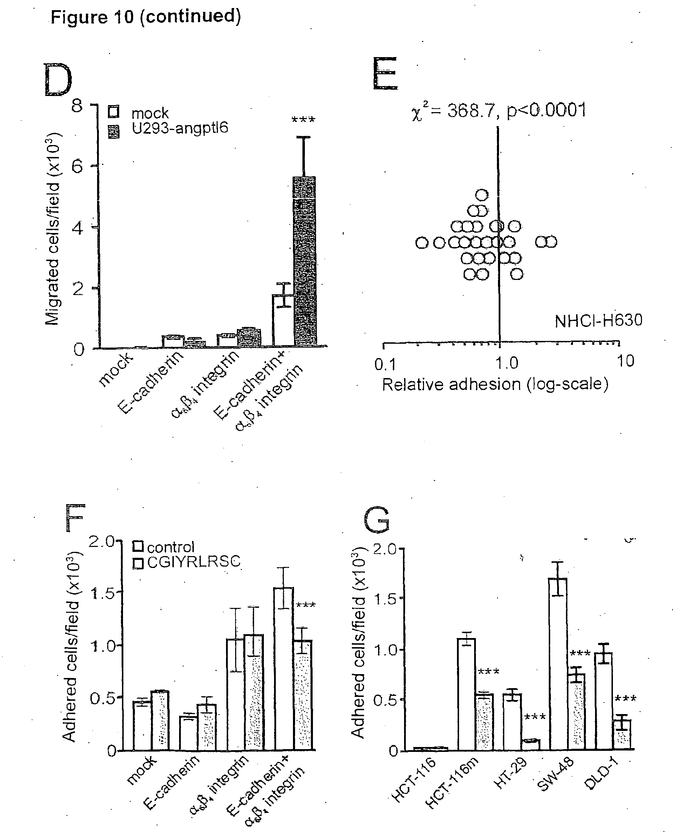

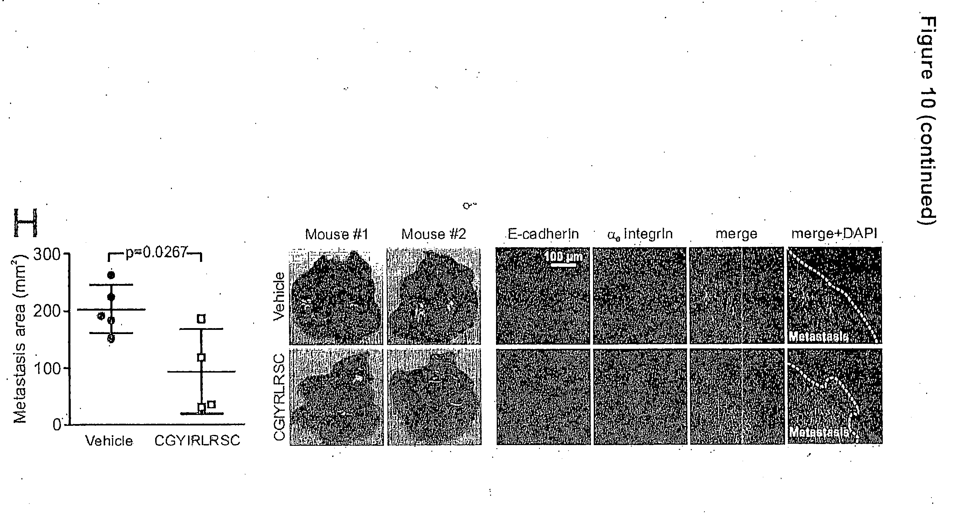

**Figure 10****: Alpha 6 integrin, E-cadherin and angiopoietin-like 6 mediate adhesion and attraction of human metastatic cells to the liver and CGIYRLRSC-peptide interferes with these functions *in vitro* and *in vivo***
   **(A, B)** The receptor side: α₆ integrin and E-cadherin are involved in the adhesion of metastatic CRC cells to the liver. HepG2 and NCI-H630 cells were incubated on 10-µm frozen sections of grossly normal human liver and adhered cells were fixed, stained and counted under a light microscope at 20x magnification. Photomicrographs representative of cell numbers (120 minutes' incubation) and morphology (5 days' incubation) are shown. The dotted line indicates a micrometastasis-like structure integrated into the hepatic tissue; arrowheads point to cell aggregates. Tissues were counterstained with hematoxylin **(A).** NCI-H630 cells silenced for *ITGA6, CDH1* or both of these mRNAs were challenged in the same assay **(B).** Results are shown as mean ± standard deviation for each experimental point in 3 independent experiments. **(C)** The ligand side: the CGIYRLRSC motif and angiopoietin-like 6 interact specifically with cells expressing the receptor proteins. Phage binding was investigated on NCI-H630 cells in which *ITGA6, CDH1* or both mRNAs were silenced. Results, normalized to fd-tet binding, are shown as mean ± standard deviation of 4 independent experiments. The same cells were evaluated for their capacity to adhere to the CGIYRLRSC peptide, laminin (as a positive control for α₆ integrin availability on the cell surface) or recombinant angiopoietin-like 6 protein. Results are shown as mean ± standard deviation for each experimental point in 3 independent experiments. **(D)** Angiopoietin-like 6 is a chemotactic factor for cells expressing the receptor proteins. U293 cells stably transduced with α₆ integrin, E-cadherin or both were co-cultured with cells producing angiopoietin-like 6. Co-cultures with mock-transfected U293 cells were exploited as a reference for basal cell motility. After 48 hours, cells on the lower side of the transwell membranes were fixed, stained and counted under a light microscope at 5x magnification. Results are shown as mean ± standard deviation for each experimental point in 2 independent experiments. **(E-G)** CGIYRLRSC-peptide inhibits liver adhesion of cells expressing the receptor proteins. For liver adhesion assays, NCI-H630 cells **(E),** U293 cells transduced with α₆ integrin, E-cadherin or both **(F)** and primary CRC cell lines **(G)** were incubated on 10-µm frozen sections of grossly normal human liver in the presence of either CGIYRLRSC or control peptide; after 30 minutes, adhered cells were fixed, stained and counted under a light microscope at 5x magnification. For NCI-H630, adhesion was evaluated on liver sections from 27 patients (Patient Ids: P29, P30, P33, P34, P35, P36, P56-76) and results are shown as the ratio of attached cells in the presence of CGIYRLRSC and control peptide. In **A-D, F** and G, differences in the experimental points were evaluated for their statistical significance by the use of ANOVA followed by Bonferroni's post-test. In **E,** a Chi-squared test was used to evaluate whether values significantly differed from the reference ratio = 1. **(H)** CGIYRLRSC-peptide interferes with liver colonization *in vivo.* LS-174T cells were injected intrahepatically into CD-1 nude mice (5x10⁶ cells/mouse), either in medium alone (vehicle) or in the presence of the soluble peptide (CGIYRLRSC). Fourteen days after surgery, animals were sacrificed and livers were explanted and photographed for the quantification of external tumor areas. Representative photomicrographs of whole livers from two mice/group are shown for macroscopic evaluation of tumor morphology; the indicated p-value is referred to statistical analysis performed with Fisher's exact test. Sample tissues were OCT-frozen, cut in 10-µm sections and subjected to immunostaining with anti-α₆ integrin and anti-E-cadherin antibodies followed by confocal microscopic imaging. E-cadherin is shown in green, α₆ integrin in red and co-localization is indicated by the yellow color. Confocal images were acquired with all the parameters constant; exemplary pictures of samples from one mouse/group are shown.

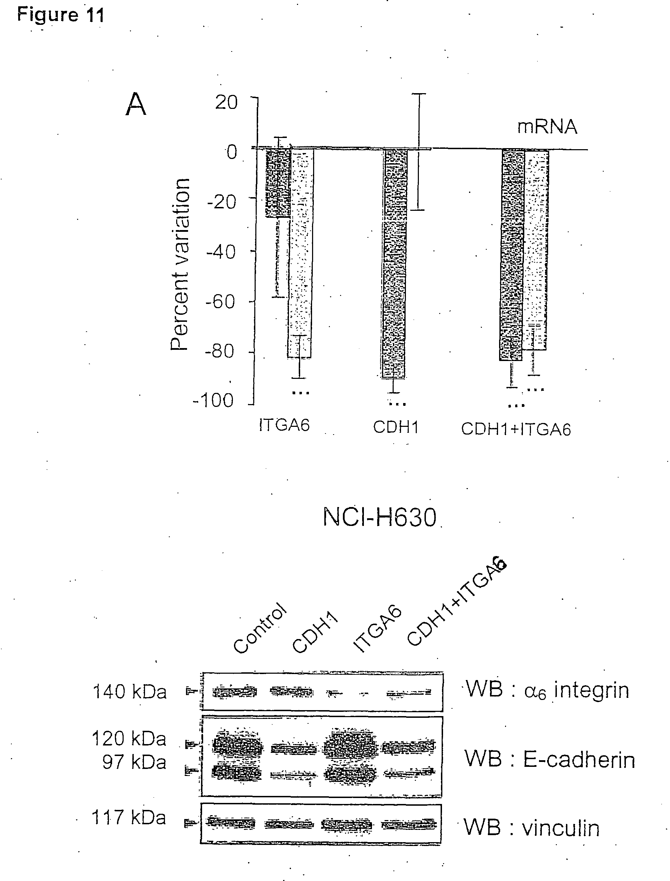

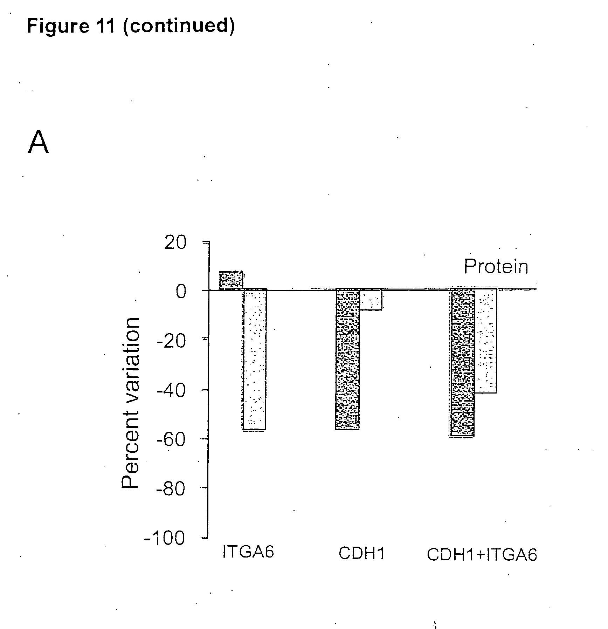

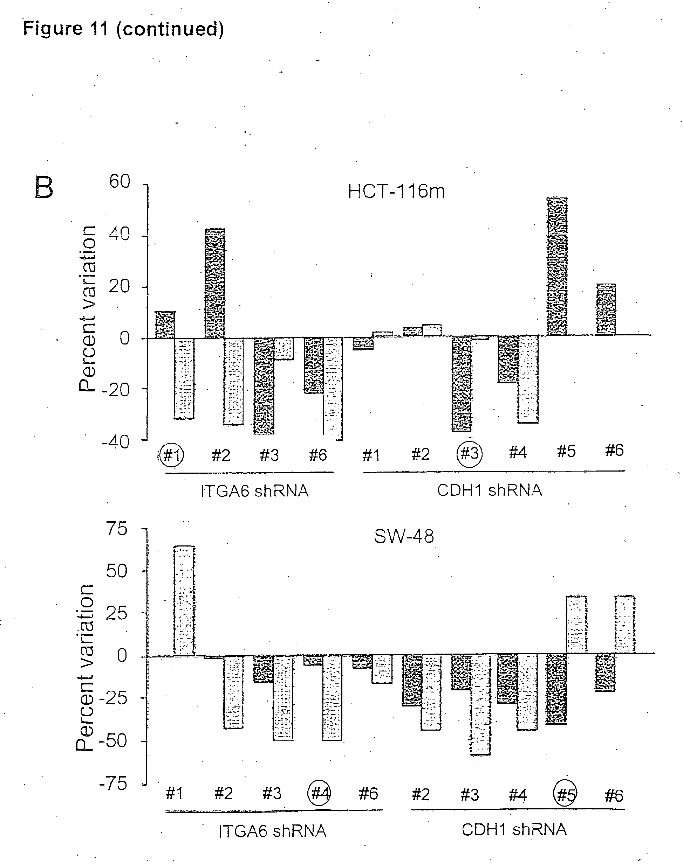

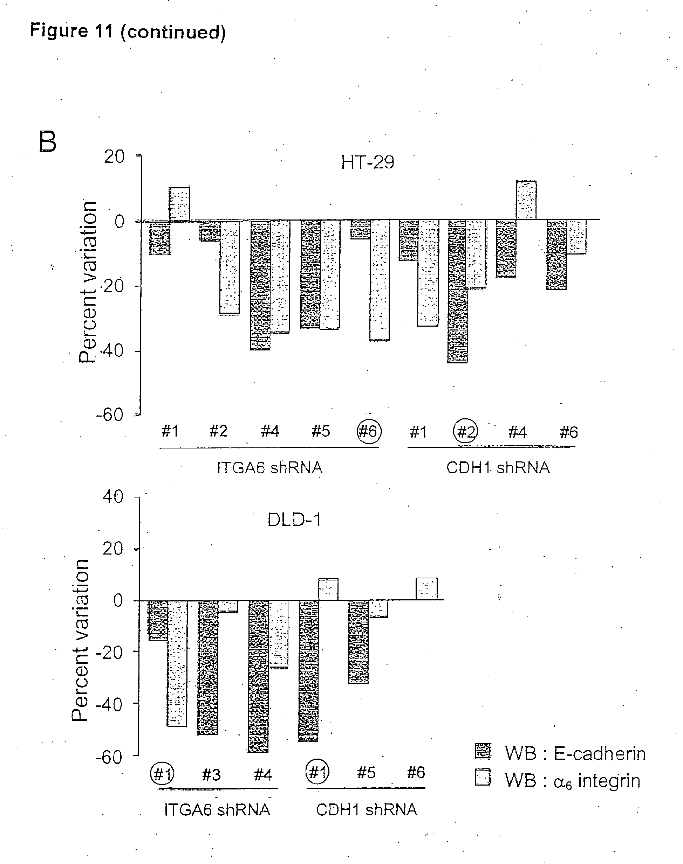

**Figure 11****: Validation of α₆ integrin and E-cadherin mRNA and protein levels in transiently- and stably-silenced cells**
   (A) Quantification of specific mRNA and protein levels in NCI-H630 cells transiently silenced for the expression of E-cadherin and α₆ integrin mRNAs. Cells were reverse-transfected with siRNA pools for ITGA6, CDH1 or both mRNAs. Transfection with a non-targeting siRNA pool was performed as a control. Messenger RNA amounts were evaluated after 24 hours by retrotranscription and Real Time PCR amplification of the specific cDNAs. A reduction of 75-85% in both mRNA levels was observed. Results are shown as mean ± standard deviation of 9 independent transfections; differences in the experimental points were evaluated for their statistical significance by ANOVA followed by Bonferroni's post-test. Protein amounts were evaluated after 72 hours by Western Blot. A reduction of 60-70% in both protein levels was observed. Vinculin staining was used as a loading control. **(B)** Quantification of specific protein levels in CRC cell lines stably silenced for the expression of E-cadherin and α₆ integrin mRNAs. HCT-116m, HT-29, SW-48 and DLD-1 cells were transfected with shRNA plasmid pools targeting ITGA6 or CDH1; non-targeting control shRNA plasmid pool A was used as a negative control. Following antibiotic selection, 6 clones for each experimental point were analyzed by dotblot immunostaining to confirm a specific protein down-regulation. Red circles indicate clones selected for successive experiments, in which a reduction of 30-60% in either protein levels was observed.

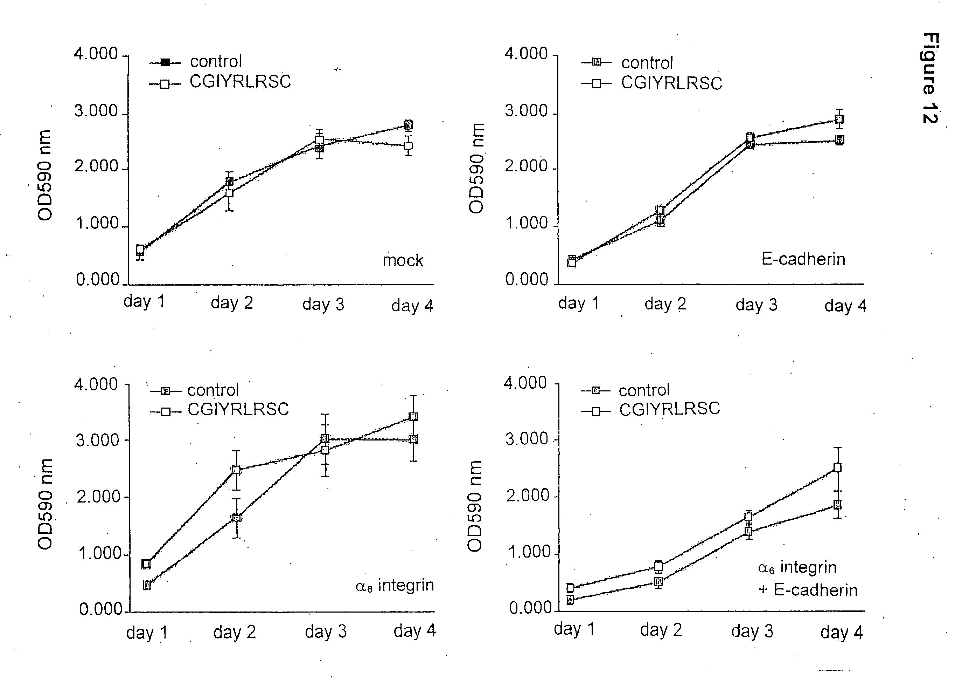

**Figure 12****: CGIYRLRSC does not influence the proliferation of cells expressing the receptor complex**
   U293 cells stably overexpressing E-cadherin, α₆ integrin or both were grown in complete (10% FCS) culture medium in the presence of either the control or CGIYRLRSC peptide. At 24 hour time points, cells were fixed and stained with crystal violet; their numbers were estimated by spectrophotometric evaluation. Results are shown as mean ± standard deviation for each experimental point in 2 independent experiments. ANOVA analysis of the data revealed no statistical significance.

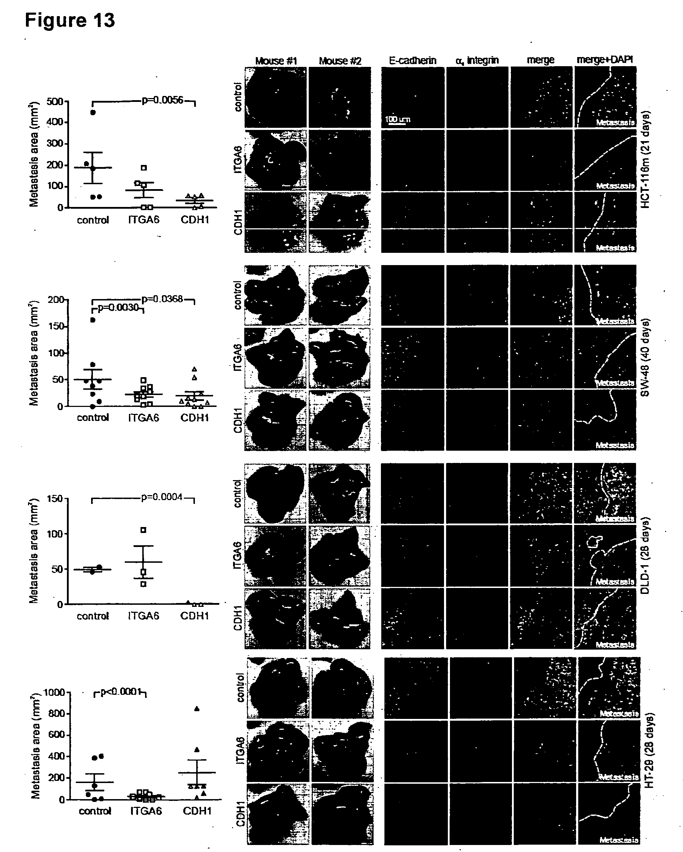

**Figure 13****: Decreased expression of α₆ integrin or E-cadherin results in impaired liver colonization by a panel of CRC cell lines *in vivo***
   HCT-116m, SW-48, DLD-1 and HT-29 cell lines with a decreased expression of either α₆ integrin or E-cadherin (see Figure 11 B) were injected intrahepatically into CD-1 nude mice (5x10⁶ cells/mouse). At the indicated time points, mice were euthanized and their livers were explanted and photographed for the quantification of external tumor areas. Representative pictures of whole livers from 2 mice/group are shown for macroscopic evaluation of tumor morphology; the indicated p-values are referred to statistical analysis performed either with Fisher's exact test (in black) or t-test (in red). Sample tissues were OCT-frozen, cut in 10-µm sections and immunostained with anti-α₆ integrin and anti-E-cadherin antibodies, followed by imaging with a confocal microscope. In these analyses, acquisition parameters were held constant to allow comparison of the absolute amounts and locations of α₆ integrin and E-cadherin among the different samples. E-cadherin is shown in green, α₆ integrin in red and co-localization is indicated by the appearance of the yellow color. Exemplary pictures of samples from one mouse/group are shown.

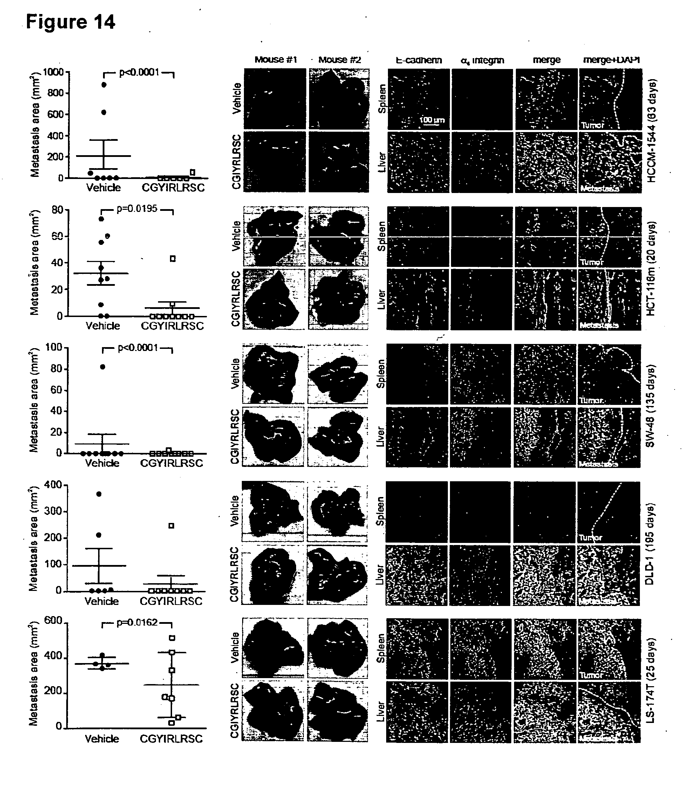

**Figure 14****: Metastasis-targeted therapy: CGIYRLRSC inhibits homing of CRC cells to the liver**
   To obtain an *in vivo* model of metastatic CRC, we implanted the patient-derived HCCM-1544 tumor as well as different CRC cell lines (HCT-116m, SW-48, DLD-1 and LS-174T) intrasplenically into CD-1 nude mice (2x10⁶/mouse). To evaluate the effect of CGIYRLRSC on liver metastasis, we injected cells either in medium alone (vehicle) or in the presence of the soluble peptide (CGIYRLRSC). At the indicated time points, mice were euthanized and their livers and spleens were explanted. Livers were photographed for the quantification of external metastatic areas. Representative pictures of whole livers from 2 mice/group are shown for macroscopic evaluation of tumor morphology; the indicated p-values are referred to statistical analysis performed either with Fisher's exact test (in black) or t-test (in red). Spleen (primary tumor) and liver (metastasis) samples were OCT-frozen, cut in 10-µm sections and immunostained with anti-α₆ integrin and anti-E-cadherin antibodies, followed by imaging with a confocal microscope. As described in Figure 13, acquisition parameters were held constant to allow comparison of the absolute amounts and locations of α₆ integrin and E-cadherin among different samples. E-cadherin is shown in green, α₆ integrin in red and co-localization is indicated by the appearance of the yellow color. Exemplary pictures of samples from one mouse/group are shown.

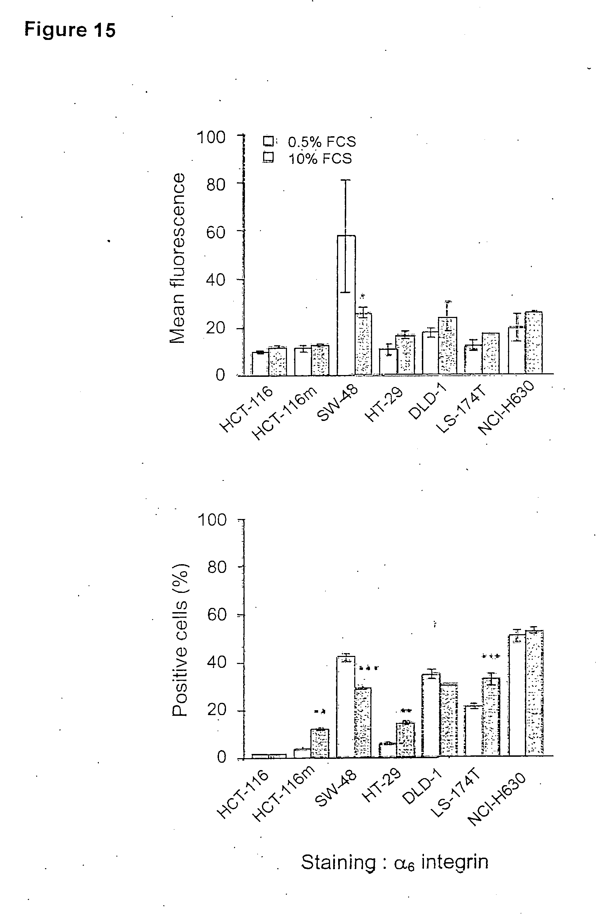

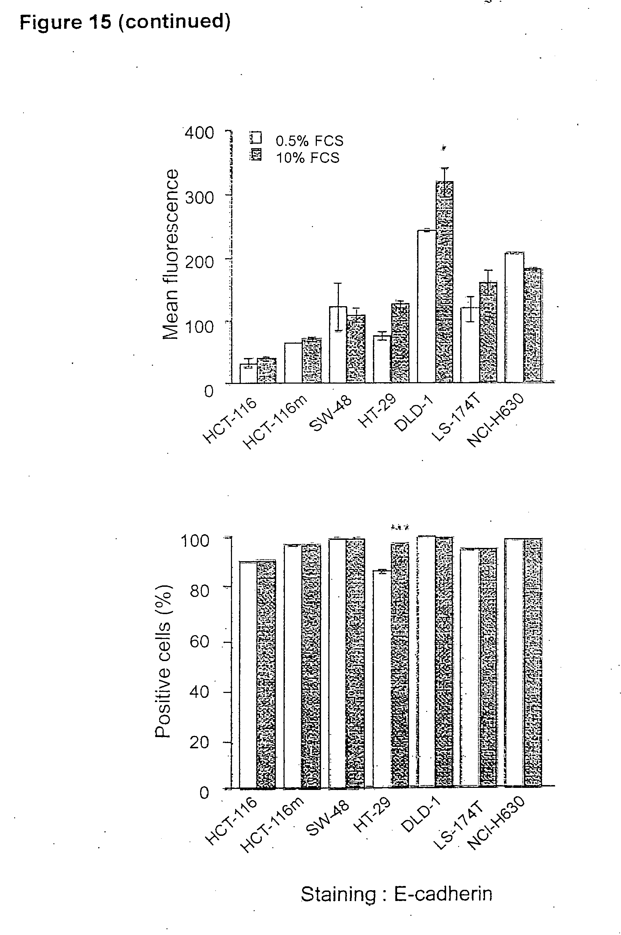

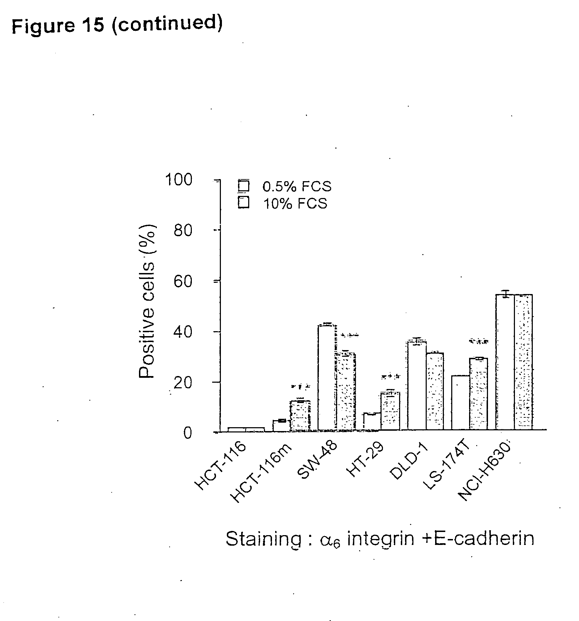

**Figure 15****: A subpopulation of CRC cells express α₆ integrin and the α₆ integrin/E-cadherin complex, in variable amounts depending on the culture conditions**
   Human cell lines derived both from primary CRCs (HCT-116 and its derivative HCT-116m, SW-48, HT-29, DLD-1, LS-174T) and from a liver metastasis secondary to CRC (NCI-H630) were grown in complete (10% FCS) or serum-deprived (0.5% FCS) culture medium for 48 hours, followed by staining with anti-α₆ integrin and anti-E-cadherin antibodies. Results of the cytofluorimetric analyses (fluorescence intensity and percent of positive cells) are shown as mean ± standard deviation for each experimental point in 3 independent experiments. Differences in the experimental points were evaluated for their statistical significance by the use of ANOVA followed by Bonferroni's post-test.

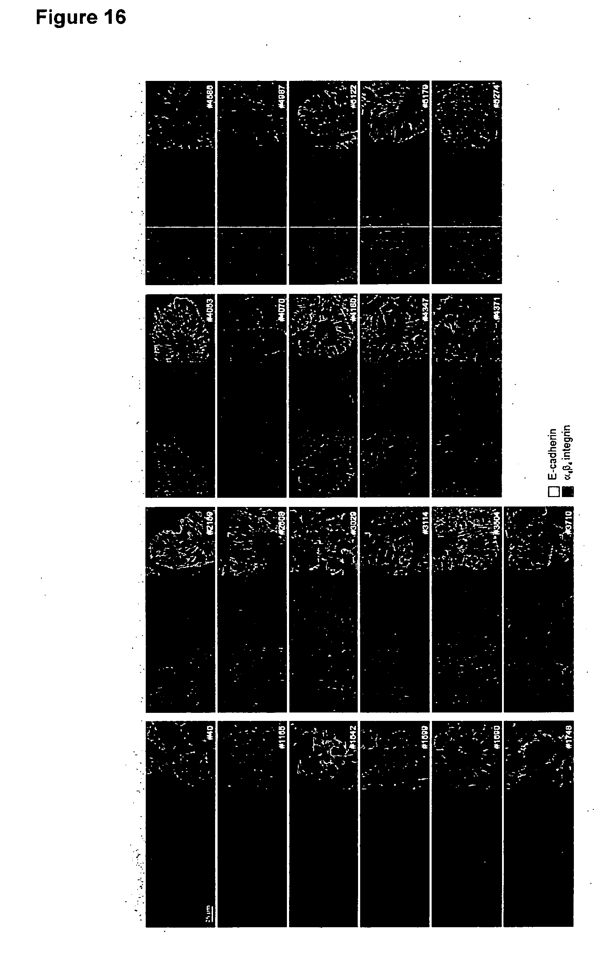

**Figure 16****: Quantification of α₆ integrin, E-cadherin and their molecular complex in metastatic CRCs (primary tumor)**
   The location of α₆ integrin and E-cadherin was evaluated on 5µm sections of paraffin-embedded primary CRCs by staining with specific antibodies followed by confocal microscope imaging. For the quantification of the fluorescent signals (illustrated in Figure 19), 3 confocal images (1024x1024 pixels, equivalent to 375x375 µm) for each sample were acquired keeping all the parameters constant and divided in two 8-bit images corresponding to the red and green fluorescence channels. These image pairs were analyzed with ImageJ, using the Colocalization Highlighter plugin to create a binary representation of co-localized pixels (yellow pixels in the merged images) and the Image Calculator option to derive the non-co-localized pixels (red or green pixels in the merged images).

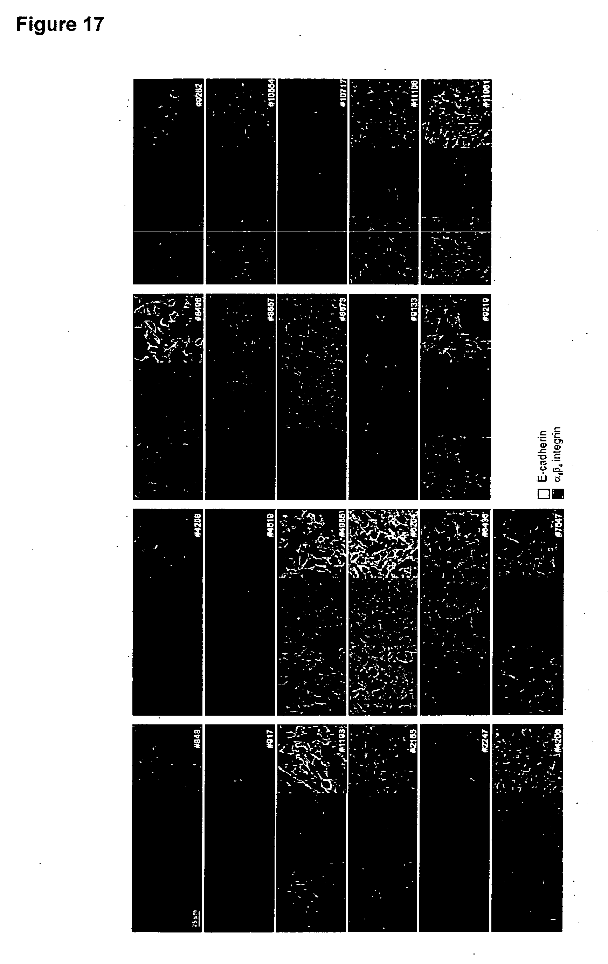

**Figure 17****: Quantification of α₆ integrin, E-cadherin and their molecular complex in different metastatic cancers (liver metastasis)**
   The location of α₆ integrin and E-cadherin in paraffin-embedded liver metastases from different primary tumors was evaluated as described in Figure 16 and in Figure 19.

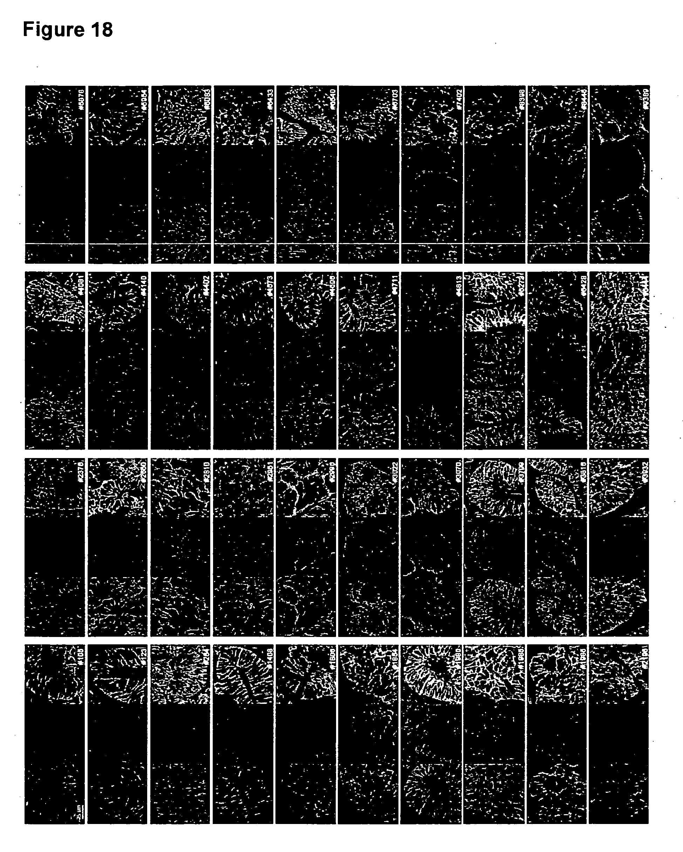

**Figure 18****: Quantification of α₆ integrin, E-cadherin and their molecular complex in metastatic CRCs (lung metastases)**
   The location of α₆ integrin and E-cadherin in paraffin-embedded lung metastases from CRCs was evaluated as described in Figure 16 and in Figure 19.
**Figure 19****: Alpha 6 integrin, E-cadherin and their molecular complex are present in advanced CRCs and in liver metastases of different tumors**
   The presence of α₆ integrin and E-cadherin was evaluated by immunostaining of 5-µm sections of a large panel of paraffin-embedded cancer tissues **(A,** primary CRCs; **B,** Liver metastases from CRCs; **C,** liver metastases from other primary tumors, the origin of which is indicated in the table; and **D,** lung metastases from CRCs). For the quantification of specific fluorescent signals, 3 confocal images (1024x1024 pixels, equivalent to 375x375 µm) for each sample were divided in two 8-bit images corresponding to the red and green fluorescence channels. These image pairs were analyzed with ImageJ, with the Colocalization Highlighter plugin to create a binary representation of co-localized pixels (yellow pixels in the merged images) and the Image Calculator option to derive the non-co-localized pixels (red or green pixels in the merged images). See Figures 4 and 16-18 for the visualization of corresponding confocal microscopic images.

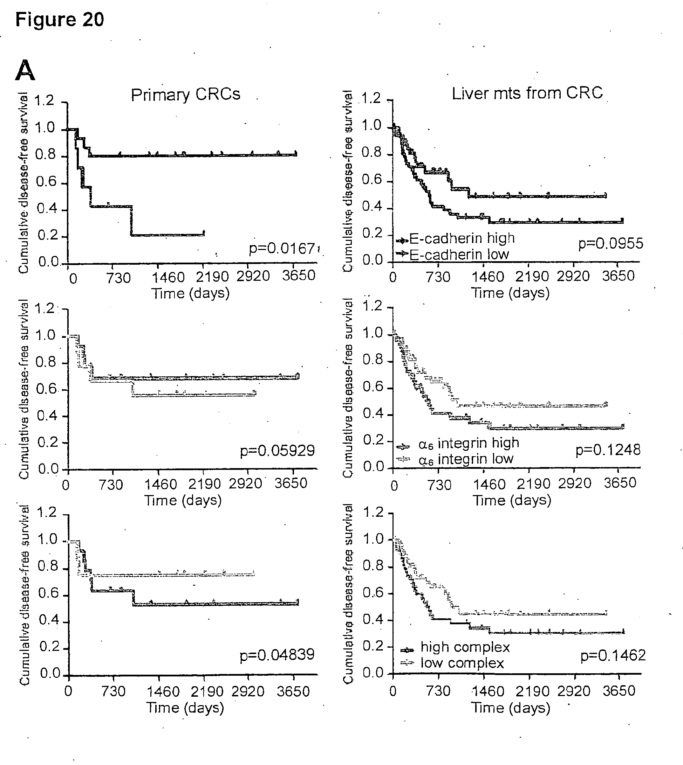

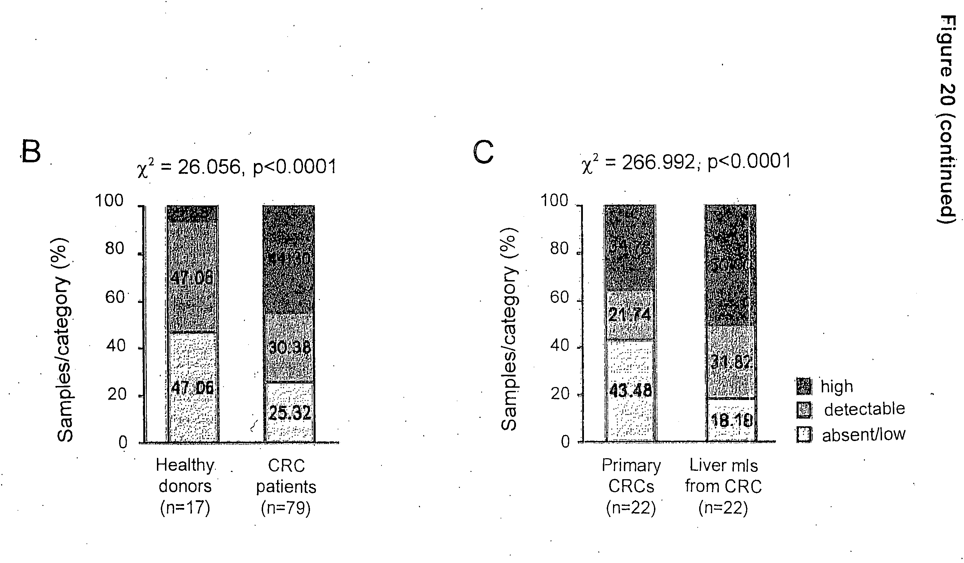

**Figure 20****: The expression levels of α₆ integrin, E-cadherin, their coincidence and angiopoietin-like 6 correlate with clinical parameters in patients with metastatic CRC**
   **(A)** The presence of the α₆ integrin/E-cadherin molecular complex is a poor prognostic factor for patients with metastatic CRC. The amounts of α₆ integrin, E-cadherin and their coincidence in primary CRCs and liver metastases from CRCs (quantified as described in Figure 19) were correlated with disease-free survival. Survival curves were drawn as Kaplan-Maier Cumulative Proportion Surviving graphs and corresponding p-values were calculated by the use of the log-rank (Mantel-Cox) test with Prism 5 GraphPad software. **(B, C)** Angiopoietin-like 6 is accumulated in blood vessels of the liver in CRC patients compared to healthy donors and its expression is increased in liver metastases compared to primary CRCs. Paraffin-embedded liver samples from healthy donors (for transplantation) and from CRC patients **(B)** and matched primary adenocarcinomas/liver metastases of CRC patients **(C)** were cut in 5-µm sections and immunostained with the anti-angiopoietin-like 6 antibody (see Figures 5 and 21 for corresponding images). Two independent researchers assigned each sample an intensity score for specific staining of angiopoietin-like 6 (absent/low, detectable, high) in hepatic vessels and in cells of primary and metastatic CRCs and the differences between sample categories were evaluated by a Chi-squared test.
**Figure 21****: Angiopoietin-like 6 expression is increased in liver metastases compared to matched primary CRCs**
   The amounts of angiopoietin-like 6 were evaluated by staining of 5 µm tissue sections of primary CRC and liver metastasis from the same patients (n=22). Pictures of 20 samples for each tissue type are shown.

### Examples

### Methods

Antibodies, recombinant proteins, and synthetic peptides. Goat polyclonal anti-α₆ integrin N-19 (used for immunoblot) (sc-6597) and anti-vinculin N-19 (sc-7649), rabbit polyclonal anti-β₄ integrin H-101 (used for ELISA) (sc-9090), and horseradish peroxidase (HRP)-conjugated donkey anti-goat IgG (sc-2033) were from Santa Cruz Biotechnology (Santa Cruz, CA). Mouse monoclonal anti-α₆ integrin clone BQ16 (used for immunoprecipitation) was from Calbiochem (San Diego, CA). Rat monoclonal anti-α₆ integrin clone GoH3 (used for immunostaining) was from AbD Serotec (Raleigh, NC). Mouse monoclonal anti-β₄ integrin clone 7 (used for immunoblot) and anti-E-cadherin clone 36 were from BD Transduction Laboratories (Franklin Lakes, NJ). Mouse monoclonal anti-β₁ integrin clone P4G11 was from Chemicon (Millipore, Billerica, MA). Rabbit polyclonal anti-fd bacteriophage (B-7786) was from Sigma (St. Louis, MO). Alexa Fluor 488 anti-rat IgG and 555 anti-mouse IgG were from Invitrogen (Carlsbad, CA). HRP-conjugated donkey anti-mouse IgG was from Jackson ImmunoResearch (West Grove, PA). Mouse monoclonal anti-CD31 clone JC70A and HRP-conjugated anti-rabbit EnVision were from DAKO (Glostrup, Denmark). Rabbit polyclonal (used for immunostaining) and mouse monoclonal clone Kairos-60 (used for immunoblot) anti-angiopoietin-like 6, and recombinant angiopoietin-like 6 were from Alexis Biochemicals (Enzo Life Sciences, Farmingdale, NY). Rabbit polyclonal anti-PRL3 (62) was a gift of Dr. Alberto Bardelli (Institute for Cancer Research and Treatment, Candiolo, Italy). Laminin (L-2020) was from Sigma. Targeting (CGIYRLRSC) and control (CARAC) peptides were from New England Peptides (Gardner, MA).

Cell lines and human samples. SW620 (ATCC CCL-227), NCI-H630 (ATCC CRL-5833), HepG2 (ATCC HB-8065), NCI-N87 (ATCC CRL-5822), A549 (ATCC CCL-185), HCT-116 (ATCC CCL-247), HT-29 (ATCC HTB-38), DLD-1 (ATCC CCL-221 ), SW-48 (ATCC CCL-231), LS-174T (ATCC CL-188), and U293 (ATCC CRL-1573) cell lines were from LGC-Promochem (Sesto San Giovanni, Italy), and were cultured according to the purchaser's instructions. The HCCM-1544 human metastatic CRC and corresponding in vitro and in vivo manipulations have been previously described (Tibbetts et al. 1993. Cancer, 71:315-321). A variant of HCT-116, selected *in vivo* for its ability to metastasize to the liver in pseudo-orthotopic models (here named HCT-116m), was provided by Dr. Alberto Bardelli. Fresh (grossly normal livers from CRC patients, primary CRCs, liver metastases secondary to CRC) and paraffin-embedded (grossly normal livers from CRC patients, primary CRCs, liver metastases of various origins) human specimens were collected by the Units of Surgical Oncology and of Pathology at the Institute for Cancer Research and Treatment. Paraffin-embedded human specimens of normal liver from healthy donors, of lung metastasis secondary to CRC, and of different healthy tissues were collected by the Unit of Pathology at the Molinette Hospital (Turin, Italy). Snap-frozen samples of lung metastasis secondary to CRC and liver metastasis secondary to renal cancer were from San Luigi Gonzaga Hospital (Orbassano, Italy); snap-frozen samples of ovarian cancer and matched metastasis were from Mario Negri Institute (Milan, Italy). Collection and manipulation of human samples were approved by the Institutes' Ethical Committees, and written informed consent was obtained from all patients in accordance with the Declaration of Helsinki. Biopanning of human samples with phage display. Fresh tissue samples were dissected with a scalpel and digested with 0.025% collagenase A (Roche Diagnostics, Monza, Italy) in Iscove's Modified Dulbecco Minimum Essential Medium (IMDM) for 2 hours at 37°C with vigorous shaking. The resulting suspension was passed through a 40 µm nylon cell strainer (BD Labware, Franklin Lakes, NJ), and cells were resuspended in binding medium (IMDM supplemented with 2% Fetal Calf Serum, FCS). With this protocol, we did not select for tumor epithelial cells; instead, we aimed at retaining the original mixed cell population (consisting mostly of epithelial, endothelial, and hematopoietic cells, and fibroblasts), as well as the tissue stroma. To preserve their phenotype, we avoided cell culture and maintained these cells in suspension in binding medium at 4°C for the duration of the experiments. 1010 transducing units (TU) of a CX7C, CX9C, or CX3CX3CX3C phage library was added to 5x105 liver metastasis cells in binding medium and cells were incubated overnight (first round). For successive rounds, phage was first pre-adsorbed on normal liver cells for 1 hour at 4°C and was subsequently incubated with liver metastasis cells for 2 hours at 4°C. Cells were washed 5 times with binding medium, and bound phages were recovered and amplified by infection of K91 Kan *Escherichia coli* bacteria in log-phase. Purification of phage particles and DNA sequencing of phage-displayed inserts were performed as described (Scott et al. 1990. Science, 249:386-390; Smith et al. 1993. Methods Enzymol 217:228-257).

Bioinformatics. The protein BLAST tool (www.ncbi.nlm.nih.gov/BLAST) was used to investigate similarities between MTS peptides and the human proteome. Due to the short length of such peptide sequences, consequent high numbers of false positive similarities would lead to result misinterpretations. The following constraints were introduced to our search: (i) at least 3 consequent amino acids of the MTS peptide motif should be identical to that of the BLAST match; (ii) each output protein should share ≥ 3 similarity matches (i.e. should be similar to at least 3 MTS peptides or to an MTS peptide in at least 3 different regions). Peptides in the reverse orientation are expected to have significantly lower probability of mimicking natural proteins; therefore, the MTS_REV dataset was used as a further internal control for the BLAST searches. GO annotations were retrieved through the DAVID Bioinformatics Resource Functional Annotation tool (http://david.abcc.ncifcrf.gov; Huang et al. 2009. Nat Protoc 4:44-57; Huang et al. 2009. Nucleic Acids Res 37:1-13), searching for GOTERM_CC_ALL, with default program settings. GO-CC-enriched categories were associated with corresponding statistical significance, represented as a Benjamini-Hochberg-corrected p-value.

Isolation and identification of MTS-targeted proteins. The following oligonucleotides were annealed and inserted into pGEX-4T.1 between *BamHI* and *NotI* sites to create the pGEX-4T.1-CGIYRLRSC plasmid:
5'-GATCCGGAGCCTGTGGAATATATAGATTAAGAAGTTGTGCGGGCGC-3' (SEQ ID No 26) and
5'-GGCCGCGCCCGCACAACTTCTTAATCTATATATTCCACAGGCTCCG-3' (SEQ ID No 27).

The corresponding fusion peptide was purified to homogeneity from BL-21 *Escherichia coli* cell lysates by affinity chromatography on glutathione-sepharose beads (GE Healthcare), according to the manufacturer's protocol. HepG2 and NCI-H630 cells were lysed in 50 mM Tris-HCl (pH 7.4), 150 mM NaCl, 0.1 % NP-40, 10% glycerol, and a protease inhibitor cocktail (SIGMA-Aldrich). 10 milligrams of total protein was pre-cleared on GST-Sepharose (GE Healthcare) prior to incubation with CGIYRLRSC-GST-Sepharose (4 µg peptide/mg total protein) overnight at 4°C. Bound proteins were eluted from Sepharose beads, separated on a 10% SDS-polyacrylamide gel, and stained with BioSafe Coomassie blue (BioRad). Specific bands were analyzed by mass spectrometry as described (Paget, 1989. Lancet, 1:571-573.). Matrix-assisted laser desorption/ionization (MALDI) mass spectra were recorded on an Applied Biosystems Voyager DE-PRO mass spectrometer equipped with a reflectron time-of-flight (TOF) analyzer and used in delayed extraction mode (Applied Biosystems, Foster City, CA). Raw data, reported as monoisotopic masses, were introduced into the MASCOT peptide mass fingerprinting search program (Matrix Science, Boston, MA) for protein identification. Liquid chromatography (LC)-mass spectrometry (MS)/MS analyses were performed on a CHIP MS Ion Trap XCT Ultra equipped with a 1100 high pressure liquid chromatography (HPLC) system and a chip cube (Agilent Technologies, Palo Alto, CA). Peptide analysis was performed by data-dependent acquisition of one MS scan (mass range from 400 to 2000 m/z) followed by MS/MS scans of the three most abundant ions in each MS scan. Raw data from nanoLC-MS/MS analyses were introduced into the MASCOT software to search the human proteome.

Validation of MTS-phage targets. Binding of single phage clones on whole cells was performed with a 10⁹ TU input of each phage on 5x10⁵ suspended cells in binding medium as described (Chambers et al. 2002. Nat Rev Cancer, 2:563-572). For overlay binding experiments, 5 x10⁹ TU/ml of each phage was incubated with 10 µm tissue sections of OCT-frozen tissues and detected as described , with the EnVision system (DAKO) and 3-amino-9-ethylcarbazole (AEC) as substrate (Arap et al 2002, Nat Med, 8:121-127; Padua et al. 2008, Cell, 133:66-77). Phage overlay images were acquired with an EC3 Leica camera (Leica Microsystems, Milan, Italy).
For the characterization of proteins targeted by CGIYRLRSC, 5 mg of synthetic peptide was immobilized on column-packed diaminodipropylamine-agarose (CarboxyLink Kit, Pierce, Euroclone, Milan, Italy) according to the manufacturer's protocol. After equilibration in PBS, the column was loaded with 10 mg of total protein from 7 pooled human samples of liver metastasis secondary to CRC, allowing recirculation for 30 minutes at 4°C. The column was washed with 10 ml of column buffer (PBS, 1 mM CaCl₂, 1 mM MgCl₂, 50 mM β-octyl-D-glucosylpyranoside, 1 mM PMSF and protease inhibitor cocktail), followed by salt elution of unspecific proteins in column buffer supplemented with 50 mM NaCl. Control and target protein elution was obtained with 2 mM of the control and CGIYRLRSC peptide, respectively, and the column was finally cleared with 0.1 M NaCl, 0.1 M Glycine pH 3.00. Protein amounts in collected fractions were followed by reading their OD at 280 nm, and selected fractions were concentrated by the use of Microcon centrifugal filter devices with cut-off 10,000 (Millipore) to remove residual synthetic peptides. Proteins were quantified with the Coomassie (Bradford) Protein Assay Kit (Pierce), and 500 ng of each sample was evaluated for the presence of specific integrin subunits with a standard ELISA assay. In parallel, the relative amount of targeted proteins was assessed by phage binding as described (66), on 2 µg of each sample and with an input of 10⁹ TU of fd-tet or CGIYRLRSC-phage. Binding to BSA-coated microwells was used for normalization.

Preparation of cells stably overexpressing the MTS-targeted proteins. The cDNA of FLAG-tagged human angiopoietin-like 6, inserted into pcDNA3.1 (+).Neo vector (pcDNA3.ANGL6), was a gift from Dr. Y. Oike (Japan Science and Technology Agency, Japan (Minn et al., 2007, Proc Natl Acad Sci USA, 104:6740-6745); the cDNA of human E-cadherin, cloned into a pcDNA3.1 (+). Neo vector (pcDNA3.CAD1) was a gift from Dr. C. Gottardi (North Western University Medical School, Chicago, IL (Bos, et al. 2009, Nature 459:1005-1009); the cDNA of β₄ integrin, cloned into a PRK5 plasmid (pRK5.ITB4), was purchased from Addgene (Cambridge, MA); the cDNA of human α₆A integrin, inserted in pLXSN plasmid (pLα6SN (Kuo et al. 1995, Proc Natl Acad Sci USA, 92:12085-12089), was a gift of Dr. A. Magrelli (La Sapienza University, Rome, Italy). The latter was PCR-amplified with the following primer pair:
5'-AAACTTAAGCTTGCCACCATGGCCGCCGCCGGGCAG-3' (SEQ ID No 28) and
5'-TACACGGGCCCTCTATGCATCAGAAGTAAGCCT-3' (SEQ ID No 29)
and subcloned into a pcDNA3.1 (+). Hygro vector between *HindIII* and *ApaI* sites to obtain the pcDNA3.ITA6A plasmid. To stably overexpress of α₆β₄ integrin, E-cadherin, and angiopoietin-like 6, U293 cells were transduced with the described plasmids by the use of a calcium phosphate transfection kit (Invitrogen), followed by selection of single cell clones in culture media supplemented by geneticin (500 µg/ml) and/or hygromicin (200 µg/ml).

Preparation of cell lines with silenced expression of the MTS-tageted genes. A transient gene silencing approach was applied to cells used exclusively for short-term, *in vitro* experiments. NCI-H630 cells were transduced with ON-TARGETplus SmartPOOL siRNA for *ITGA6* and *CDH1,* or with control siRNA (Dharmacon, Lafayette, CO). For each experimental point, 2x10⁵ cells were reverse-transfected with either control, *ITGA6, CDH1,* or both siRNA pools, according to the manufacturer's protocol. To quantify the downmodulation of targeted genes, RNA and protein levels were evaluated after 24 and 72 hours, respectively. A stable gene silencing approach was preferred for cells used for long-term, *in vivo* experiments. For this purpose, 2x10⁵ HCT-116m, SW-48, HT-29, or DLD-1 cells were transfected with shRNA plasmid pools targeting *ITGA6* (sc-43129-SH) or *CDH1* (sc-35242-SH), or with non-targeting control shRNA plasmid pool A (sc-108060) (all from Santa Cruz Biotechnologies), according to the manufacturer's protocol. Following selection in medium supplemented with 2.5 µg/ml puromycin, 6 clones for each experimental point were subjected to dotblot analysis to confirm selective protein down-regulation. For this purpose, cell lysates (1 µg each) were spotted onto PVDF membranes; after drying, membranes were subjected to specific antibody staining with standard procedures.

Retrotranscription, real-time PCR and end-point PCR. RNA was retrotranscribed using the High Capacity cDNA Reverse Transcription Kit (Applied Biosystems) and was amplified with the Power SYBR Green PCR Master Mix (Applied Biosystems). For quantification of residual transcripts in silenced cells, the following primer pairs were used for real-time PCR amplification of the cDNAs in an ABI PRISM 7700 instrument:
*ITGA6:* 5'-TGAGTGTCCCCCGGTATCTTC-3' (SEQ ID No 30) and
   5'-CAGTATCAGCCGCTTTCAGATTTT-3' (SEQ ID No 31);
*CDH1:* 5'-GCTGGTTATAATCCTTCAATATCAATTGT-3' (SEQ ID No 32)
   and
   5'-TTGGGCTCAGAACCTTGGTTT-3' (SEQ ID No 33);
*GAPDH:* 5'-GAAGGTGAAGGTCGGAGTC-3' (SEQ ID No 34) and
   5'-GAAGATGGTGATGGGATTTC-3' (SEQ ID No 35).

To evaluate the presence of different splicing forms of α₆ integrin, cDNAs from cell lines and from biopsies of human liver metastases secondary to CRC were subjected to end-point PCR amplification with the following primer pair:
3'-GACTCTTAACTGTAGCGTGA-5' (SEQ ID No 36) and
3'-ATCTCTCGCTCTTCTTTCCG-5' (SEQ ID No 37).

Immunostaining. OCT-frozen tissues were cut in 10 µm sections, paraffin-embedded tissues in 5 µm sections. For immunostaining of cell lines, 10⁴ cells were plated on a SuperFrost Plus glass slide (Menzel-Glaser, Braunschweig, Germany) and were grown for 24 hours followed by fixation in 4% paraformaldehyde in PBS for 5 minutes at room temperature. Immunostaining was performed according to standard protocols. Fluorescent images were acquired with either a DMIRE2 confocal microscope or with a DMI 3000D microscope equipped with a DFC 360FX digital camera (all from Leica. Visible images were acquired with either an EC3 Leica (immunostaining of frozen tissues) or a High-Performance IEEE 1394 FireWire Digital CCD Camera (QIMAGING, Surrey, BC, Canada) (immunostaining of paraffin-embedded tissues). For the quantification of specific fluorescent signals, 3 confocal images (1024x1024 pixels, equivalent to 375x375 µm) for each sample, acquired by keeping all the parameters constant, were divided in two 8-bit images corresponding to the red and green fluorescence channels. These image pairs were analyzed with the Image Processing and Analysis software in Java (ImageJ, version 1.44h, http://rsbweb.nih.gov/ij/, using the Colocalization Highlighter plugin to create a binary representation of co-localized pixels, and the Image Calculator option to derive the non-co-localized pixels (Dong et al. 1994, J Natl Cancer Inst 86:913-920).

Cytofluorimetric analyses were performed with the use of the Cytofix/Cytoperm™ Kit (BD Transduction Laboratories), following the manufacturer's protocol.

lmmunoblot and immunoprecipitation. Cells were lysed in 4 pack cell volumes of phosphate buffered saline (PBS), 1 mM CaCl₂, 1 mM MgCl₂, 1 mM phenylmethylsulfonyl fluoride (PMSF), protease inhibitor cocktail, supplemented with either 50 mM β-octyl-D-glucosylpyranoside (interaction studies) or 0.1% NP-40 (expression studies). Tissues were homogenized in a Potter-Elvehjem grinder in the same buffer (∼1 ml/100 mg tissue). Homogenates were cleared by centrifugation followed by filtration through 0.45 µm pore filters. For immunoprecipitation, lysates were pre-cleared for 1 hour at 4°C on Protein G-Sepharose (GE Healthcare, Chalfont St. Giles, UK), followed by incubation with specific antibodies for 1 hour at 4°C and addition of Protein G-Sepharose for another 2 hours at 4°C. Proteins were separated on 10% SDS-polyacrylamide gels and were blotted onto polyvinylidene fluoride (PVDF) membranes (Millipore, Billerica, MA). For protein quantification, densitometric analysis of the detected bands was performed with the QuantityOne software (BioRad, Hercules, CA); values were normalized to the intensity of vinculin at each experimental point.

Adhesion, proliferation, and migration assays. All the described *in vitro* tests were performed at least in triplicate. For investigation of cell adhesion to peptides and proteins, 1 µg of each substrate was incubated per well of a 96 well-plate for 1 hour at 37°C. After a blocking step in IMDM, 2% FCS for 1 hour at 37°C, 10⁴ cells were allowed to adhere for 1 hour at 37°C. Samples were washed gently in PBS, and cells were fixed in 8% glutaraldehyde and stained in 0.25% crystal violet in 10% methanol. For tissue adhesion, OCT-frozen grossly normal liver samples were cut into 10 µm sections. Tissues were blocked in IMDM, 2% FCS for 30 minutes at 37°C, followed by incubation with 5x10⁴ cells in 5% CO₂ at 37°C, for the indicated periods of time. Samples were washed 4 times in the same medium and once in PBS, fixed in 4% para-formaldehyde, and stained with hematoxylin (BioOptica). Adhered cells were counted manually under a light microscope.

To test the effects of peptides on cell proliferation, we seeded 2x10⁴ cells per well in a 24-well plate, in the presence of either control or CGIYRLRSC peptide (100 µM). At the indicated time points, cells were fixed in glutaraldehyde, stained in crystal violet, and solubilized in 10% acetic acid. Cell growth was evaluated by absorbance at 590 nm in a microplate reader (Perkin Elmerm Waltham, MA). A calibration curve was set up with known numbers of cells and a linear correlation between absorbance and cell counts was established up to 5x10⁵ cells/well.

Cell migration was evaluated in 24-well plates by the use of PET track-etched membrane transwells (8 µm pores) previously equilibrated in PBS for 1 hour at 37°C. To produce a gradient of angiopoietin-like 6, 5x10⁴ ligand-producing U293 cells were seeded into the lower chamber in 800 µl of complete culture medium. After 24 hours, transwells were inserted, control (mock-transfected) or receptor-expressing U293 cells were seeded into the upper chamber (1x10⁵ cells in 200 µl of complete culture medium), and the plates were incubated at 37°C in 5% CO₂ for 48 hours. Transwells were subsequently removed, cells on their upper side were scraped off, and migrated cells were fixed in glutaraldehyde and stained with crystal violet (BioOptica). Migrated cells were counted manually under a light microscope.

Animal models of human metastatic CRC. Six-week female CD1-nude mice were purchased from Charles River (Lecco, Italy). Animals were subjected to intraperitoneal anaesthesia with a mixture composed by 0.75 mg/ml xylazine (Xilor^{®}, BIO98, Milan, Italy), 1 mg/ml tiletamine - 1mg/ml zolazepam (Zoletil^{®}, Virbac, Milan, Italy), in physiological solution. After the mice were deeply asleep, a midline incision was performed and target organs were gently exposed. Two or five million suspended cells were injected in 50 µl of culture medium intrasplenically (Tibbettset al. 1993. Cancer, 71:315-321) or into the median liver lobe (Grothey, et al. 2009. Nat Rev Clin Oncol, 6:507-518), respectively. To investigate a pharmacological intervention on liver homing and/or colonization of CRC cells, animals were divided in two arms, receiving medium alone (vehicle) or supplemented with 100 µM CGIYRLRSC peptide. The wound was closed by a double suture and each animal was given 0.1 mg caprofen (Rymadil^{®}, Pfizer, Milan, Italy) in a physiological solution to allow post-operative pain relief and rehydration. Mice were strictly monitored until completely awake, and oral ampicillin was administered for 5 days following the surgery. Mice were euthanized at the indicated time points, and organs were photographed with a PL-200 digital photocamera (Samsung Electronics, Milan, Italy). External metastatic areas were quantified using ImageJ software.

Statistical analyses. All the analyses were performed with Prism 5 software (GraphPad, La Jolla, CA): two-way analysis of variance (ANOVA) followed by Bonferroni's post-test was used to evaluate differences within treatments; t-test and Fisher's exact test (two-tailed) were used to compare selected experimental points; Chi-squared test was used to analyze contingency tables; survival curves were drawn as Kaplan-Meier Cumulative Proportion Surviving graphs and p-values were calculated by the use of the log-rank (Mantel-Cox) test. In all the graphs, unless differently specified, asterisks indicate the following p-value ranges: * = p<0.05, ** = p<0.01, *** = p<0.001.

### Example 1: Towards an extracellular protein signature of human liver metastases from CRC.

A protocol for the isolation of heterogeneous cell populations by tissue fractionation of human liver metastases immediately after surgical removal was designed and cells extracted from matching adjacent, grossly normal livers as negative controls were used. Three phage-displayed peptide libraries with the general arrangements CX₇C, CX₉C and CX₃CX₃CX₃C (C = Cys and X = any residue) were screened on a panel of tumor/non-tumor paired samples in several independent biopanning experiments. In 13 out of 22 experiments (59%), enrichment of phage populations binding to liver metastases in comparison to the corresponding normal liver controls was observed (see **Figure 1A** for a detailed flowchart of the experimental and bioinformatics approaches). Overall, a total of 265 phage clones were recovered, purified and DNA-sequenced (Scott et al. 1990. Science 249:386-390; Smith et al. 1993, Methods Enzymol 217:228-257). Analysis of the cognate 265 metastasis-binding (MTS) peptides revealed 203 unique sequences; the tripeptide LRS was the most represented motif, shared by ∼23% of the phage clones. Because the biopanning experiments were performed on a mixture of intact cells and tissue stroma, MTS peptides represent prototype ligands for the extracellular microenvironment of liver metastases. To identify natural ligands mimicked by these peptides, non-redundant databases of human proteins were searched with the BLAST tool. Forward- (FW) and reverse- (REV) oriented MTS peptide sequences were searched as described (Arap et al 2002, Nat Med 8:121-127). The results were organized by grouping the output proteins for numbers of similarity matches and thereby created the MTS_FW and MTS_REV datasets, respectively. Corresponding genes were assigned a Gene Ontology Cell Component (GO-CC) annotation by using the DAVID Bioinformatics Resources Functional Annotation tool (Huang et al. 2009 Nat Protoc 4:44-57; Huang et al. 2009, Nucleic Acids Res 37:1-13). A related statistical analysis confirmed marked enrichment in extracellular matrix (ECM), plasma membrane and soluble components. The difference between the MTS_FW and MTS_REV datasets was striking when genes coding for proteins with ≥ 3 similarity matches were compared **(****Figure 1 B)****.**
We therefore extracted from the cognate MTS_FW dataset all the genes coding for extracellular proteins and termed this sub-dataset *"extracellular protein signature"* of liver metastases secondary to CRCs **(Table 1).**

**Table 1: The extracellular protein signature of human liver metastasis secondary to colon cancer**

| | |
|---|---|
| The 177 genes coding for the ECM, plasma membrane and soluble proteins annotated in the MET_FW dataset (≥ 3 similarity matches) have been using the DAVID Bioinformatics Resources Functional Annotation tool. Official names are listed alphabetically. | |

| **Entrez Gene_ID** | **Gene name** |
|---|---|
| 3357 | 5-hydroxytryptamine (serotonin) receptor 2b |
| 3360 | 5-hydroxytryptamine (serotonin) receptor 4 |
| 81792 | ADAM metallopeptidase with thrombospondin type 1 motif, 12 |
| 11093 | ADAM metallopeptidase with thrombospondin type 1 motif, 13 |
| 170690 | ADAM metallopeptidase with thrombospondin type 1 motif, 16 |
| 80070 | ADAM metallopeptidase with thrombospondin type 1 motif, 20 |
| 11173 | ADAM metallopeptidase with thrombospondin type 1 motif, 7 |
| 56999 | ADAM metallopeptidase with thrombospondin type 1 motif, 9 |
| 54507 | ADAMTS-like 4 |
| 375790 | agrin |
| 351 | amyloid beta (a4) precursor protein (peptidase nexin-ii, alzheimer disease) |
| 83854 | angiopoietin-like 6 |
| 338 | apolipoprotein b (including ag(x) antigen) |
| 552 | arginine vasopressin receptor 1a |
| 19 | ATP-binding cassette, sub-family a (ABC1), member 1 |
| 24 | ATP-binding cassette, sub-family a (ABC1), member 4 |
| 4363 | ATP-binding cassette, sub-family c (cftr/mrp), member 1 |
| 10057 | ATP-binding cassette, sub-family c (cftr/mrp), member 5 |
| 368 | ATP-binding cassette, sub-family c (cftr/mrp), member 6 |
| 5099 | bh-protocadherin (brain-heart) |
| 575 | brain-specific angiogenesis inhibitor 1 |
| 9620 | cadherin, egf lag seven-pass g-type receptor 1 (flamingo homolog, drosophila) |
| 8913 | calcium channel, voltage-dependent, alpha 1g subunit |
| 774 | calcium channel, voltage-dependent, I type, alpha 1 b subunit |
| 779 | calcium channel, voltage-dependent, I type, alpha 1s subunit |
| 773 | calcium channel, voltage-dependent, p/q type, alpha 1 a subunit |
| 1048 | carcinoembryonic antigen-related cell adhesion molecule 5 |
| 933 | CD22 antigen |
| 1462 | chondroitin sulfate proteoglycan 2 (versican) |
| 1464 | chondroitin sulfate proteoglycan 4 (melanoma-associated) |
| 2155 | coagulation factor vii (serum prothrombin conversion accelerator) |
| 1277 | collagen, type I, alpha 1 |
| 1278 | collagen, type I, alpha 2 |
| 1280 | collagen, type II, alpha 1 (primary osteoarthritis, spondyloepiphyseal dysplasia, congenital) |
| 1281 | collagen, type IIII, alpha 1 (ehlers-danlos syndrome type iv, autosomal dominant) |
| 1282 | collagen, type IV, alpha 1 |
| 1284 | collagen, type IV, alpha 2 |
| 1285 | collagen, type IV, alpha 3 (goodpasture antigen) |
| 1287 | collagen, type IV, alpha 5 (alport syndrome) |
| 1288 | collagen, type IV, alpha 6 |
| 1297 | collagen, type IX, alpha 1 |
| 1289 | collagen, type V, alpha 1 |
| 1290 | collagen, type V, alpha 2 |
| 50509 | collagen, type V, alpha 3 |
| 1293 | collagen, type VI, alpha 3 |
| 1294 | collagen, type VII, alpha 1 (epidermolysis bullosa, dystrophic, dominant and recessive) |
| 1295 | collagen, type VIII, alpha 1 |
| 1301 | collagen, type XI, alpha 1 |
| 1302 | collagen, type XI, alpha 2 |
| 1303 | collagen, type XII, alpha 1 |
| 80781 | collagen, type XVIII, alpha 1 |
| 85301 | collagen, type XXVII, alpha 1 |
| 8292 | collagen-like tail subunit (single strand of homotrimer) of asymmetric acetylcholinesterase |
| 1378 | complement component (3b/4b) receptor 1 (knops blood group) |
| 719 | complement component 3a receptor 1 |
| 730 | complement component 7 |
| 5199 | complement factor properdin |
| 51232 | cysteine rich transmembrane bmp regulator 1 (chordin-like) |
| 1755 | deleted in malignant brain tumors 1 |
| 1826 | down syndrome cell adhesion molecule |
| 1950 | epidermal growth factor (beta-urogastrone) |
| 2057 | erythropoietin receptor |
| 2195 | fat tumor suppressor homolog 1 (drosophila) |
| 2200 | fibrillin 1 (marfan syndrome) |
| 2201 | fibrillin 2 (congenital contractural arachnodactyly) |
| 84467 | fibrillin 3 |
| 2244 | fibrinogen beta chain |
| 2335 | fibronectin 1 |
| 2322 | fms-related tyrosine kinase 3 |
| 80144 | FRAS1 |
| 158326 | FRAS1 related extracellular matrix 1 |
| 166752 | FRAS1 related extracellular matrix 3 |
| 341640 | FRAS1 related extracellular matrix protein 2 |
| 2897 | glutamate receptor, ionotropic, kainate 1 |
| 2912 | glutamate receptor, metabotropic 2 |
| 2811 | glycoprotein ib (platelet), alpha polypeptide |
| 2812 | glycoprotein ib (platelet), beta polypeptide |
| 51206 | glycoprotein vi (platelet) |
| 83872 | hemicentin 1 |
| 3339 | heparan sulfate proteoglycan 2 (perlecan) |
| 3547 | immunoglobulin superfamily, member 1 |
| 3645 | insulin receptor-related receptor |
| 3482 | insulin-like growth factor 2 receptor |
| 3655 | integrin, alpha 6 |
| 8516 | integrin, alpha 8 |
| 3681 | integrin, alpha d |
| 3685 | integrin, alpha v (vitronectin receptor, alpha polypeptide, antigen cd51) |
| 3687 | integrin, alpha x (complement component 3 receptor 4 subunit) |
| 3691 | integrin, beta 4 |
| 3693 | integrin, beta 5 |
| 3697 | inter-alpha (globulin) inhibitor h1 |
| 3587 | interleukin 10 receptor, alpha |
| 3588 | interleukin 10 receptor, beta |
| 182 | jagged 1 (alagille syndrome) |
| 3714 | jagged 2 |
| 3938 | lactase |
| 284217 | laminin, alpha 1 |
| 3908 | laminin, alpha 2 (merosin, congenital muscular dystrophy) |
| 3910 | laminin, alpha 4 |
| 3911 | laminin, alpha 5 |
| 3912 | laminin, beta 1 |
| 3913 | laminin, beta 2 (laminin s) |
| 8425 | latent transforming growth factor beta binding protein |
| 4052 | latent transforming growth factor beta binding protein 1 |
| 2615 | leucine rich repeat containing 32 |
| 11025 | leukocyte immunoglobulin-like receptor, subfamily b (with tm and itim domains), member 3 |
| 7804 | low density lipoprotein receptor-related protein 8, apolipoprotein e receptor |
| 4035 | low density lipoprotein-related protein 1 (alpha-2-macroglobulin receptor) |
| 4036 | low density lipoprotein-related protein 2 |
| 4481 | macrophage scavenger receptor 1 |
| 4318 | matrix metallopeptidase 9 (gelatinase b, 92kda gelatinase, 92kda type iv collagenase) |
| 4583 | mucin 2, intestinal/tracheal |
| 4584 | mucin 3a, intestinal |
| 4585 | mucin 4, tracheobronchial |
| 4586 | mucin 5, subtypes a and c, tracheobronchial/gastric |
| 8777 | multiple pdz domain protein |
| 4340 | myelin oligodendrocyte glycoprotein |
| 9378 | neurexin 1 |
| 23620 | neurotensin receptor 2 |
| 4914 | neurotrophic tyrosine kinase, receptor, type 1 |
| 4811 | nidogen 1 |
| 4854 | notch homolog 3 (drosophila) |
| 4855 | notch homolog 4 (drosophila) |
| 60506 | nyctalopin |
| 10178 | odz, odd oz/ten-m homolog 1 (drosophila) |
| 23596 | opsin 3 (encephalopsin, panopsin) |
| 146183 | otoancorin |
| 89932 | papilin, proteoglycan-like sulfated glycoprotein |
| 8643 | patched homolog 2 (drosophila) |
| 22925 | phospholipase a2 receptor 1, 180kda |
| 5310 | polycystic kidney disease 1 (autosomal dominant) |
| 3780 | potassium intermediate/small conductance calcium-activated channel, subfamily n, member 1 |
| 3782 | potassium intermediate/small conductance calcium-activated channel, subfamily n, member 3 |
| 3756 | potassium voltage-gated channel, subfamily h (eag-related), member 1 |
| 5069 | pregnancy-associated plasma protein a, pappalysin 1 |
| 8842 | prominin 1 |
| 5787 | protein tyrosine phosphatase, receptor type, b |
| 5795 | protein tyrosine phosphatase, receptor type, j |
| 11122 | protein tyrosine phosphatase, receptor type, t |
| 10076 | protein tyrosine phosphatase, receptor type, u |
| 56138 | protocadherin alpha 11 |
| 56145 | protocadherin alpha 3 |
| 5754 | ptk7 protein tyrosine kinase 7 |
| 5027 | purinergic receptor p2x, ligand-gated ion channel, 7 |
| 5029 | purinergic receptor p2y, g-protein coupled, 2 |
| 5649 | reelin |
| 23145 | sco-spondin homolog (bos taurus) |
| 6401 | selectin e (endothelial adhesion molecule 1) |
| 80274 | signal peptide, cub domain, egf-like 1 |
| 6326 | sodium channel, voltage-gated, type ii, alpha 2 |
| 6331 | sodium channel, voltage-gated, type v, alpha (long qt syndrome 3) |
| 6332 | sodium channel, voltage-gated, type vii, alpha |
| 6557 | solute carrier family 12 (sodium/potassium/chloride transporters), member 1 |
| 6558 | solute carrier family 12 (sodium/potassium/chloride transporters), member 2 |
| 6582 | solute carrier family 22 (organic cation transporter), member 2 |
| 3177 | solute carrier family 29 (nucleoside transporters), member 2 |
| 7781 | solute carrier family 30 (zinc transporter), member 3 |
| 6531 | solute carrier family 6 (neurotransmitter transporter, dopamine), member 3 |
| 9152 | solute carrier family 6 (neurotransmitter transporter, glycine), member 5 |
| 6530 | solute carrier family 6 (neurotransmitter transporter, noradrenalin), member 2 |
| 23428 | solute carrier family 7 (cationic amino acid transporter, y+ system), member 8 |
| 64093 | SPARC related modular calcium binding 1 |
| 124912 | sperm acrosome associated 3 |
| 23166 | stabilin 1 |
| 6768 | suppression of tumorigenicity 14 (colon carcinoma) |
| 3371 | tenascin c (hexabrachion) |
| 7143 | tenascin r (restrictin, janusin) |
| 7148 | tenascin xb |
| 7038 | thyroglobulin |
| 54106 | toll-like receptor 9 |
| 7357 | udp-glucose ceramide glucosyltransferase |
| 54346 | unc-93 homolog a (c. elegans) |
| 7399 | usher syndrome 2a (autosomal recessive, mild) |
| 2066 | v-erb-a erythroblastic leukemia viral oncogene homolog 4 (avian) |
| 2065 | v-erb-b2 erythroblastic leukemia viral oncogene homolog 3 (avian) |
| 7450 | von willebrand factor |
| 6098 | v-ros ur2 sarcoma virus oncogene homolog 1 (avian) |

### Example 2: The extracellular signature: selectivity of LRS-peptides and tissue distribution of their targets.

The enrichment for LRS sequences among the MTS peptide population was suggestive of a role as a relevant ligand motif within the microenvironment of particularly the liver metastasis. For an initial molecular analysis, we selected a panel of LRS-containing peptides (n=7): CLRSGRGSC, CLRPGLRSC, CGIYRLRSC, CMRYALRSC, CARPGLRSC, CLRSGSGSC and CGVYSLRSC. We first evaluated binding of the cognate phage clones to a panel of human cell lines from different primary tumors and metastases (n=3) (Figure 2A). All the selected phages showed binding preference for NCI-H630 cells (liver metastasis from CRC), with CGIYRLRSC (in red) and CGVYSLRSC (in yellow) as the most specific ligands. Next, binding of the LRS-displaying phage clones to cells freshly-extracted from patients (n=5) **(****Figure 2B****)** were evaluated and it was found that most phages recognized liver metastases with high selectivity. Specific phage binding was higher on heterogeneous primary cells (range, 2.8-50.2) compared to the NCI-H630 cell line (range, 1.6-10.3), a result indicating that cell types other than metastatic epithelial cells might be targeted and/or that in fresh human tissues the molecular targets are more than in the cognate cell line. To evaluate the tissue distribution of LRS-phage targets, binding overlay assays on a panel of different human epithelial tumor types and their corresponding metastases (n=31) **(Table 2)** were performed. Consistently, CGIYRLRSC-phage identified >75% of the liver metastases secondary to CRCs. Notably, cell clusters in proximity to blood vessels were generally well-targeted by the CGIYRLRSC-phage **(****Figure 2C****).** In these same assays, CGVYSLRSC-phage showed similar distribution and staining (data not shown).

**Table 2: CGIYRLRSC-phage targets human liver metastases with high selectivity**

| | |
|---|---|
| Ten micrometer cryostatic tissue sections were incubated with 10⁸ TU of either the insertless fd-tet or the CGIYRLRSC-phage. The intensity of the immunostaining was estimated by comparison with the controls. Patient lDs for liver metastasis were P24, P29, P30, P33, P34, P35, P38, P39, P40, P41, P42, P43, P45, P47; patient lDs for ovary cancer/sigma metastasis were PO218, P0219, P0226, P0229, P0235 and PO . | |

| **Human tissues** | **Positive/total samples** |
|---|---|
| Metastatic CRC (liver) | 11/14(79%) |
| Ovary cancer | 1/6 (17%) |
| Metastatic ovary cancer (sigma) | 1/7 (14%) |

### Example 3:The receptor side of the the signature: α₆ integrin and E-cadherin are targets for an LRS-peptide and form a molecular complex in human liver metastases from CRC.

We produced soluble CGIYRLRSC as a fusion peptide with Glutathione S-Transferase (CGIYRLRSC-GST), to exploit the interaction with NCI-H630 cell surfaces toward the identification of potential receptor(s). HepG2 cells, which do not bind the CGIYRLRSC-phage specifically (Figure 2A), served as a negative control. CGIYRLRSC-GST was incubated with both cell lysates, followed by separation of the bound proteins by SDS-PAGE. Specific bands (n=11) were analyzed by mass spectrometry; 37 proteins with an identification score >50 were thereby obtained **(Table 3).** Of this set, 8 cell-surface proteins (i.e. putative receptors for CGIYRLRSC) and 23 cytoskeletal proteins were found, a result indicating that a protein or protein complex involved in cell adhesion and/or motility might be responsible for this interaction. Among the cell adhesion proteins found, α₆ integrin and E-cadherin exhibited the highest identification score.

**Table 3: An LRS-containing MTS peptide is a candidate ligand for an adhesion complex on liver metastasis cells**

| | | | | |
|---|---|---|---|---|
| NCI-H630 (target) and HepG2 (control) cell lysates were incubated with GST-CGIYRLRSC. Selectively bound protein were separated by gel elecrophoresis and were identified by LC-MS/MS. Swiss Prot entries, protein names and MASCOT identification scores of the identified proteins are listed. Examples of protein local izations/functions are also shown in the table. | | | | |

| **Swiss pro** | **Protein name** | **Score** | **Localization** | **Function** |
|---|---|---|---|---|
| P12830 | E-cadherin | 173 | cell surface | adhesion |
| P23229 | alpha-6 integrin | 98 | cell surface | adhesion |
| P56470 | galectin-4 | 75 | cell surface | adhesion |
| P16444 | microsomal dipeptidase | 117 | cell surface | protease |
| P05026 | Na/K-ATPase β1 chain | 94 | cell surface | channel |
| P07900, P08238 | HSP 90-α, HSP 90-β | 125 | cell surface/ cytoplasm | chaperone |
| P19338 | nucleolin | 236 | cell surface/nucleus | chromatin binding |
| Q00839 | hnRNP U | 124 | cell surface/nucleus | DNA/RNA binding |
| P35579 | myosin-9 | 4750 | cytoplasm | cytoskeleton |
| Q7Z406 | myosin-14 | 2408 | cytoplasm | cytoskeleton |
| Q01082 | spectrin β chain | 1989 | cytoplasm | cytoskeleton |
| 094832 | myosin Id | 1804 | cytoplasm | cytoskeleton |
| P09327 | villin-1 | 1782 | cytoplasm | cytoskeleton |
| Q00610 | clathrin heavy chain 1 | 1556 | cytoplasm | cytoskeleton |
| 043795 | myosin Ib (Myosin Iα) | 1252 | cytoplasm | cytoskeleton |
| P07355 | annexin A2 | 994 | cytoplasm | cytoskeleton |
| O00159 | myosin Ic (Myosin Iβ) | 908 | cytoplasm | cytoskeleton |
| P60709 | actin | 895 | cytoplasm | cytoskeleton |
| Q13813 | Spectrin alpha chain | 790 | cytoplasm | cytoskeleton |
| Q9NYL9 | tropomodulin-3 | 758 | cytoplasm | cytoskeleton |
| Q12965 | myosin le (Myosin Ic) | 583 | cytoplasm | cytoskeleton |
| P06753 | tropomyosin 3 | 420 | cytoplasm | cytoskeleton |
| P68363 | α-tubulin | 258 | cytoplasm | cytoskeleton |
| P09525 | annexin A4 | 237 | cytoplasm | cytoskeleton |
| P35580 | myosin-10 | 235 | cytoplasm | cytoskeleton |
| Q9P2M7 | cingulin | 224 | cytoplasm | cytoskeleton |
| O15143 | actin-related protein 2/3 complex sub 1B | 193 | cytoplasm | cytoskeleton |
| P35611 | α-adducin | 98 | cytoplasm | cytoskeleton |
| O15144 | actin-related protein 2/3 complex sub 2 | 96 | cytoplasm | cytoskeleton |
| P68371 | tubulin β-2 chain | 84 | cytoplasm | cytoskeleton |
| Q9UJZ1 | stomatin-like protein 2 | 81 | cytoplasm | cytoskeleton |
| P09874 | poly[ADP-ribose]polymerase | 106 | cytoplasm | enzyme |
| P16152 | NADPH-carbonyl reductase | 74 | cytoplasm | enzyme |
| P63092 | G nucleotide-binding protein | 98 | cytoplasm | G protein |
| P61247 | 40S ribosomal prot S3a | 224 | cytoplasm | ribosome |
| P25705 | ATP synthase α chain | 200 | mitochondrium | enzyme |
| P45880 | voltage-dependent channel | 111 | mitochondrium | channel |

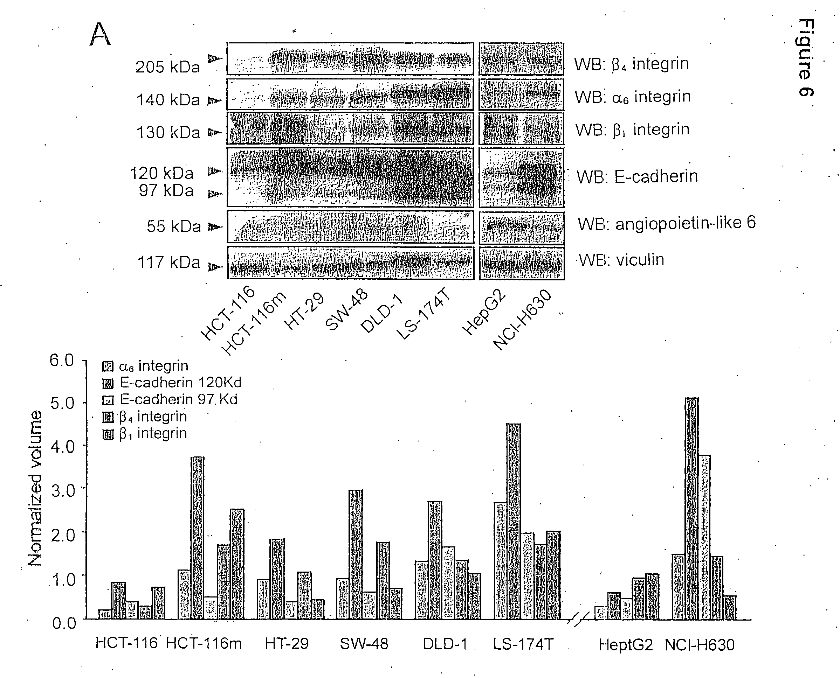

The location of α₆ integrin and E-cadherin in NCI-H630 and HepG2 cells was evaluated by confocal microscopy imaging **(****Figure 3A****).** There was co-localization of these proteins in the liver metastasis cell line NCI-H630, in which both α₆ integrin and E-cadherin were highly represented on cell membranes; in contrast, barely detectable immunostaining of α₆ integrin and no co-localization with E-cadherin were observed in the primary tumor cell line HepG2. It was suspected that these proteins could be part of a supramolecular complex in liver metastasis cells, as indicated by mass spectrometry and by their coincident location on the cell surface. Co-immunoprecipitation assays confirmed that α₆ integrin and E-cadherin, physically interact in NCI-H630 cells **(****Figure 3A** and **Figure 6A** for protein quantification in cell lines). Confocal imaging analyses performed on liver metastasis samples from CRC patients (n=6) (see also **Figure** 4) revealed that α₆ integrin and E-cadherin were expressed by selected groups of cells, with regions of overlap; in contrast, α₆ integrin was barely detectable and the two proteins did not co-localize in matched normal livers. A co-immunoprecipitation assay performed on proteins from 5 pooled liver metastases confirmed the presence of the α₆ integrin/E-cadherin complex in these tissues; this complex could not be detected in samples of grossly normal livers from the same patients **(****Figure 3B****).**

These results show that α₆ integrin and E-cadherin are expressed and coincident in regions of i.a. human liver metastases, where they participate in a molecular complex.

### Example 4: The ligand side of the signature: angiopoietin-like 6 mimics an LRS-peptide and is enriched in blood vessels of the liver in humans.

A BLAST search specific for proteins similar to the closely-related CGIYRLRSC and CGVYSLRSC peptides revealed a set of extracellular proteins (n=54), 4 of which were listed in the extracellular protein signature as well (Table 1), i.e. angiopoietin-like 6, perlecan, laminin α₂ and nyctalopin. In this analysis, angiopoietin-like 6 received the highest identification score, because it shares similarity with the targeting peptides in two different regions of its fibrinogen-like domain. Interestingly, angiopoietin-like 6 mRNA has been detected particularly in the liver in humans (Kim et al. 2000, Biochem J 346 Pt 3:603-610; Oike et al. 2003, Proc Natl Acad Sci USA 100:9494-9499). To investigate whether angiopoietin-like 6 could actually be a ligand for the hepatic homing of metastatic CRC cells, we evaluated the presence of this protein in several tissue types from healthy donors (Figure 7). We confirmed that normal hepatic tissues produce high amounts of angiopoietin-like 6, although its expression was detectable in most tissues. A fine histological evaluation performed on grossly normal livers (n=79) from metastatic CRC patients (Figure 3C, see also Figure 5) revealed that in these tissues angiopoietin-like 6 is present both in large blood vessels (branches of the portal vein) and in capillaries (sinusoids, lymphatics), all potential sites for the molecular recognition of circulating CRC cells through specific ligand/receptor interactions.

### Example 5: Coupling receptors and ligands (1): ex vivo and in vivo models of metastasis/host tissue interaction.

By confocal microscopy imaging we observed that angiopoietin-like 6 accumulates in hepatic blood vessels of metastatic CRC patients (Figure 3D). In a small subset of samples of grossly normal liver, a few cellular aggregates were observed that were positive for the metastatic marker Phosphatase of Regenerating Liver 3 (PRL3) and for α₆ integrin (Saha et al. 2001, Science 294:1343-1346). Such micrometastatic foci were specifically associated with blood vessels, where an extensive co-localization of hepatic angiopoietin-like 6 and metastatic α₆ integrin was evident **(****Figure 3D****).**

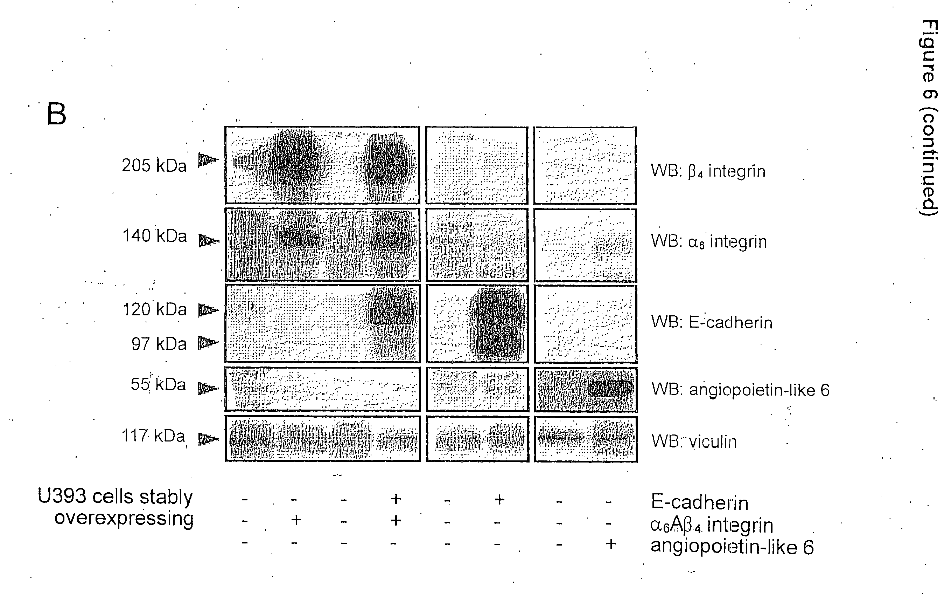

With the intention of designing a molecularly-defined cell model capable of reproducing these metastasis/host interactions, the nature of the integrin component in the receptor complex were dissected. The α₆ integrin (i) can form heterodimers with either β₁ or β₄, depending on the cell type (Humphries et al. 2006 J Cell Sci 119:3901-3903; Hemler et al. 1988, J Biol Chem 264:6529-6535; Hemler et al. 1988, J Biol Chem 263:7660-7665) and (ii) is encoded by two splicing variants of a single mRNA, resulting in proteins with an alternative cytoplasmic tail, namely α₆A and α₆B (Tamura et al. 1991, Proc Natl Acad Sci USA 88:10183-10187; Hogervorst et al . 1991, Eur J Biochem 199:425-433; Hogervorst, et al. 1993, J Cell Biol 121:179-191). Lysates of human liver metastases were affinity-purified with column-immobilized CGIYRLRSC-peptide, observing a substantial enrichment in the β₄ subunit in peptide- and phage-targeted protein fractions in comparison with the controls **(****Figure 8****).** Interestingly, β₄ integrin was listed in the extracellular signature as well **(Table 1),** further suggesting a role for this subunit in the protein network of the hepatic metastasis microenvironment. The presence of alternative transcripts in CRC cells (n=7) and in samples (n=45) of liver metastasis from CRC was evaluated, demonstrating a prevalence of an α₆A/α₆B ratio >1 both in cell lines (71%) and in human specimens (62%) **(****Figure 9****).** Based on these results, we produced U293 cells stably overexpressing α₆Aβ₄ (hereafter abbreviated as α₆) integrin together with E-cadherin. To reproduce the microenvironment of the host tissue, we also prepared U293 cells stably producing angiopoietin-like 6 (see **Figure 6B** for protein characterization of all these cell lines). Interestingly, when cells transduced with the receptor complex were mixed with cells secreting the ligand, the former segregated in metastasis-like cell aggregates, surrounded by soluble angiopoietin-like 6 and in tight contact with the ligand-producing cells **(****Figure 3E****).**

These results demonstrate that angiopoietin-like 6 could represent a potential, thus far unrecognized ligand for metastatic cells that express the α₆ integrin/E-cadherin receptor complex.

### Example 6: Coupling receptors and ligands (2): the α₆ integrin/E-cadherin complex and angiopoietin-like 6 mediate the adhesion of human metastatic CRC cells to the liver in vitro.

To evaluate a potential role for the α₆ integrin/E-cadherin complex in the process of hepatic metastasis, the capacity of HepG2 and NCI-H630 cells was compared to adhere to normal livers. Cells were incubated on human liver sections for increasing periods of time, ranging from 30 minutes to 5 days. At all timepoints, HepG2 cells adhered weakly to normal liver and grew in separate, individual aggregates. In contrast, the adhesion of NCI-H630 cells was significantly higher; moreover, these cells could grow and integrate into the hepatic tissue **(****Figure 10A****)** with a morphology reminiscent of the metastasis/host tissue model described in **Figure 3C****.** siRNA-mediated downmodulation of α₆ integrin (ITGA6), E-cadherin (CDH1), or both mRNAs **(****Figure 11A****)** in NCI-H630 cells was obtained. All the silenced NCI-H630 cells exhibited impaired adherence to normal liver, with the double-silenced cells showing the slowest adhesion (Figure 10B). These experiments confirm that α₆ integrin and E-cadherin are active mediators in the adhesion of metastatic CRC cells to the liver.

For a molecular dissection of the ligand/receptor system, the capacity of silenced NCI-H630 cells to bind the CGIYRLRSC motif and to interact with angiopoietin-like 6 was evaluated. NCI-H630 cells in which ITGA6 or both mRNAs were silenced lost the capacity to bind the CGIYRLRSC-phage. Consistently, these cells also exhibited an impaired adherence to microwells coated with the CGIYRLRSC-peptide; this effect was particularly pronounced when both α₆ integrin and E-cadherin were downmodulated. In parallel assays, NCI-H630 cells in which both mRNAs were silenced exhibited significantly lower binding to microwells coated with recombinant angiopoietin-like 6 **(****Figure 10C****).**

These results indicate that CGIYRLRSC-mimicked ligand proteins, such as angiopoietin-like 6, can act as microenvironment addresses for metastatic cells that express α₆ integrin/E-cadherin receptor complex.

### Example 7: Coupling receptors and ligands (3): angiopoietin-like 6 mediates the attraction of cells expressing the α₆ integrin/E-cadherin complex.

Besides being accumulated in liver blood vessels, therefore contributing to the recognition of metastatic cells by the host tissue (Figure 3B), angiopoietin-like 6 is a secreted factor whose chemotactic activity on endothelial cells has been reported (Oike et al. 2004. Blood 103:3760-3765). Therefore, it was investigated if soluble angiopoietin-like 6 could affect the motility of cells expressing the α₆ integrin/E-cadherin receptor complex. For this purpose, U293 cells transduced with α₆ integrin, E-cadherin, or a combination of both **(****Figure 6B****)** were co-cultured with angiopoietin-like 6-producing cells in a transwell system and their migration toward the ligand gradient was evaluated after 48 hours. Co-cultures with mock-transfected U293 cells were exploited as a reference for basal cell motility. The presence of either α₆ integrin or E-cadherin slightly increased the capacity of U293 cells to migrate in basal conditions; however, this feature was not influenced by the presence of soluble angiopoietin-like 6. On the contrary, U293 cells expressing both α₆ integrin and E-cadherin showed a basal pro-migratory phenotype, which was significantly stimulated by angiopoietin-like 6 **(****Figure 10D****).**

These results demonstrate that the presence of both α₆ integrin and E-cadherin is necessary for target cells to respond to a chemotactic gradient of angiopoietin-like 6 protein, further confirming a functional role for this ligand/receptor system.

### Example 8:Uncoupling ligands and receptors (1): CGIYRLRSC-peptide inhibits the adhesion and attraction of metastatic CRC cells to the liver in vitro.

Since the CGIYRLRSC-peptide mimics a liver-enriched ligand for the α₆ integrin/E-cadherin receptor complex, it was investigated whether it could interfere with this interaction, thus inhibiting the adhesion of metastatic cells to normal liver. It was observed that when NCI-H630 cells were incubated on human liver sections from several patients (n=27), a significant decrease in cell adhesion occurred after treatment with CGIYRLRSC (Figure 10E). Also, the capacity to adhere to the liver of molecularly-defined cell lines, i.e. U293 cells stably transduced with the cDNA for α₆ integrin, E-cadherin, or both, was investigated. U293 cells in which α₆ integrin was overexpressed, either alone or in combination with E-cadherin, showed an increased affinity for the hepatic tissue; liver adhesion was the most pronounced in cells expressing both components of the receptor complex. Notably, CGIYRLRSC-peptide inhibited liver adhesion of U293 cells only when they concomitantly expressed α₆ integrin and E-cadherin **(****Figure 10F****).** The adhesive properties of different cell lines of primary CRC were evaluated with proven *in vivo* metastatic behavior, i.e. HCT-116m (a metastatic variant of HCT-116), HT-29, SW-48 and DLD-1. All these cells exhibited remarkable adhesiveness on hepatic tissues, which again was significantly inhibited by CGIYRLRSC. Notably, the non-metastatic HCT-116 cells, which express very low levels of the receptor proteins **(****Figure 6A****),** adhered poorly to normal hepatic tissues and were not influenced by the presence of the CGIYRLRSC-peptide **(****Figure 10G****).**

These data show that CGIYRLRSC specifically inhibits the adhesion of metastatic CRC cells to the liver, possibly through interference with the angiopoietin-like 6/α₆ integrin/E-cadherin ligand/receptor system.

### Example 9: Uncoupling ligands and receptors (2): interfering with liver colonization in animal models of human CRC.

In sum, the *in vitro* data indicated that two pivotal steps for the onset of liver metastasis, i.e. tumor/host tissue recognition and metastatic cell attraction, could be driven by the α₆ integrin/Ecadherin/angiopoietin-like 6 system. Accordingly, the interference with the described ligand/receptor pair was investigated to result in impaired liver colonization and homing *in vivo.*
An animal model of hepatic colonization was established by direct injection of human CRC cells into the livers of CD-1 nude mice. For a first set of experiments LS-174T, a cell line derived from a primary CRC that exhibits high expression of the complex proteins **(****Figure 6A****)** and an extremely aggressive behavior *in vivo* was used (Price et al. 1989, Clin Exp Metastasis 7:55-68). To evaluate the effect(s) of CGIYRLRSC, animals were injected with LS-174T cells either in medium alone or in the presence of the soluble peptide. After 14 days, the livers were explanted for photographic documentation and tumor quantification. A significant reduction of liver tumors in mice injected with LS-174T cells in the presence of CGIYRLRSC was observed, although the overall morphology and the levels of receptor complex in the tumors were unchanged **(****Figure 10H****).**

These results suggest that the CGIYRLRSC-peptide interferes with early steps of tumor/host tissue recognition, while not influencing successive tumor growth. Consistently, we observed that soluble CGIYRLRSC has no effects on the proliferation of U293 cells transduced with either α₆ integrin, Ecadherin or both **(****Figure 12****).**

To dissect the receptor side of the hepatic colonization, stable shRNA-mediated silencing of ITGA6 or CDH1 mRNA in different cell lines of primary CRC, i.e. HCT-116m, SW-48, DLD-1 and HT-29, were used **(****Figure 11 B)****.** By confocal microscope imaging it was further confirmed that protein downmodulation is retained *in vivo,* resulting in an almost complete disappearance of α₆ integrin/E-cadherin co-localization from the tumor tissues **(****Figure 13****).** In this set of experiments, all the cells in which either component of the receptor complex was silenced had an impaired capability to form tumors when injected into the liver; this effect reached a statistic significance when α₆ integrin was silenced in SW-48 and HT-29 cells and when E-cadherin was silenced in HCT-116m, SW-48 and DLD-1 cells **(****Figure 13****).**

These data demonstrate that even an incomplete depletion of only one receptor protein is sufficient to alter liver colonization by CRC cells in small animal models.

### Example 10: Uncoupling ligands and receptors (3): interfering with liver homing for anti-metastatic therapy.

Also, the interference of CGIYRLRSC-peptide with the homing of CRC cells to the liver was investigated. For this purpose,the human metastatic CRC tumor HCCM-1544 (Tibbetts et al. 1993, Cancer 71:315-321), as well as different CRC cell lines, i.e. HCT-116m, SW-48, DLD-1 and LS-174T, were implanted into the spleens of CD-1 nude mice. Cells were injected either in medium alone or in the presence of the soluble peptide. Mice were euthanized at different time points after cell injection, ranging from 20 days (HCT-116m) to 195 days (DLD-1). At the time of sacrifice, in all the tumor models a primary splenic mass and a variable number of liver metastases were present. The frequency of hepatic involvement in the vehicle arms varied from 11% (DLD-1) to 100% (LS-174T), reflecting the different aggressiveness of these CRC cells. Treatment with CGIYRLRSC resulted in a diminished homing of CRC cells to the liver, which was significant in all the models investigated, with the exclusion of the poorly metastatic DLD-1 cell line **(****Figure 14****).** These data confirm that the CGIYRLRSC-peptide inhibits liver homing of CRC cells independently from the features (timing, size and number of metastases) peculiar of each model, thus suggesting potential developments in the design of future anti-metastatic approaches with broad application.

Remarkably, in all the experimental settings including the HCCM-1544 human tumor, a substantial increase in the presence of both α₆ integrin and E-cadherin was observed, with diffuse regions of co-localization, in metastatic hepatic tissue compared to primary spleen tumors **(****Figure 14****).** This result indicates that the expression of such proteins is upregulated and/or that highly-expressing cell clones are selected during metastatic progression. A cytofluorimetric analysis of α₆ integrin and E-cadherin expression in primary and metastatic CRC cell lines following different culture conditions was performed. While the great majority of cells (range, 85-100%) expressed E-cadherin, although in variable levels, only a fraction of them produced α₆ integrin. Furthermore, the amounts of α₆ integrin varied not only among different CRC cell lines (from <2% in the non-metastatic HCT-116 cell line to >50% in the liver metastasis NCI-H630 cell line), but also as a consequence of different culture conditions, resulting in variable numbers of cell concomitantly expressing α₆ integrin and E-cadherin (Figure 15). The results indicate that a clonal selection of cells expressing both partners of the complex is preferred.

### Example 11: The α₆ integrin/E-cadherin complex and angiopoietin-like 6 are correlated to the aggressiveness of human metastatic CRCs.

The results obtained both *in vitro* and in animal models prompted to investigate the role of the described ligand/receptor system in a clinical context. By quantitative confocal imaging, the amounts of α₆ integrin, E-cadherin and their complex were evaluated in the following tumoral settings: primary CRCs (Duke's stage IV) (n=22) **(****Figure 16****),** liver metastases from CRC (n=100) **(****Figure 4****),** liver metastases from other cancers (n=22) **(****Figure 17****)** and lung metastases from CRCs (n=40) **(****Figure 18****).** This analysis revealed that the presence of the α₆ integrin/E-cadherin complex is constant in advanced CRCs, from primary adenocarcinomas to liver and lung metastases; conversely, hepatic metastases from different primary tumors exhibited a more variegated expression of α₆ integrin and E-cadherin, resulting in co-localization of these two proteins in ∼50% of the samples examined **(****Figure 19****).**

A correlation analysis with the clinical outcome of CRC patients revealed that high levels of α₆ integrin, E-cadherin and their complex in liver metastases were all associated with shorter disease-free survival **(****Figure 20A****).** Interestingly, in primary adenocarcinomas, despite an inverse correlation with the levels of E-cadherin and lack of correlation for the expression α₆ integrin, high amounts of complex were still associated to shorter disease-free survival **(****Figure 20A****).** It was investigated if angiopoietin-like 6 was differently expressed in macroscopically normal livers from metastatic CRC patients (n=79) compared to livers from healthy donors (n=17) **(****Figure 5****),** observing that, although the overall levels of angiopoietin-like 6 were similar in the two tissue panels, this ligand was significantly accumulated in blood vessels in the livers of cancer patients compared to those of healthy individuals **(****Figure 20B****).** Finally, it was observed that CRC cells themselves produce angiopoietin-like 6 **(****Figure 21****)** and that its expression is significantly increased in liver metastases compared to primary adenocarcinomas of the same patients (n=22) **(****Figure 20B****).**

These results indicate that α₆ integrin, E-cadherin and angiopoietin-like 6 are correlated with the progression of metastasis and could therefore act as prognostic markers and/or specific molecular targets for the development of anti-metastatic approaches to be translated into the clinics.

## Claims

1. An inhibiting agent of the α₆ integrin/E-cadherin molecular complex for use as a medicament.

2. An inhibiting agent of claim 1, wherein the α₆ integrin/E-cadherin molecular complex is formed by direct and/or indirect molecular interaction between the full length α₆ integrin protein (SEQ ID No 23) or a proteolytic fragment thereof and the full length E-cadherin protein (SEQ ID No 24) or a proteolytic fragment thereof.

3. An inhibiting agent of claim 1 or 2, wherein the α₆ integrin/E-cadherin molecular complex is expressed by tumor cells, preferably metastatic tumor cells, preferably metastatic tumor cells of primary colorectal, bone, brain, breast, cervix, colon, gastric, liver, lung, pancreas, exocrine pancreas, duodenum, ovarian, renal, prostate, stomach, soft tissue, bone marrow, esophagus, skin cancer or metastatic tumor cells of primary lymphoma, more preferably by metastatic tumor cells of primary colorectal cancer.

4. The inhibiting agent of any of claims 1-3 which is selected from
(a) an inhibitor of α₆ integrin/E-cadherin molecular complex on the protein level, or
(b) an inhibitor of a₆ integrin/E-cadherin molecular complex on the nucleic acid level.

5. The inhibiting agent of claim 4 which is selected from an inhibitor of α₆ integrin/E-cadherin molecular complex on the protein level.

6. The inhibiting agent of claim 4 or 5 which binds to the α₆ integrin/E-cadherin molecular complex.

7. The inhibiting agent of any of claims 4-6, wherein the inhibiting agent of the α₆ integrin/E-cadherin molecular complex is selected from an antibody, an antibody fragment, antigen-binding fragment or an aptamer against the α₆ integrin/E-cadherin molecular complex.

8. The inhibiting agent of any of claims 4-7, which is a peptide having the sequence motif LRS and a length of 6 to 100 amino acids.

9. The inhibiting agent of any of claims 4-8, which comprises at least a modified amino acid, e.g. a non-genetically encoded amino acid, or an amino acid mimetic and/or an amino acid in D-conformation.

10. The inhibiting agent of any of claims 4-9 which is a linear or a cyclic peptide.

11. The inhibiting agent of claim 8, which comprises an amino acid sequence selected from the group consisting of: ARPGLRS (SEQ ID NO. 1), MRYALRS (SEQ ID NO. 2), LRPGLRS (SEQ ID NO. 3), LRSGSGS (SEQ ID NO. 4), GIYRLRS (SEQ ID NO. 5), GVYSLRS (SEQ ID NO. 6), LRSGRGS (SEQ ID NO. 7), RREGLRS (SEQ ID NO. 8), SWYTLRS (SEQ ID NO. 9), LAYRLRS (SEQ ID NO. 10), LTYRLRS (SEQ ID NO. 11), VRPGLRS (SEQ ID NO. 12), LRSGRGS (SEQ ID NO. 13), preferably GIYRLRS (SEQ ID NO. 5) or GVYSLRS (SEQ ID NO. 6).

12. The inhibiting agent of claim 11, which comprises the amino acid sequence CGIYRLRSC (SEQ ID NO. 14) or CGVYSLRSC (SEQ ID NO. 15).

13. The inhibiting agent of claim 4, wherein the inhibiting agent of the α₆ integrin/E-cadherin molecular complex is a α₆ integrin/E-cadherin gene expression inhibitor, preferably selected from nucleic acid effector molecules directed against α₆ integrin or/and E-cadherin mRNA, such as an RNAi molecule, such as siRNA, a precursor or a template or a precursor thereof, an antisense molecule or a ribozyme.

14. The inhibiting agent of claim 4 or 13, wherein the siRNA molecule has a sense strand selected from:
(i) SEQ ID NO. 18, SEQ ID NO. 19, SEQ ID NO. 20, SEQ ID NO. 21, SEQ ID NO. 22, SEQ ID NO. 23, SEQ ID NO. 24 or SEQ ID NO. 25; or
(ii) a nucleotide sequence which has an identity degree of at least 85%, at least 90% or at least 95% to any one of the sequences according to (i).

15. The inhibiting agent of any of claims 1-14 for use as a medicament for the prevention or/and treatment of metastasis of a primary cancer disease.

16. The inhibiting agent of claim 15, wherein the primary cancer disease is selected from the group consisting of colorectal, bone, brain, breast, cervix, colon, gastric, liver, lung, pancreas, exocrine pancreas, duodenum, ovarian, renal, prostate, stomach, soft tissue, bone marrow, esophagus or skin cancer or lymphoma, particularly colorectal cancer.

17. The inhibiting agent of claim 15 or 16, wherein the inhibiting agent is used to prevent or/and reduce metastasis in liver tissue, breast tissue, lung tissue, lymph nodes, bone tissue or brain tissue, preferably in liver tissue.

18. The inhibiting agent of any of the claims 15-17 in combination with an additional anticancer or/and antiviral therapy.

19. The inhibiting agent of claim 18, wherein the additional anti-cancer therapy is selected from chemotherapy, radiation therapy, surgical intervention, immunotherapy, gene therapy, target therapy or combinations thereof.

20. The inhibiting agent of any of claims 18-19 in combination with at least one additional chemotherapeutic agent.

21. The inhibiting agent of claim 20, wherein the chemotherapeutic agent is selected from antimetabolites, DNA-fragmenting agents, DNA-crosslinking agents, intercalating agents, protein synthesis inhibitors, Topoisomerase 1 and 2 inhibitors, microtubule-directed agents, kinase inhibitors, hormones and hormone antagonists, anti-tumor antibodies, or any combination thereof.

22. A pharmaceutical composition or kit comprising as an active agent at least one inhibiting agent as defined in any of claims 1 to 14, together with a pharmaceutically acceptable carrier, diluent and/or adjuvant.

23. The pharmaceutical composition or kit of claim 22, further comprising at least one anti-cancer or/and anti-viral agent as defined in any one of claims 19-21.

24. A method of treating or/and preventing a subject suffering from metastasis of a primary cancer disease comprising administering to a subject in need thereof a pharmaceutically effective amount of an inhibiting agent according to any of claims 1-14.

25. A method of screening for an inhibiting agent for the α₆ integrin/E-cadherin molecular complex, comprising the steps of:
(i) incubating an α₆ integrin/E-cadherin molecular complex with an agent under conditions suitable to induce binding of the agent to the complex,
(ii) detecting the binding of the agent to the complex,
(iii) comparing the result obtained in step (ii) with a predetermined binding score, and
(iv) evaluating the agent to be an inhibiting agent for α₆ integrin/E-cadherin molecular complex.

26. The method of claim 25, wherein the binding of the agent to the α₆ integrin/E-cadherin molecular complex is detected via phage displayed peptide binding assay, radio- or dye-labelled ligand binding or/and surface plasmon resonance assay, preferably by phage displayed peptide binding assay.

27. A method for determining the prognosis of metastatic homing of a primary cancer disease, in particular the aggressiveness of the metastatic potential of a primary cancer disease, comprising the steps of:
(i) providing a sample of a patient suffering or suspicious to suffer from metastasis of a primary cancer disease,
(ii) determining the expression or/and amount of the α₆ integrin/E-cadherin molecular complex or/and the amount of angiopoietin-like 6 in the sample, and
(iii) optionally classifying the results obtained in step (ii) in predetermined disease states.

28. The method of claim 27, wherein the primary cancer disease is selected from the group consisting of colorectal, bone, brain, breast, cervix, colon, gastric, liver, lung, pancreas, ovarian, renal, pancreas, prostate, stomach, soft tissue, bone marrow or skin cancer or lymphoma, particularly colorectal cancer.

29. The method of claim 27 or 28, wherein the metastatic homing in liver tissue, breast tissue, lung tissue, lymph nodes, brain tissue or bone tissue, preferably in liver tissue, is determined.

30. The method of any of the claims 27-29, wherein high amounts or upregulated expression of α₆ integrin/E-cadherin molecular complex and/or its ligand are associated with advanced metastasis homing and shorter disease-free survival.

31. The method of any of claims 27-30, wherein the ligand of the α₆ integrin/E-cadherin molecular complex is angiopoietin-like 6 protein.


SEQUENCE LISTING

<110>  University of Torino
       Fondazione Piemontese per la Ricerca sul Cancro

<120>  Inhibitors of alpha6 Integrin/E-Cadherin Complex

<130>  50579A

<160>  43

<170>  PatentIn version 3.5

<210>  1
<211>  7
<212>  PRT
<213>  Artificial Sequence

<220>
<223>  Peptide inhibiting the E-cadherin/alpha6 integrin complex

<400>  1

Ala Arg Pro Gly Leu Arg Ser
1               5


<210>  2
<211>  7
<212>  PRT
<213>  Artificial Sequence

<220>
<223>  Peptide inhibiting the E-cadherin/alpha6 integrin complex

<400>  2

Met Arg Tyr Ala Leu Arg Ser
1               5


<210>  3
<211>  7
<212>  PRT
<213>  Artificial Sequence

<220>
<223>  Peptide inhibiting the E-cadherin/alpha6 integrin complex

<400>  3

Leu Arg Pro Gly Leu Arg Ser
1               5


<210>  4
<211>  7
<212>  PRT
<213>  Artificial Sequence

<220>
<223>  Peptide inhibiting the E-cadherin/alpha6 integrin complex

<400>  4

Leu Arg Ser Gly Ser Gly Ser
1               5


<210>  5
<211>  7
<212>  PRT
<213>  Artificial Sequence

<220>
<223>  Peptide inhibiting the E-cadherin/alpha6 integrin complex

<400>  5

Gly Ile Tyr Arg Leu Arg Ser
1               5


<210>  6
<211>  7
<212>  PRT
<213>  Artificial Sequence

<220>
<223>  Peptide inhibiting the E-cadherin/alpha6 integrin complex

<400>  6

Gly Val Tyr Ser Leu Arg Ser
1               5


<210>  7
<211>  7
<212>  PRT
<213>  Artificial Sequence

<220>
<223>  Peptide inhibiting the E-cadherin/alpha6 integrin complex

<400>  7

Leu Arg Ser Gly Arg Gly Ser
1               5


<210>  8
<211>  7
<212>  PRT
<213>  Artificial Sequence

<220>
<223>  Peptide inhibiting the E-cadherin/alpha6 integrin complex

<400>  8

Arg Arg Glu Gly Leu Arg Ser
1               5


<210>  9
<211>  7
<212>  PRT
<213>  Artificial Sequence

<220>
<223>  Peptide inhibiting the E-cadherin/alpha6 integrin complex

<400>  9

Ser Trp Tyr Thr Leu Arg Ser
1               5


<210>  10
<211>  7
<212>  PRT
<213>  Artificial Sequence

<220>
<223>  Peptide inhibiting the E-cadherin/alpha6 integrin complex

<400>  10

Leu Ala Tyr Arg Leu Arg Ser
1               5


<210>  11
<211>  7
<212>  PRT
<213>  Artificial Sequence

<220>
<223>  Peptide inhibiting the E-cadherin/alpha6 integrin complex

<400>  11

Leu Thr Tyr Arg Leu Arg Ser
1               5


<210>  12
<211>  7
<212>  PRT
<213>  Artificial Sequence

<220>
<223>  Peptide inhibiting the E-cadherin/alpha6 integrin complex

<400>  12

Val Arg Pro Gly Leu Arg Ser
1               5


<210>  13
<211>  7
<212>  PRT
<213>  Artificial Sequence

<220>
<223>  Peptide inhibiting the E-cadherin/alpha6 integrin complex

<400>  13

Leu Arg Ser Gly Arg Gly Ser
1               5

<210>  14
<211>  9
<212>  PRT
<213>  Artificial Sequence

<220>
<223>  Peptide inhibiting the E-cadherin/alpha6 integrin complex

<400>  14

Cys Gly Ile Tyr Arg Leu Arg Ser Cys
1               5


<210>  15
<211>  9
<212>  PRT
<213>  Artificial Sequence

<220>
<223>  Peptide inhibiting the E-cadherin/alpha6 integrin complex

<400>  15

Cys Gly Val Tyr Ser Leu Arg Ser Cys
1               5


<210>  16
<211>  1073
<212>  PRT
<213>  Homo sapiens

<400>  16

Met Ala Ala Ala Gly Gln Leu Cys Leu Leu Tyr Leu Ser Ala Gly Leu
1               5                   10                  15

Leu Ser Arg Leu Gly Ala Ala Phe Asn Leu Asp Thr Arg Glu Asp Asn
            20                  25                  30

Val Ile Arg Lys Tyr Gly Asp Pro Gly Ser Leu Phe Gly Phe Ser Leu
        35                  40                  45

Ala Met His Trp Gln Leu Gln Pro Glu Asp Lys Arg Leu Leu Leu Val
    50                  55                  60

Gly Ala Pro Arg Ala Glu Ala Leu Pro Leu Gln Arg Ala Asn Arg Thr
65                  70                  75                  80

Gly Gly Leu Tyr Ser Cys Asp Ile Thr Ala Arg Gly Pro Cys Thr Arg
                85                  90                  95

Ile Glu Phe Asp Asn Asp Ala Asp Pro Thr Ser Glu Ser Lys Glu Asp
            100                 105                 110

Gln Trp Met Gly Val Thr Val Gln Ser Gln Gly Pro Gly Gly Lys Val
        115                 120                 125

Val Thr Cys Ala His Arg Tyr Glu Lys Arg Gln His Val Asn Thr Lys
    130                 135                 140

Gln Glu Ser Arg Asp Ile Phe Gly Arg Cys Tyr Val Leu Ser Gln Asn
145                 150                 155                 160

Leu Arg Ile Glu Asp Asp Met Asp Gly Gly Asp Trp Ser Phe Cys Asp
                165                 170                 175

Gly Arg Leu Arg Gly His Glu Lys Phe Gly Ser Cys Gln Gln Gly Val
            180                 185                 190

Ala Ala Thr Phe Thr Lys Asp Phe His Tyr Ile Val Phe Gly Ala Pro
        195                 200                 205

Gly Thr Tyr Asn Trp Lys Gly Ile Val Arg Val Glu Gln Lys Asn Asn
    210                 215                 220

Thr Phe Phe Asp Met Asn Ile Phe Glu Asp Gly Pro Tyr Glu Val Gly
225                 230                 235                 240

Gly Glu Thr Glu His Asp Glu Ser Leu Val Pro Val Pro Ala Asn Ser
                245                 250                 255

Tyr Leu Gly Phe Ser Leu Asp Ser Gly Lys Gly Ile Val Ser Lys Asp
            260                 265                 270

Glu Ile Thr Phe Val Ser Gly Ala Pro Arg Ala Asn His Ser Gly Ala
        275                 280                 285

Val Val Leu Leu Lys Arg Asp Met Lys Ser Ala His Leu Leu Pro Glu
    290                 295                 300

His Ile Phe Asp Gly Glu Gly Leu Ala Ser Ser Phe Gly Tyr Asp Val
305                 310                 315                 320

Ala Val Val Asp Leu Asn Lys Asp Gly Trp Gln Asp Ile Val Ile Gly
                325                 330                 335

Ala Pro Gln Tyr Phe Asp Arg Asp Gly Glu Val Gly Gly Ala Val Tyr
            340                 345                 350

Val Tyr Met Asn Gln Gln Gly Arg Trp Asn Asn Val Lys Pro Ile Arg
        355                 360                 365

Leu Asn Gly Thr Lys Asp Ser Met Phe Gly Ile Ala Val Lys Asn Ile
    370                 375                 380

Gly Asp Ile Asn Gln Asp Gly Tyr Pro Asp Ile Ala Val Gly Ala Pro
385                 390                 395                 400

Tyr Asp Asp Leu Gly Lys Val Phe Ile Tyr His Gly Ser Ala Asn Gly
                405                 410                 415

Ile Asn Thr Lys Pro Thr Gln Val Leu Lys Gly Ile Ser Pro Tyr Phe
            420                 425                 430

Gly Tyr Ser Ile Ala Gly Asn Met Asp Leu Asp Arg Asn Ser Tyr Pro
        435                 440                 445

Asp Val Ala Val Gly Ser Leu Ser Asp Ser Val Thr Ile Phe Arg Ser
    450                 455                 460

Arg Pro Val Ile Asn Ile Gln Lys Thr Ile Thr Val Thr Pro Asn Arg
465                 470                 475                 480

Ile Asp Leu Arg Gln Lys Thr Ala Cys Gly Ala Pro Ser Gly Ile Cys
                485                 490                 495

Leu Gln Val Lys Ser Cys Phe Glu Tyr Thr Ala Asn Pro Ala Gly Tyr
            500                 505                 510

Asn Pro Ser Ile Ser Ile Val Gly Thr Leu Glu Ala Glu Lys Glu Arg
        515                 520                 525

Arg Lys Ser Gly Leu Ser Ser Arg Val Gln Phe Arg Asn Gln Gly Ser
    530                 535                 540

Glu Pro Lys Tyr Thr Gln Glu Leu Thr Leu Lys Arg Gln Lys Gln Lys
545                 550                 555                 560

Val Cys Met Glu Glu Thr Leu Trp Leu Gln Asp Asn Ile Arg Asp Lys
                565                 570                 575

Leu Arg Pro Ile Pro Ile Thr Ala Ser Val Glu Ile Gln Glu Pro Ser
            580                 585                 590

Ser Arg Arg Arg Val Asn Ser Leu Pro Glu Val Leu Pro Ile Leu Asn
        595                 600                 605

Ser Asp Glu Pro Lys Thr Ala His Ile Asp Val His Phe Leu Lys Glu
    610                 615                 620

Gly Cys Gly Asp Asp Asn Val Cys Asn Ser Asn Leu Lys Leu Glu Tyr
625                 630                 635                 640

Lys Phe Cys Thr Arg Glu Gly Asn Gln Asp Lys Phe Ser Tyr Leu Pro
                645                 650                 655

Ile Gln Lys Gly Val Pro Glu Leu Val Leu Lys Asp Gln Lys Asp Ile
            660                 665                 670

Ala Leu Glu Ile Thr Val Thr Asn Ser Pro Ser Asn Pro Arg Asn Pro
        675                 680                 685

Thr Lys Asp Gly Asp Asp Ala His Glu Ala Lys Leu Ile Ala Thr Phe
    690                 695                 700

Pro Asp Thr Leu Thr Tyr Ser Ala Tyr Arg Glu Leu Arg Ala Phe Pro
705                 710                 715                 720

Glu Lys Gln Leu Ser Cys Val Ala Asn Gln Asn Gly Ser Gln Ala Asp
                725                 730                 735

Cys Glu Leu Gly Asn Pro Phe Lys Arg Asn Ser Asn Val Thr Phe Tyr
            740                 745                 750

Leu Val Leu Ser Thr Thr Glu Val Thr Phe Asp Thr Pro Asp Leu Asp
        755                 760                 765

Ile Asn Leu Lys Leu Glu Thr Thr Ser Asn Gln Asp Asn Leu Ala Pro
    770                 775                 780

Ile Thr Ala Lys Ala Lys Val Val Ile Glu Leu Leu Leu Ser Val Ser
785                 790                 795                 800

Gly Val Ala Lys Pro Ser Gln Val Tyr Phe Gly Gly Thr Val Val Gly
                805                 810                 815

Glu Gln Ala Met Lys Ser Glu Asp Glu Val Gly Ser Leu Ile Glu Tyr
            820                 825                 830

Glu Phe Arg Val Ile Asn Leu Gly Lys Pro Leu Thr Asn Leu Gly Thr
        835                 840                 845

Ala Thr Leu Asn Ile Gln Trp Pro Lys Glu Ile Ser Asn Gly Lys Trp
    850                 855                 860

Leu Leu Tyr Leu Val Lys Val Glu Ser Lys Gly Leu Glu Lys Val Thr
865                 870                 875                 880

Cys Glu Pro Gln Lys Glu Ile Asn Ser Leu Asn Leu Thr Glu Ser His
                885                 890                 895

Asn Ser Arg Lys Lys Arg Glu Ile Thr Glu Lys Gln Ile Asp Asp Asn

900                 905                 910

Arg Lys Phe Ser Leu Phe Ala Glu Arg Lys Tyr Gln Thr Leu Asn Cys
        915                 920                 925

Ser Val Asn Val Asn Cys Val Asn Ile Arg Cys Pro Leu Arg Gly Leu
    930                 935                 940

Asp Ser Lys Ala Ser Leu Ile Leu Arg Ser Arg Leu Trp Asn Ser Thr
945                 950                 955                 960

Phe Leu Glu Glu Tyr Ser Lys Leu Asn Tyr Leu Asp Ile Leu Met Arg
                965                 970                 975

Ala Phe Ile Asp Val Thr Ala Ala Ala Glu Asn Ile Arg Leu Pro Asn
            980                 985                 990

Ala Gly Thr Gln Val Arg Val Thr  Val Phe Pro Ser Lys  Thr Val Ala
        995                 1000                1005

Gln Tyr  Ser Gly Val Pro Trp  Trp Ile Ile Leu Val  Ala Ile Leu
    1010                1015                1020

Ala Gly  Ile Leu Met Leu Ala  Leu Leu Val Phe Ile  Leu Trp Lys
    1025                1030                1035

Cys Gly  Phe Phe Lys Arg Asn  Lys Lys Asp His Tyr  Asp Ala Thr
    1040                1045                1050

Tyr His  Lys Ala Glu Ile His  Ala Gln Pro Ser Asp  Lys Glu Arg
    1055                1060                1065

Leu Thr  Ser Asp Ala
    1070

<210>  17
<211>  882
<212>  PRT
<213>  Homo sapiens

<400>  17

<210>  18
<211>  21
<212>  RNA
<213>  Artificial Sequence

<220>
<223>  siGENOME ON-TARGETplus SMART pool duplex (5), J-007214-05, ITGA6
       Sense Sequence

<400>  18
ggaucgaguu ugauaacgau u                                                21

<210>  19
<211>  21
<212>  RNA
<213>  Artificial Sequence

<220>
<223>  siGENOME ON-TARGETplus SMART pool duplex (6), J-007214-06, ITGA6
       Sense Sequence

<400>  19
ggauaugccu ccagguuaau u                                                21

<210>  20
<211>  21
<212>  RNA
<213>  Artificial Sequence

<220>
<223>  siGENOME ON-TARGETplus SMART pool duplex (7), J-007214-07, ITGA6
       Sense Sequence

<400>  20
gaaagggauu guucguguau u                                                21

<210>  21
<211>  21
<212>  RNA
<213>  Artificial Sequence

<220>
<223>  siGENOME ON-TARGETplus SMART pool duplex (8), J-007214-08, ITGA6
       Sense Sequence

<400>  21
acagauagau gauaacagau u                                                21


<210>  22
<211>  21
<212>  RNA
<213>  Artificial Sequence

<220>
<223>  siGENOME ON-TARGETplus SMART pool duplex (8), J-003877-08, CDH1
       Sense Sequence

<400>  22
ggccugaagu gacucguaau u                                                21


<210>  23
<211>  21
<212>  RNA
<213>  Artificial Sequence

<220>
<223>  siGENOME ON-TARGETplus SMART pool duplex (9), J-003877-09, CDH1
       Sense Sequence

<400>  23
gagaacgcau ugccacauau u                                                21


<210>  24
<211>  21
<212>  RNA
<213>  Artificial Sequence

<220>
<223>  siGENOME ON-TARGETplus SMART pool duplex (10), J-003877-10, CDH1
       Sense Sequence

<400>  24
gggacaacgu uuauuacuau u                                                21


<210>  25
<211>  21
<212>  RNA
<213>  Artificial Sequence

<220>
<223>  siGENOME ON-TARGETplus SMART pool duplex (11), J-003877-11, CDH1
       Sense Sequence

<400>  25
gacaaugguu cuccaguugu u                                                21


<210>  26
<211>  46
<212>  DNA
<213>  Artificial Sequence

<220>
<223>  oligonucleotide annealed and inserted into pGEX-4T.1 between
       BamHI and NotI sites to create the pGEX-4T.1-CGIYRLRSC plasmid

<400>  26
gatccggagc ctgtggaata tatagattaa gaagttgtgc gggcgc                    46


<210>  27
<211>  46
<212>  DNA
<213>  Artificial Sequence

<220>
<223>  oligonucleotide annealed and inserted into pGEX-4T.1 between
       BamHI and NotI sites to create the pGEX-4T.1-CGIYRLRSC plasmid

<400>  27
ggccgcgccc gcacaacttc ttaatctata tattccacag gctccg                    46


<210>  28
<211>  36
<212>  DNA
<213>  Artificial Sequence

<220>
<223>  Oligonucleotide used for the preparation of cells stably
       overexpressing the MTS-targeted proteins

<400>  28
aaacttaagc ttgccaccat ggccgccgcc gggcag                               36


<210>  29
<211>  33
<212>  DNA
<213>  Artificial Sequence

<220>
<223>  oligonucleotide used for the preparation of cells stably
       overexpressing the MTS-targeted proteins

<400>  29
tacacgggcc ctctatgcat cagaagtaag cct                                  33


<210>  30
<211>  21
<212>  DNA
<213>  Artificial Sequence

<220>
<223>  For quantification of residual transcripts in silenced cells, the
       oligonucleotide was used for real-time PCR amplification of the
       cDNAs in an ABI PRISM 7700 instrument

<400>  30
tgagtgtccc ccggtatctt c                                               21


<210>  31
<211>  24
<212>  DNA
<213>  Artificial Sequence

<220>
<223>  For quantification of residual transcripts in silenced cells, the

oligonucleotide was used for real-time PCR amplification of the
      cDNAs in an ABI PRISM 7700 instrument

<400>  31
cagtatcagc cgctttcaga tttt                                              24


<210>  32
<211>  29
<212>  DNA
<213>  Artificial Sequence

<220>
<223>  For quantification of residual transcripts in silenced cells, the
      oligonucleotide was used for real-time PCR amplification of the
      cDNAs in an ABI PRISM 7700 instrument

<400>  32
gctggttata atccttcaat atcaattgt                                         29


<210>  33
<211>  21
<212>  DNA
<213>  Artificial Sequence

<220>
<223>  For quantification of residual transcripts in silenced cells, the
      oligonucleotide was used for real-time PCR amplification of the
      cDNAs in an ABI PRISM 7700 instrument

<400>  33
ttgggctcag aaccttggtt t                                                 21


<210>  34
<211>  19
<212>  DNA
<213>  Artificial Sequence

<220>
<223>  For quantification of residual transcripts in silenced cells, the
      oligonucleotide was used for real-time PCR amplification of the
      cDNAs in an ABI PRISM 7700 instrument

<400>  34
gaaggtgaag gtcggagtc                                                    19


<210>  35
<211>  20
<212>  DNA
<213>  Artificial Sequence

<220>
<223>  For quantification of residual transcripts in silenced cells, the
      oligonucleotide was used for real-time PCR amplification of the
      cDNAs in an ABI PRISM 7700 instrument

<400>  35
gaagatggtg atgggatttc                                                   20


<210>  36
<211>  20

<212>  DNA
<213>  Artificial Sequence

<220>
<223>  oligonucleotide used to evaluate the presence of different
       splicing forms of alpha6 integrin

<400>  36
gactcttaac tgtagcgtga                                                  20


<210>  37
<211>  20
<212>  DNA
<213>  Artificial Sequence

<220>
<223>  oligonucleotide used to evaluate the presence of different
       splicing forms of alpha6 integrin

<400>  37
atctctcgct cttctttccg                                                  20


<210>  38
<211>  9
<212>  PRT
<213>  Artificial Sequence

<220>
<223>  protein used for the validation of MTS phage specificity

<400>  38

Cys Leu Arg Ser Gly Arg Gly Ser Cys
1               5


<210>  39
<211>  9
<212>  PRT
<213>  Artificial Sequence

<220>
<223>  protein used for the validation of MTS phage specificity

<400>  39

Cys Leu Arg Pro Gly Leu Arg Ser Cys
1               5


<210>  40
<211>  9
<212>  PRT
<213>  Artificial Sequence

<220>
<223>  protein used for the validation of MTS phage specificity

<400>  40

Cys Met Arg Tyr Ala Leu Arg Ser Cys
1               5

<210>  41
<211>  9
<212>  PRT
<213>  Artificial Sequence

<220>
<223>  protein used for the validation of MTS phage specificity

<400>  41

Cys Ala Arg Pro Gly Leu Arg Ser Cys
1               5


<210>  42
<211>  9
<212>  PRT
<213>  Artificial Sequence

<220>
<223>  protein used for the validation of MTS phage specificity

<400>  42

Cys Leu Arg Ser Gly Ser Gly Ser Cys
1               5


<210>  43
<211>  5
<212>  PRT
<213>  Artificial Sequence

<220>
<223>  peptide used as negative control

<400>  43

Cys Ala Arg Ala Cys
1               5